# EUROPÄISCHE PATENTANMELDUNG

(11) **EP 4 201 433 A1**
(43) Veröffentlichungstag der Anmeldung: **28.06.2023**
(21) Anmeldenummer: 22214312.5
(22) Anmeldetag: 16.12.2022
(51) Int. Cl.: A61L 2/00, A61L 2/08, B65B 55/08, B29C 49/42, B67C 7/00

(54) **VORRICHTUNG UND VERFAHREN ZUM BEHANDELN UND INSBESONDERE STERILISIEREN VON BEHÄLTNISSEN**

(30) Priorität: 23.12.2021 DE 102021134499
(71) Anmelder: Krones AG, 93073 Neutraubling (DE)
(72) Erfinder: Gette, Viktor, 93073 Neutraubling (DE); Senn, Konrad, 93073 Neutraubling (DE); Pense, Andreas, 93073 Neutraubling (DE); Fuchs, Florian, 93073 Neutraubling (DE); Feigl, Alexander, 93073 Neutraubling (DE); Schoenberger, Wolfgang, 93073 Neutraubling (DE); Eichenseher, Andreas, 93073 Neutraubling (DE); Hoffmann, Florian, 93073 Neutraubling (DE); Knott, Josef, 93073 Neutraubling (DE); Vornehm, Andreas, 93073 Neutraubling (DE); Neubauer, Michael, 93073 Neutraubling (DE); Kornprobst, Stefan, 93073 Neutraubling (DE)
(74) Vertreter: Bittner, Bernhard

(57) **Zusammenfassung**

Die Erfindung betrifft eine Behältnisbehandlungsvorrichtung (1), insbesondere Behältnissterilisationsvorrichtung, mit mehreren Transporteinrichtungen (100, 200, 300), zum Transport eines Behältnisses (10) entlang eines vorgegebenen Transportpfads (T), wobei mindestens eine der Transporteinrichtungen (100, 200, 300) mindestens eine Halteeinrichtung (140, 240) zum Halten mindestens eines Behältnisses (10) während einer Behältnisbehandlung und/oder des Transports entlang des Transportpfads (T) aufweist, wobei die Transporteinrichtung (100, 200, 300) einen rotierbaren Träger (120, 220) und eine Vielzahl von an diesem Träger angeordneten Halteelementen (140, 240) aufweist, wobei ein Haltelement (140, 240) in einem ersten Sektor (270), in dem eine Aufnahme eines Behältnisses (10) erfolgt, eine Höhe (H) gegenüber einer senkrechten Projektion des Transportpfads (T) aufweist, welche von derjenigen in einem zweiten Sektor (274) verschieden ist, in welchem eine Abgabe eines Behältnisses (10) erfolgt und wobei ein Sektor (276), in welchem ein zumindest teilweiser Ausgleich des Höhenunterschieds der Halteelemente erfolgt, entlang einer Rotationsrichtung des Trägers nach dem zweiten Sektor (274) und vor dem ersten Sektor (270) liegt. Weiterhin betrifft die Erfindung ein Verfahren zur Behandlung, insbesondere zur Sterilisation von Behältnissen (10).

## Beschreibung

Die vorliegende Erfindung betrifft eine Behältnisbehandlungsvorrichtung, insbesondere Behältnissterilisationsvorrichtung, mit mehreren Transporteinrichtungen, zum Transport eines Behältnisses entlang eines vorgegebenen Transportpfads innerhalb eines Gehäuses, wobei mindestens eine der Transporteinrichtungen eine Behältnisaußenflächenbehandlungseinrichtung ist und mindestens eine der Transporteinrichtungen eine Behältnisinnenflächenbehandlungseinrichtung ist, welche jeweils mindestens eine Halteeinrichtung zum Halten mindestens eines Behältnisses während einer Behältnisbehandlung und/oder des Transports entlang des Transportpfads aufweisen. Weiterhin betrifft die Erfindung ein Verfahren zum Behandeln von Behältnissen, insbesondere zur Sterilisation von Behältnissen.

Aus dem Stand der Technik sind verschiedene Behältnisbehandlungsvorrichtungen bekannt. Oftmals werden in einer solchen Behältnisbehandlungsvorrichtung Vorformlinge behandelt, die in einem folgenden Schritt beispielsweise zu Flaschen oder anderen Gebinden umgeformt werden. Dies hat den Vorteil, dass eine vergleichsweise geringe Fläche behandelt werden muss. Dies hat insbesondere bei Sterilisationsprozessen einen Vorteil, da so eine kleinere Fläche sterilisiert werden muss und Sterilisationsmittel und/oder Energie eingespart werden kann.

Die Sterilisation von Behältnissen erfolgt üblicherweise in einem zumindest weitgehend geschlossenen Gehäuse, um Verunreinigungen von außen zu vermeiden. In einigen Systemen umfasst zumindest ein Sterilisationsprozess eine Beaufschlagung mit Strahlung. Auch eine dafür vorgesehene Strahlenquelle ist üblicherweise zumindest abschnittsweise in einem Gehäuse angeordnet und gibt die Strahlung in das Innere des Gehäuses ab, wobei das Gehäuse abschirmende Eigenschaften für diese Strahlung aufweist. Dadurch können Personen in der Nähe einer solchen Sterilisationseinrichtung vor Streustrahlung geschützt werden.

Insbesondere bei der Sterilisation verschiedener Bereiche von Behältnissen nacheinander ergibt sich die Problematik, dass alle diese Einrichtungen innerhalb des Gehäuses angeordnet sein sollten, um zwischenzeitliche Kontamination der bereits sterilisierten Flächen zu vermeiden. Sind mehrere derartige Sterilisationseinrichtungen innerhalb eines gemeinsamen Gehäuses angeordnet, bilden Sie ein sogenanntes Sterilisationsmodul. Dieses ist aufgrund der vielen Sterilisationseinrichtungen und der Transporteinrichtungen zur Übergabe einzelner Behältnisse von einer Sterilisationseinrichtung zur nächsten oft sehr voluminös und erfordert eine große Stellfläche.

Aus dem internen Stand der Technik der Anmelderin ist es dabei bekannt, Kunststoffvorformlinge nach der Erwärmung in einem Ofen an den Außen- und Innenflächen zu sterilisieren. Die Sterilisation eines zu befüllenden Behälters ist neben dem eigentlichen Füllvorgang der zentrale Prozessschritt in einer aseptischen Abfüllanlage. Neuere Entwicklungen nutzen dabei eine ionisierende Strahlung, um eine Keimreduzierung zu erreichen. Diese Strahlung besteht in den meisten Anwendungen aus beschleunigten Elektronen, die in einer entsprechenden Anlage erzeugt werden und die zu sterilisierenden Behältnisse behandeln, wobei Systeme, die zur Sterilisation eingesetzt werden, aus einer Elektronenerzeugungsvorrichtung und einem Strahlfinger zur Entkeimung der inneren Oberflächen und einer Elektronenerzeugungsvorrichtung und einem Flächenstrahler zum Entkeimen von äußeren Oberflächen bestehen. Die Behandlungseinrichtungen zur Sterilisation der Außenflächen und die Sterilisation der Innenflächen sind dabei jeweils auf einem Karussell oder Transportstern angeordnet, welche mit einem Teilungsverzugsstern verbunden sind.

Die bei der Sterilisation entstehende Röntgenstrahlung muss dabei durch geeignete Abschirmungen von der Umwelt abgeschirmt werden, so dass die beiden Behandlungseinrichtungen in einer strahlungsabschirmenden Vorrichtung eingebettet bzw. eingehaust sind. Eine derartige Abschirmungseinrichtung ist beispielsweise in EP 2 845 610 A1 näher beschrieben. Die darin beschriebenen Ausführungsformen stellen jeweils auch bevorzugte Ausführungsformen des Gehäuses der vorliegenden Erfindung dar. Die Anmelderin behält sich ausdrücklich vor, Merkmale dieser Druckschrift auch zur Definition bevorzugter Ausführungsformen der vorliegenden Erfindung heranzuziehen. Um die Strahlungsabschirmung auch an dem Ein- und Auslauffenstern zu der eingehausten Vorrichtung zu gewährleisten ist jeweils noch ein Einlaufstern vorgeschaltet bzw. ein Auslaufstern nachgeschaltet. Dadurch ergibt sich eine Gesamtvorrichtung, welche mit fünf Sternen bzw. Karussellen ausgestattet ist.

Hierdurch entsteht jedoch ein sehr langer Transfer bzw. eine sehr lange Transportstrecke vom Ofen zu der Blasmaschine bzw. eine lange Verweilzeit der Behältnisse in dem Behandlungsmodul. Dadurch ergeben sich große Schwierigkeiten beim Ausformen von Kunststoffvorformlingen zu fertigen Kunststoffbehältnissen, da die Vorformlinge auf der Transportstrecke zu sehr abkühlen. Zusätzlich liegt in den häufigen Übergaben, innerhalb eines nur schlecht zugänglichen strahlungsabschirmenden Gehäuses, eine erhöhte Gefahr von fehlerhaften Übergaben, vor allem beim Übergang vom Einlaufstern in das Außenbehandlungsmodul, da hier eine punktuelle Übergabe von einer Halteklammer an einen Haltedorn stattfindet. Fehlgriffe wie Schiefsitzer oder ähnliches können dann bei den folgenden Übergaben zum Verlust des Vorformlings oder auch zu Beschädigungen der Übergabeeinrichtungen führen. Bei größeren Leistungen erhöht sich auch der Footprint der Anlage, da diese pro vorhandenen Stern bzw. Karussell zunimmt.

Es besteht daher der Bedarf, eine Behältnisbehandlungsvorrichtung, insbesondere eine Behältnissterilisationsvorrichtung bereitzustellen, welche kompakter realisierbar ist und dennoch einen hohen Durchsatz an Behältnissen bietet. Eine solche Behältnisbehandlungsvorrichtung sollte möglichst modular aufgebaut sein und mit anderen vor- oder nachgelagerten Behältnisbehandlungsvorrichtungen wie einer Blasformeinrichtung oder einer Heizeinrichtung kompatibel sein. Weiterhin besteht ein Bedarf an einem Verfahren für eine effektive Behältnisbehandlung auf einem kurzen Transportpfad.

Diese Aufgabe wird durch die Gegenstände der unabhängigen Patentansprüche gelöst. Vorteilhafte Ausführungsformen und Weiterbildungen sind Gegenstand der Unteransprüche.

Eine erfindungsgemäße Lösung des zugrundeliegenden Problems wird somit durch eine Behältnisbehandlungsvorrichtung, insbesondere Behältnissterilisationsvorrichtung, mit mehreren Transporteinrichtungen, zum Transport eines Behältnisses entlang eines vorgegebenen Transportpfads innerhalb eines Gehäuses gelöst, wobei mindestens eine der Transporteinrichtungen eine Behältnisaußenflächenbehandlungseinrichtung ist und mindestens eine der Transporteinrichtungen eine Behältnisinnenflächenbehandlungseinrichtung ist. Sowohl die Behältnisaußenflächenbehandlungseinrichtung als auch die Behältnisinnenflächenbehandlungseinrichtung weist jeweils mindestens eine Halteeinrichtung zum Halten mindestens eines Behältnisses während einer Behältnisbehandlung und/oder des Transports entlang des Transportpfads auf.

Wesentlich für eine erste Lösung des Problems ist, dass eine Halteeinrichtung der Behältnisaußenflächenbehandlungseinrichtung und/oder der Behältnisinnenflächenbehandlungseinrichtung zumindest zeitweise in den Bereich einer Öffnung einer Gehäusewandung bringbar ist, um ein in den vom Gehäuse umgebenen Raum zu bringendes Behältnis aufzunehmen oder ein aus dem vom Gehäuse umgebenen Raum auszuführendes Behältnis abzugeben. Durch die Möglichkeit, bei einer solchen Behältnisbehandlungsvorrichtung eine Halteeinrichtung der Behältnisaußenflächenbehandlungseinrichtung und/oder der Behältnisinnenflächenbehandlungseinrichtung zumindest zeitweise in den Bereich einer Öffnung einer Gehäusewandung bringen zu können, wird ermöglicht, das Behältnis direkt an eine Behältnisbehandlungseinrichtung zu übergeben oder es von einer solchen aufzunehmen. Dadurch kann auf eine Transporteinrichtung verzichtet werden, welche innerhalb des Gehäuses das eingebrachte Behältnis an die erste entlang des Transportpfads folgende Behältnisbehandlungseinrichtung übergibt und/oder das behandelte Behältnis von der innerhalb des Gehäuses letzten Behältnisbehandlungseinrichtung übernimmt und aus dem Gehäuse ausgibt.

Vorzugsweise ist eine Halteeinrichtung der Behältnisaußenflächenbehandlungseinrichtung und/oder der Behältnisinnenflächenbehandlungseinrichtung bezüglich einer Behältnisbehandlungseinrichtung relativbeweglich. Ergänzend oder alternativdazu ist in einer besonders bevorzugten Ausführungsform eine Position und/oder Ausrichtung eines Behältnisses im Bereich der Behältnisaußenflächenbehandlungseinrichtung und/oder der Behältnisinnenflächenbehandlungseinrichtung bezüglich einer senkrechten Projektion des Transportpfads veränderbar. Diese Bewegung von Behältnissen relativ zueinander oder bezüglich der Projektion des Transportpfads ermöglicht beispielsweise das Einführen eines Strahlfingers oder eine Düse in das Innere des Behältnisses zur Behältnisinnenflächenbehandlung. Ebenso kann für einige Anwendungen auch eine Relativbewegung im Bereich der Behältnisaußenflächenbehandlungseinrichtung vorteilhaft sein, beispielsweise um ein Behältnis gegenüber einer Düse oder Strahlungsquelle zu rotieren und/oder zu kippen, um auch zuvor abgeschattete Oberflächenbereiche für die Behältnisaußenflächenbehandlungseinrichtung zugänglich zu machen.

In einer bevorzugten Ausführungsform weisen die Behältnisaußenflächenbehandlungseinrichtung und/oder die Behältnisinnenflächenbehandlungseinrichtung jeweils einen rotierbaren Träger auf. Derartige rotierbare Träger sind insbesondere im Bereich der Handhabung von Flaschen oft verwendet, so dass sich bei dieser Ausführungsform eine besonders gute Möglichkeit der Integration der Behältnisbehandlungsvorrichtung in bestehende oder neu auszurüstende Anlagen bietet. Vorzugsweise verlaufen die Rotationsachsen der Behältnisaußenflächenbehandlungseinrichtung und der Behältnisinnenflächenbehandlungseinrichtung im Wesentlichen parallel, insbesondere bevorzugt genau parallel. Durch diese Ausgestaltung kann ein besonders ruhiger Lauf der Träger bezüglich einander erreicht werden.

Bevorzugt weist die Behältnisaußenflächenbehandlungseinrichtung und/oder die Behältnisinnenflächenbehandlungseinrichtung eine Vielzahl von Halteelementen auf. Dadurch ist eine Behandlung mehrerer Behältnisse während einer wiederkehrenden Bewegung der jeweiligen Behandlungseinrichtung möglich, was den Durchsatz steigert.

In einer bevorzugten Ausführungsform ist eine Antriebseinrichtung mindestens einer der innerhalb des Gehäuses angeordnete Transporteinrichtungen auf bezüglich einer durch den Transportpfad aufgespannten Fläche auf einer anderen Seite angeordnet als eine Antriebseinrichtung mindestens einer anderen innerhalb des Gehäuses angeordneten Transporteinrichtung. Dadurch kann vermieden werden, dass alle Antriebseinrichtungen und eventuell notwendige Leitungen auf einer Seite der durch den Transportpfad aufgespannten Fläche angeordnet sind. Vielmehr ist es möglich, dass beispielsweise die Antriebseinrichtung für eine oder mehrere der innerhalb des Gehäuses angeordnete Transporteinrichtungen oberhalb dieser Fläche angeordnet ist und diejenigen für mindestens eine andere innerhalb des Gehäuses angeordnete Transporteinrichtung unterhalb dieser Ebene. Dadurch ist ein Versatz dieser Antriebseinrichtungen möglich, so dass der Raum innerhalb des Gehäuses effizienter ausgenutzt werden kann. Dies ermöglicht eine kompaktere Ausgestaltung der Behältnisbehandlungsvorrichtung.

Insbesondere ist bevorzugt, dass mindestens eine Antriebseinrichtung mindestens einer der innerhalb des Gehäuses angeordnete Transporteinrichtungen außerhalb des Gehäuses angeordnet ist. Insbesondere handelt es sich dabei um eine Antriebseinrichtung, die oberhalb der durch den Transportpfad aufgespannten Fläche angeordnet ist. Dies ermöglicht den Zugang zu der Antriebseinrichtung, beispielsweise zu Wartungsarbeiten, ohne dass dazu das Gehäuse geöffnet werden muss.

Als besonders bevorzugt hat sich eine Ausführungsform herausgestellt, bei der eine Antriebseinrichtung der Behältnisaußenflächenbehandlungseinrichtung bezüglich einer durch den Transportpfad aufgespannten Ebene auf einer anderen Seite liegt als die Antriebseinrichtung der Behältnisinnenflächenbehandlungseinrichtung und mindestens einer weiteren Transporteinrichtung. Insbesondere ist bevorzugt, dass eine Antriebseinrichtung der Behältnisaußenflächenbehandlungseinrichtung oberhalb der durch den Transportpfad aufgespannten Ebene angeordnet ist. Dies hat sich insbesondere dann als bevorzugt erwiesen, wenn die Halteelemente der Behältnisaußenflächenbehandlungseinrichtung das jeweilige Behältnis innen greifende Halteelemente sind. Bei dieser Ausgestaltung ist der Raum unterhalb der Behältnisaußenflächenbehandlungseinrichtung zumindest weitgehend frei. Die hängend transportierten Behältnisse werden somit nicht von eventuell in den Transportpfad hineinragenden Gegenständen wie beispielsweise eine Zuleitung zur Antriebseinrichtung gestört. Weiterhin bietet diese Ausgestaltung den Vorteil, dass bei einer Steuerung der Halteelemente, beispielsweise eine Verschiebung eines Haltelements relativ zu einem anderen Halteelement beispielsweise in Höhenrichtung, durch eine Steuerkurve, diese Steuerkurve als bevorzugt einziges Element unterhalb des Trägers der Behältnisaußenflächenbehandlungseinrichtung angeordnet ist und daher für Einstellungen besonders leicht zugänglich ist.

In einer bevorzugten Ausführungsform umfasst die Behältnisinnenflächenbehandlungseinrichtung eine Vielzahl von Behandlungseinrichtungen, beispielsweise Düsen oder Strahlfinger, die bevorzugt jeweils zur Behandlung eines Behältnisses zumindest abschnittsweise in das Innere dieses Behältnisses einführbar sind. Auch dies ermöglicht eine Steigerung des Durchsatzes, da die Behandlung mehrerer Behältnisse während einer wiederkehrenden Bewegung, bevorzugt einer Rotation um eine zentrale Achse, der Behältnisinnenflächenbehandlungseinrichtung ermöglicht wird.

Vorzugsweise sind innerhalb des Gehäuses zusätzlich zu der Behältnisaußenflächenbehandlungseinrichtung und der Behältnisinnenflächenbehandlungseinrichtung höchstens zwei weitere Transporteinrichtungen angeordnet. Dadurch kann das Volumen des vom Gehäuse umschlossenen Raums weiter reduziert werden und die Behältnisbehandlungsvorrichtung besonders kompakt ausgeführt sein. In einer weiter bevorzugten Ausführungsform ist lediglich eine weitere Transporteinrichtung zum Transportieren eines Behältnisses durch den vom Gehäuse umgebenen Raum vorhanden. Diese Ausgestaltung ermöglicht eine weitere Reduzierung des Volumens. Außerdem ist es bei dieser Ausführungsform möglich, die Behältnisse nach der Behandlung durch die Behältnisbehandlungsvorrichtung an eine im Transportpfad folgende Behandlungseinrichtung in derselben Weise abzugeben, wie dies bei den aus dem Stand der Technik bekannten Behandlungsvorrichtungen mit beispielsweise fünf Transporteinrichtungen der Fall ist. Durch die bei dieser Ausführungsform ebenfalls ungerade Anzahl aller Transporteinrichtungen innerhalb des vom Gehäuse umgebenen Raums ist der Drehsinn der entlang des Transportpfads ersten und letzten Transporteinrichtung innerhalb des Gehäuses identisch.

Vorzugsweise weist die mindestens eine weitere Transporteinrichtung einen rotierbaren Träger auf. Somit ist sie besonders einfach mit der Behältnisaußenflächenbehandlungseinrichtung und/oder der Behältnisinnenflächenbehandlungseinrichtung kombinierbar und kann Behältnisse an diese übergeben und/oder von dieser aufnehmen. Insbesondere ist bevorzugt, dass die weitere Transporteinrichtung ein Transportstern ist. Derartige Transportsterne sind aus dem Stand der Technik bekannt und die Einbindung in eine Behältnisbehandlungsvorrichtung mit vertretbarem Aufwand möglich.

Bevorzugt umschließt das Gehäuse einen Reinraum. Dies ist insbesondere bevorzugt, wenn es sich bei der Behältnisbehandlungsvorrichtung um eine Sterilisationsvorrichtung handelt. So kann innerhalb des Gehäuses zumindest ein Teilbereich steril gehalten werden und eine Kontamination der innerhalb des Gehäuses sterilisierten Behältnisse vermieden werden.

Alternativ oder ergänzend dazu ist bevorzugt, dass das Gehäuse eine Strahlungsbarriere ist. Wird zur Sterilisation einer Behältnisoberfläche eine Strahlung eingesetzt, kann so der Austritt dieser Strahlung oder daraus resultierender Streustrahlung aus dem Gehäuse vermieden werden. Dies dient insbesondere dem Schutz von Personen, die sich im Umfeld einer solchen Behältnisbehandlungsvorrichtung aufhalten.

Vorzugsweise ist ein Abstand zwischen zwei auf dem Transportpfad unmittelbar aufeinander folgenden Behältnissen im Bereich der Behältnisaußenflächenbehandlungseinrichtung und/oder der Behältnisinnenflächenbehandlungseinrichtung veränderbar. Dies ermöglicht, dass das Behältnis in einer individuell einstellbaren Geschwindigkeit an der Behältnisbehandlungseinrichtung vorbeizuführen. Somit kann die Behandlung auf die benötigte Dauer angepasst werden, ohne dass die Geschwindigkeit des gesamten Trägers, an welchem die Halteeinrichtung angeordnet ist, angepasst werden muss. Dadurch ergibt sich die Möglichkeit eines hohen Durchsatzes bei gleichzeitig ausreichender Behältnisbehandlungsdauer.

Vorzugsweise ist eine Halteeinrichtung der Behältnisaußenflächenbehandlungseinrichtung eine das Behältnis innengreifende Halteeinrichtung und/oder eine Halteeinrichtung der Behältnisinnenflächenbehandlungseinrichtung eine das Behältnis außengreifende Halteeinrichtung. Diese Ausgestaltungen ermöglichen es, dass das Behältnis zumindest an einer Behältnisbehandlungseinrichtung, auf einer Seite von einer Halteeinrichtung an einer Behältnisoberfläche gehalten wird, die der zu behandelnden Behältnisoberfläche gegenüber liegt. Damit müssen keine Teile der Halteeinrichtung die zu behandelnde Behältnisoberfläche kontaktieren, so dass diese für die von der Behältnisbehandlungseinrichtung vorzunehmenden Behandlung frei zugänglich ist. Beispielsweise ist es dadurch möglich, die gesamte zu behandelnde Oberfläche mit einem Sterilisationsmedium wie einer Sterilisationsmittellösung oder sterilisierender Strahlung zu beaufschlagen.

Als Behältnis soll im Folgenden jegliches Behältnis verstanden werden, welches zur Aufnahme eines Mediums geeignet ist. Sofern auf ein erstes Behältnis und ein zweites Behältnis Bezug genommen wird, können diese identisch oder verscheiden sein. Identische Behältnisse können jedoch verschiedene Inhalte aufweisen. Handelt es sich bei der Behandlungsvorrichtung beispielsweise um eine Füllvorrichtung, kann ein erstes Behältnis ein gasförmiges Medium enthalten, das zweite Behältnis eine Flüssigkeit oder ein anderes Gas. Auch wäre denkbar, dass es sich bei der Behandlungsvorrichtung um eine Sterilisationsvorrichtung handelt. Ein erstes Behältnis könnte in diesem Fall beispielsweise ein unsteriles Behältnis sein und das zweite Behältnis ein steriles Behältnis.

Bevorzugt handelt es sich bei dem zu behandelnden (ersten und/oder zweiten) Behältnis um Flaschen und/oder Vorformlinge. Die Behandlungsvorrichtung ist bevorzugt eine Sterilisationsvorrichtung beziehungsweise ein Sterilisationsmodul. Denkbar wäre jedoch auch, dass die Behandlungsvorrichtung auch mindestens eine Behandlungseinrichtung aufweist, die ausgewählt ist aus einer Gruppe, die Verschlusseinrichtung, Blasstation, Befülleinrichtung, Heizeinrichtung, Kühleinrichtung und Etikettiereinrichtung umfasst.

Vorzugsweise sind die Behältnisaußenflächenbehandlungseinrichtung und die Behältnisinnenflächenbehandlungseinrichtung entlang des Transportpfads unmittelbar aufeinander folgend angeordnet. Dadurch kann auf weitere Transporteinrichtungen zwischen diesen beiden Behältnisflächenbehandlungseinrichtungen verzichtet werden, was eine kompaktere Bauform der Behältnisbehandlungsvorrichtung ermöglicht. Darüber hinaus werden zusätzliche zu wartende Teile in diesem Abschnitt vermieden, so dass der Wartungsaufwand sinkt, der einen Stillstand der Behältnisbehandlungsvorrichtung sowie das Öffnen des Gehäuses und eine daraus eventuell resultierende Kontamination des vom Gehäuse umschlossenen Raums bewirken kann.

Bevorzugt folgt die Behältnisinnenflächenbehandlungseinrichtung entlang des Transportpfads der Behältnisaußenflächenbehandlungseinrichtung. Daher ist bevorzugt, dass die Behältnisaußenflächenbehandlungseinrichtung und die Behältnisinnenflächenbehandlungseinrichtung insbesondere für eine Übergabe eines Behältnisses von Behältnisaußenflächenbehandlungseinrichtung an die Behältnisinnenflächenbehandlungseinrichtung vorgesehen und eingerichtet sind. Dies hat beispielsweise bei einer Sterilisation den Vorteil, dass nach der Sterilisation von zumindest eines Abschnitts der Außenfläche, bei welcher das Behältnis vorzugsweise von einem innengreifenden Haltelement gehalten wird, das Behältnis nicht erneut innenseitig gegriffen werden muss. Dadurch können Kontaminationen des Behältnisinnenraums vermieden werden.

In einer bevorzugten Ausführungsform weist die Behältnisaußenflächenbehandlungseinrichtung und/oder die Behältnisinnenflächenbehandlungseinrichtung einen rotierbaren Träger und eine Vielzahl von an diesem Träger angeordneten und auf einer Kreisbahn und/oder auf einer Umlaufbahn mit variablem Abstand zum Mittelpunkt führbaren Halteelementen auf. Im Folgenden soll unter "Kreisbahn" auch eine wie oben erwähnte Umlaufbahn um einen Mittelpunkt verstanden werden, bei dem in einem oder mehreren Sektoren geringfügige Abweichungen von der idealen Kreisbahn vorliegen. Diese können beispielsweise wie im Folgenden beschrieben ist dadurch auftreten, dass der Abstand einzelner Haltelemente vom Mittelpunkt individuell verändert wird, um beispielsweise die Kreisbahn in bestimmten Bereichen abzuflachen oder sogar eine weitgehend lineare Führung von Halteelementen und den daran angeordneten Behältnissen in einem Sektor zu ermöglichen. Vorzugsweise weist ein Haltelement in einem ersten Sektor dieser Kreisbahn, in dem eine Aufnahme eines Behältnisses erfolgt, eine Höhe gegenüber einer senkrechten Projektion des Transportpfads auf, welche von derjenigen in einem zweiten Sektor verschieden ist, in welchem eine Abgabe eines Behältnisses erfolgt. Dadurch wird ermöglicht, dass die Übernahme eines Behältnisses (bezüglich der senkrechten Projektion des Transportpfads) auf einer anderen Höhe erfolgen kann als dessen Abgabe. Dies kann vorteilhaft sein, wenn die entlang des Transportpfads benachbarten Transporteinrichtungen auf verschiedenen Höhenniveaus sind.

Insbesondere bietet dies jedoch dann einen Vorteil, wenn die Behandlung durch die Behältnisaußenflächenbehandlungseinrichtung und/oder die Behältnisinnenflächenbehandlungseinrichtung eine Höhenverschiebung des Behältnisses vorsieht. In diesem Fall ist es nämlich nicht notwendig, dass das Behältnis mehrfach in der Höhe verschoben wird, beispielsweise hoch und wieder runter in die Ausgangsposition. Vielmehr ist denkbar, dass während der Behandlung die Höhenverschiebung lediglich über die für den Prozess unbedingt notwendige Strecke erfolgt und die Halteeinrichtung erst nach Abschluss des Prozesses und der Abgabe des behandelten Behältnisses wieder auf diejenige Höhe verschoben wird, in der ein weiteres Behältnis aufgenommen wird. Dadurch ist es insbesondere bei rotierenden Behältnisflächenbehandlungseinrichtungen möglich, dieses Verschieben des nicht von einem Behältnis besetzten Halteelements in einem Sektor einer Kreisbahn vorzunehmen, in welchem keine Behandlung eines Behältnisses vorgenommen wird und welcher somit ungenutzt bleiben würde.

Vorteilhaft ist, wenn die Behältnisaußenflächenbehandlungseinrichtung und die Behältnisinnenflächenbehandlungseinrichtung entlang des Transportpfads der Behältnisse unmittelbar aufeinander folgend angeordnet sind. Da bei einer Behältnisflächenbehandlung oftmals eine Verschiebung des Behältnisses entlang der Höhenrichtung vorgesehen ist, weist eine Behältnisflächenbehandlungseinrichtung oftmals ohnehin eine Einrichtung zum Verschieben des Halteelements entlang der Höhenrichtung auf, so dass dies besonders einfach umsetzbar ist.

Insbesondere ist bevorzugt, dass die Behältnisaußenflächenbehandlungseinrichtung und die Behältnisinnenflächenbehandlungseinrichtung für eine Übergabe eines Behältnisses von Behältnisaußenflächenbehandlungseinrichtung an die Behältnisinnenflächenbehandlungseinrichtung vorgesehen und eingerichtet sind. Vorzugsweise folgt somit die Behältnisinnenflächenbehandlungseinrichtung entlang des Transportpfads der Behältnisaußenflächenbehandlungseinrichtung. Dies bietet oftmals einen Vorteil, wenn zur Behältnisaußenflächenbehandlung innengreifende Halteelemente eingesetzt werden. Dabei hängt das Behältnis üblicherweise unterhalb eines Arms, an dem das innengreifende Halteelement angeordnet ist. Um eine Berührung von Armen der verschiedenen Behältnisflächenbehandlungseinrichtungen zu vermeiden, bietet es sich daher an, dass die Übergabe an die Behältnisinnenflächenbehandlungseinrichtung auf einem vergleichsweis niedrigen Höhenniveau erfolgt.

Vorzugsweise weist ein Haltelement der Behältnisinnenflächenbehandlungseinrichtung in dem ersten Sektor bezüglich einer senkrechten Projektion des Transportpfads eine geringere Höhe auf als in dem zweiten Sektor. Wie oben beschrieben bietet es sich an, dass die Behältnisinnenflächenbehandlungseinrichtung ein Behältnis auf einem vergleichsweise niedrigen Höhenniveau aufnimmt. Die Höhe einer senkrechten Projektion des Transportpfads im Sektor der Behältnisaufnahme ist somit gering. Vorzugsweise erfolgt während der Behältnisinnenflächenbehandlung ein Verschieben des Behältnisses in der Höhenrichtung. Sofern eine (vollständige) Rückführung des Behältnisses auf die ursprüngliche Höhe (also diejenige mit der es durch die Behältnisinnenflächenbehandlungseinrichtung aufgenommen wurde) für die Behältnisinnenflächenbehandlung nicht notwendig ist, bietet die nicht vollständige Rückführung des Behältnisses auf die ursprüngliche Höhe einen Vorteil. Dieser besteht darin, dass bei gleicher Rotationsgeschwindigkeit des Trägers die Behältnisinnenflächenbehandlung über einen längeren Streckenabschnitt der Kreisbahn erfolgen kann, da die Rückführung des dann unbesetzten Halteelements auf die ursprüngliche Höhe nach Abgabe des innenbehandelten Behältnisses erfolgen kann.

Vorzugsweise ist ein Haltelement der Behältnisinnenflächenbehandlungseinrichtung in einem dritten Sektor, welcher entlang des Transportpfads zwischen dem ersten und zweiten Sektor liegt, zumindest abschnittsweise bezüglich einer senkrechten Projektion des Transportpfads auf einer größeren Höhe bezüglich einer senkrechten Projektion des Transportpfads angeordnet als in dem ersten Sektor und als in dem zweiten Sektor. Folglich ist das von dem Halteelement gehaltene Behältnis im dritten Sektor bei dieser Ausführungsform zumindest zeitweise auf einer Höhe angeordnet, die sowohl oberhalb der Höhe liegt, auf dem das Behältnis von der Behältnisinnenflächenbehandlungseinrichtung aufgenommen wird als auch derjenigen, auf der das Behältnis abgegeben wird. Dies ist insbesondere dann vorteilhaft, wenn das Behältnis zur Behältnisinnenflächenbehandlung entlang einer Beaufschlagungseinrichtung geführt wird und dabei in der Höhe verändert wird. Dies ermöglicht insbesondere die Beaufschlagung verschiedener Höhenbereiche des Behältnisses durch die (in ihrer Höhenposition vorzugsweise unbewegliche) Beaufschlagungseinrichtung.

Insbesondere ist bevorzugt, dass in einem dritten Sektor zumindest abschnittsweise eine Behältnisbehandlungseinrichtung zumindest abschnittsweise im inneren eines Behältnisses angeordnet ist. Bei einer solchen Behältnisbehandlungseinrichtung kann es sich beispielsweise um eine Düse oder einen sogenannten Strahlfinger handeln, welcher sterilisierende Strahlung emittiert und eine Behältnisoberfläche damit beaufschlagt. Derartige Strahlfinger sind oft sehr empfindlich und mit einer Strahlung und/oder Hochspannungsquelle verbunden. Es ist zwar möglich, einen solchen Strahlfinger beweglich bezüglich eines Trägers auszubilden, jedoch ist dies aufgrund der Empfindlichkeit und des Gewichts weniger bevorzugt. Vielmehr ist bevorzugt, dass die Behältnisbehandlungseinrichtung bezüglich der senkrechten Projektion des Transportpfads eine konstante Höhe aufweist. Es ist üblicherweise wesentlich einfacher, ein kostengünstiges, üblicherweise leichtes und wenig empfindliches Behältnis bezüglich der Behältnisbehandlungseinrichtung wie einem (bezüglich des gemeinsamen Trägers feststehenden) Strahlfinger zu bewegen, als die oft aufwändige und teure Behältnisbehandlungseinrichtung auf das Behältnis zuzustellen, um eine für die Behältnisbehandlung notwendige Relativbewegung zwischen Behältnisbehandlungseinrichtung und Behältnis zu erzeugen.

Insbesondere ist bevorzugt, dass das Behältnis entlang dessen Längsachse auf die Behältnisbehandlungseinrichtung, beispielsweise einen Strahlfinger, zugestellt wird, wobei vorzugsweise die Behältnisbehandlungseinrichtung zumindest abschnittsweise in das Innere des Behältnisses eindringt. Anschließend wird das Behältnis wieder von der Behältnisbehandlungseinrichtung entfernt, so dass letztere wieder vollständig außerhalb des Behältnisses liegt.

Bevorzugt ist eine Behältnisaufnahme beziehungsweise eine Halteeinrichtung ein passives Element, beispielsweise eine Klammer. Aktive Einrichtungen, die zur Übernahme und Übergabe notwendig sind, beispielsweise zum Eindrücken eines Vorformlings in eine Klammer und das Herausziehen aus einer Klammer entgegen der Haltekraft der Klammer, sind bevorzugt auf die Übergabeeinrichtung und die Übernahmeeinrichtung ausgelagert. In einer bevorzugten Ausführungsform ist eine Halteeinrichtung der Behältnisaußenflächenbehandlungseinrichtung und/oder der Behältnisinnenflächenbehandlungseinrichtung ein aktives Element. Bei einem solchen aktiven Element ist vorzugsweise die Kraft, mit der ein Behältnis gegriffen wird, veränderbar. Beispielsweise kann diese Veränderung sektorabhängig erfolgen. Diese Ausführungsform ist bevorzugt, da mit den aktiven Haltelementen / Klammern eine bessere und sicherere Übernahme und/oder Übergabe gewährleistet werden kann.

Weiterhin wird die der Erfindung zugrundeliegende Aufgabe durch ein Verfahren zur Behandlung von Behältnissen gelöst, bei welchem die Behältnisse entlang eines vorgegebenen Transportpfads durch einen vom Gehäuse umgebenen Raum transportiert werden. Die Behältnisse werden dabei mindestens abschnittsweise durch eine Behältnisaußenflächenbehandlungseinrichtung und eine Behältnisinnenflächenbehandlungseinrichtung transportiert, wobei sie während des Transports und/oder einer Behältnisbehandlung jeweils von mindestens einer Halteeinrichtung gehalten werden.

In einer ersten Variante dieses Verfahrens wird die Lösung der oben genannten Aufgabe dadurch erreicht, dass eine Halteeinrichtung der Behältnisaußenflächenbehandlungseinrichtung oder der Behältnisinnenflächenbehandlungseinrichtung zumindest zeitweise in den Bereich einer Öffnung einer Gehäusewandung gebracht wird, um ein in den vom Gehäuse umgebenen Raum zu bringendes Behältnis aufzunehmen oder ein aus den vom Gehäuse umgebenen Raum auszuführendes Behältnis abzugeben. Wie oben bereits bezüglich der Vorrichtung beschrieben wurde, bietet dies die Möglichkeit, die Anzahl der innerhalb des Gehäuses angeordneten Transporteinrichtungen zu reduzieren und das vom Gehäuse umschlossene Volumen gering zu halten.

Vorzugsweise erfolgt eine Übergabe eines Behältnisses zwischen der Behältnisaußenflächenbehandlungseinrichtung und der Behältnisinnenflächenbehandlungseinrichtung. Dadurch kann auf eine zwischen der Behältnisaußenflächenbehandlungseinrichtung und der Behältnisinnenflächenbehandlungseinrichtung angeordnete Transporteinrichtungen verzichtet werden, was ebenfalls dazu dienen kann, das vom Gehäuse umschlossene Volumen gering zu halten. Insbesondere ist bevorzugt, dass ein Behältnis von der Behältnisaußenflächenbehandlungseinrichtung an die Behältnisinnenflächenbehandlungseinrichtung übergeben wird.

Vorzugsweise wird eine Höhe eines Behältnisses über einer senkrechten Projektion des Transportpfads innerhalb des vom Gehäuse umgebenen Raums mindestens einmal, bevorzugt mindestens zweimal, insbesondere bevorzugt mindestens dreimal verändert. Der Transportpfad erstreckt sich somit nicht lediglich über zwei Dimensionen, sondern nutzt auch eine dritte Dimension, insbesondere bevorzugt eine Höhenrichtung. Dies ist beispielsweise vorteilhaft, um verschiedene Bereiche eines Behältnisses in einen Beaufschlagungsbereich einer Behandlungseirichtung zu bringen. Wie oben bereits vorrichtungsseitig beschrieben ist, kann dadurch beispielsweise das Behältnis relativ zu einem Strahlfinger bewegt werden, um diesen abschnittsweise in das Innere des Behältnisses einzubringen und dort für die Beaufschlagung der inneren Behältnisoberfläche mit Strahlung aus kurzer Distanz zu sorgen. Eine mehrfache Verschiebung eines Behältnisses entlang der Höhenrichtung kann vorteilhaft oder sogar notwendig sein, beispielsweise ein Absenken des zuvor angehobenen Behältnisses, um den Strahlfinger wieder aus dem Behältnisinneren zu entfernen.

Eine weitere Lösung der oben definierten Aufgabe ergibt sich, wenn die Behältnisse zumindest abschnittsweise durch eine Halteeinrichtung der Behältnisaußenflächenbehandlungseinrichtung und/oder die Behältnisinnenflächenbehandlungseinrichtung auf einem Abschnitt des Transportpfads geführt werden, dessen senkrechte Projektion ein Abschnitt einer Kreisbahn ist, wobei ein Behältnis in einem ersten Sektor von einem Haltelement auf einer ersten Höhe bezüglich der senkrecht Projektion des Transportpfads aufgenommen wird und in einem zweiten Sektor auf einer von der ersten Höhe verschiedenen zweiten Höhe abgegeben wird.

Diese und alle weiteren dargestellten Lösungen können ergänzend oder alternativ zu der oben beschriebenen ersten Lösung verwirklicht sein. Auch die im Rahmen bevorzugter Varianten beschriebenen Verfahrensschritte und Ausführungsformen der beschriebenen Vorrichtung sind nicht auf diejenige Lösung beschränkt, in deren Zusammenhang sie erstmals erwähnt sind. Vielmehr können bevorzugte Verfahrensschritte und Ausführungsformen auch Vorteile für Verfahren und/oder Vorrichtungen bringen, die nicht im unmittelbaren Zusammenhang mit dieser Variante beschrieben wurden, sofern diese Kombination technisch realisierbar ist.

So ist beispielsweise die oben bezüglich der ersten Lösung der Aufgabe beschriebene einmalige oder mehrmalige Veränderung der Höhe eines Behältnisses über einer senkrechten Projektion des Transportpfads innerhalb des vom Gehäuse umgebenen Raum auch eine bevorzugte Verfahrensvariante der erst danach beschriebenen weiteren Lösung der Aufgabe.

In der oben genannten weiteren Lösung der Aufgabe bietet die Veränderung der Höhe eines Behältnisses von der Höhe im ersten Sektor zu der Höhe im zweiten Sektor den Vorteil, dass eine für die Behältnisbehandlung nicht zwingend erforderliche Höhenveränderung des (dann nicht von einem Behältnis besetzten) Haltelements in einem vierten Sektor der Kreisbahn, entlang der das Haltelement bewegt wird, vorgenommen werden kann. Dadurch kann die für diese Verschiebung notwendige Zeit aus dem durch die Transportgeschwindigkeit und den Durchmesser des Trägers definierten Sektor, in dem die Behältnisbehandlung stattfindet, in den vierten Sektor verschoben werden. Die Zeit, in der sich ein Halteelement in diesem vierten Sektor befindet, wird üblicherweise als Totzeit bezeichnet, da in diesem Bereich keine für die Behältnisbehandlung notwendige Tätigkeit durchgeführt werden kann. Die Möglichkeit, diesen Sektor für eine für die Behältnisbehandlung notwendige Tätigkeit, nämlich die Rückführung der Halteeinrichtung auf die im ersten Sektor notwendige Höhe, zu nutzen, ermöglicht im Umkehrschluss einen geringeren Radius / Durchmesser des Trägers und damit eine Verringerung des Volumens des vom Gehäuse umschlossenen Raums.

Vorzugsweise nimmt die Halteeinrichtung der Behältnisaußenflächenbehandlungseinrichtung und/oder die Behältnisinnenflächenbehandlungseinrichtung im ersten Sektor das Behältnis von einer Behältnisaußenflächenbehandlungseinrichtung oder Behältnisinnenflächenbehandlungseinrichtung auf. Diese Verfahrensvariante bietet - wie oben bereits beschrieben - die Möglichkeit, auf eine zusätzliche Transporteinrichtung zwischen Behältnisaußenflächenbehandlungseinrichtung und die Behältnisinnenflächenbehandlungseinrichtung zu verzichten, was ebenfalls zu einer Verringerung des Volumens des vom Gehäuse umschlossenen Raums führt. Darüber hinaus ist eine Verschiebung eines Behältnisses entlang der Höhenrichtung im Bereich der Behältnisaußenflächenbehandlungseinrichtung und/oder der Behältnisinnenflächenbehandlungseinrichtung oftmals vorteilhaft, um eine Behandlung der gesamten Innen- beziehungsweise Außenfläche des Behältnisses zu ermöglichen. Dementsprechend weist zumindest eine dieser Behältnisflächenbehandlungseinrichtungen eine Einrichtung zur Verschiebung des Behältnisses entlang der Höhenrichtung auf, so dass diese im Rahmen dieser Variante ebenfalls genutzt werden kann.

In einer bevorzugten Variante wird das Behältnis im zweiten Sektor in einer zweiten Höhe abgegeben, die (bezüglich einer senkrechten Projektion des Transportpfads auf eine Ebene) größer ist als die erste Höhe, in der das Behältnis im ersten Sektor aufgenommen wird. Insbesondere bei einer Übergabe von einem innengreifenden Halteelement, bei welchem das Behältnis üblicherweise unterhalb eines Arms hängt, an ein außengreifendes Halteelement bietet sich eine Aufnahme auf einem vergleichsweise niedrigen Höhenniveau an, um eine Berührung von Armen und/oder Haltelementen der verschiedenen Behältnisflächenbehandlungseinrichtungen zu vermeiden.

Vorzugsweise wird das Behältnis in einem dritten Sektor, der auf dem Transportpfad zwischen dem ersten und dem zweiten Sektor angeordnet ist, durch eine Behältnisbehandlungseinrichtung mit einem Behandlungsmittel beaufschlagt. Wie oben dargelegt, kann durch die Durchführung einer nicht unmittelbar zur Behältnisbehandlung notwendigen Verschiebung des (nicht von einem Behältnis besetzten) Haltelements in einen Bereich außerhalb dieses dritten Sektors, der dritte Sektor in einem größeren Umfang (bevorzugt nahezu vollständig) für die unmittelbar zur Behältnisbehandlung notwendigen Prozessschritte genutzt werden.

In einer bevorzugten Verfahrensvariante wird die Halteeinrichtung in einem vierten Sektor, der nicht Teil des Transportpfads des Behältnisses ist, von der zweiten Höhe auf die erste Höhe verschoben, in der das Behältnis im ersten Sektor aufgenommen wird. Die Halteeinrichtung ist in diesem vierten Sektor wie oben beschrieben bevorzugt nicht mit einem Behältnis besetzt.

Vorzugsweise wird das Behältnis während der Behältnisinnenflächenbehandlung in der Höhenrichtung verschoben. Insbesondere ist bevorzugt, dass zumindest ein Teil des Behältnisses dabei auf eine Behandlungseinrichtung, beispielsweise eine Düse oder eine Strahlungsquelle, zugestellt wird. Insbesondere ist im Bereich der Behältnisinnenflächenbehandlung bevorzugt, dass das Behältnis über einen (bezüglich des Trägers vorzugsweise unbeweglichen (Strahl-) Finger gestülpt wird, sodass der Finger zumindest abschnittsweise in das Innere des Behältnisses hineinragt.

Insbesondere wenn bei der Übernahme eines Behältnisses durch die Behältnisinnenflächenbehandlungseinrichtung ein Behältnis nach unten von einem innengreifenden Halteelement abgezogen werden muss, ergibt es sich oftmals, dass die Wegstrecke, die in Höhenrichtung vom Behältnis zurückgelegt werden muss, damit ein Strahlfinger bis in den Bereich, der die effektive Sterilisation des Behältnisbodens ermöglicht, in das Behältnis eindringt, größer ist als diejenige, die beim Abziehen des Behältnisses vom Strahlfinger notwendigerweise zurückgelegt werden muss, damit der Strahlfinger vollständig aus dem Behältnisinneren entfernt ist. Sobald der Strahlfinger jedoch vollständig außerhalb des Behältnisses angeordnet ist, ist eine Übergabe des Behältnisses an eine im Transportpfad folgende Transporteinrichtung möglich. Das Verschieben des (dann unbesetzten) Halteelements kann nach der Abgabe des Behältnisses erfolgen.

Das oben beschriebene Verfahren mit seinen bevorzugten Varianten ermöglicht es, den Transportpfad eines Behältnisses im Bereich einer Behältnisflächenbehandlungseinrichtung zu reduzieren, da für die Behältnisbehandlung nicht unmittelbar notwendige Prozessschritte in Bereichen außerhalb des Transportpfads der Behältnisse ausgeführt werden. Dementsprechend kann der Anteil des Transportpfads der Behandlungsvorrichtung, in welchem eine Behandlung des Behältnisses stattfindet, erhöht werden. Dadurch kann die Behandlungsvorrichtung insgesamt kleiner gestaltet oder die Anzahl der parallel möglichen Behandlungen erhöht werden. Bei gleicher Leistung ist eine Gewichts- und Durchmesserreduzierung möglich.

Bevorzugt ist vorgesehen, dass die Halteeinrichtung auf dem beweglichen Träger zumindest abschnittsweise auf einer Kreisbahn geführt wird. Wie oben ausgeführt und in einigen der unten beschriebenen Figuren dargestellt ist der bewegliche Träger bevorzugt ein Transportstern, der um eine zentrale Achse rotierbar ist. Dadurch, dass das Halteelement an dem beweglichen Träger angeordnet ist, folgt es dieser Rotation. Wie oben beschrieben, kann ein Halteelement jedoch zusätzlich bezüglich des beweglichen Trägers in senkrechter oder radialer Richtung bewegt werden.

Bevorzugt erstreckt sich eine Längsachse eines Behältnisses während dessen Transports entlang des Transportpfads im Wesentlichen Senkrecht zu einer durch den Transportpfad aufgespannten Ebene. Das Behältnis erstreckt sich somit in einer Höhenrichtung, die senkrecht zum Transportpfad beziehungsweise der durch den Transportpfad aufgespannten Ebene steht. Vorzugsweise ist die Senkrechte der senkrechten Projektion des Transportpfads parallel zu der Höhe eines, bevorzugt jedes Behältnisses. Die Höhenrichtung über der senkrechten Projektion des Transportpfads ist somit parallel zu der Höhenrichtung des oder der Behältnisse.

Eine weitere Lösung der der vorliegenden Erfindung zugrundeliegenden Aufgabe, den Transportpfad für Kunststoffbehältnisse innerhalb einer Behältnisbehandlungsvorrichtung zu verkürzen besteht in einer Behältnisbehandlungsvorrichtung zum Transportieren von Kunststoffbehältnissen und insbesondere Kunststoffvorformlingen entlang eines vorgegebenen Transportpfads, wobei die Behältnisbehandlungsvorrichtung wenigstens eine erste Transporteinrichtung aufweist, welche eine Vielzahl von Halteelementen, insbesondere Halteklammern, zum Halten der Kunststoffbehältnisse während des Transport aufweist, und eine zweite Transporteinrichtung, welche eine Vielzahl von Halteelementen, insbesondere Dornen bzw. Haltedornen aufweist, und die Kunststoffbehältnisse von der ersten Transporteinrichtung an die zweite Transporteinrichtung übergeben werden.

Bei dieser Lösung - die jedoch auch eine Ergänzung zu weiteren im Rahmen dieser Erfindung dargestellten Lösungen und Ausführungsformen sein kann - handelt es sich bei der ersten Transporteinrichtung und bei der zweiten Transporteinrichtung jeweils um einen Teilungsverzugsstern. Bevorzugt handelt es sich demnach bei der ersten Transporteinrichtung um einen (ersten) Teilungsverzugsstern und bei der zweiten Transporteinrichtung um einen (zweiten) Teilungsverzugsstern. Ein Teilungsverzugsstern ist dabei dazu geeignet und bestimmt die Teilung aufeinanderfolgender bzw. benachbart transportierter Kunststoffbehältnisse zu verändern und insbesondere zu vergrößern und/oder zu verringern. Bevorzugt wird dies dadurch erreicht, dass die Halteelemente der ersten Transporteinrichtung und die Halteelemente der zweiten Transporteinrichtung beweglich und/oder schwenkbar an der jeweiligen Transporteinrichtung / dem jeweiligen Teilungsverzugsstern gelagert sind und insbesondere radial oder tangential gegenüber dem Transportpfad der jeweiligen Transporteinrichtung schwenkbar gelagert sind.

Teilungsverzugssterne werden demnach immer dort eingesetzt, wo Teilungen variiert bzw. verändert werden sollen. Sind noch andere Funktionen nötig, werden diese in der Regel nicht auf dem Teilungsverzugsstern ausgeführt, sondern es sind weitere Transportsterne oder Transporteinrichtungen vor und/oder nach den Teilungsverzugssternen notwendig. Beispielsweise sind bei einer aus dem internen Stand der Technik der Anmelderin bekannten Vorrichtung zwei Transportsterne notwendig, um die Behältnisse mit Haltedornen für eine Außenbehandlung aufzunehmen und nachgelagert ein Teilungsverzugsstern, welcher nur dazu vorgehsehen ist eine Teilung zu ändern. Demnach können aus dem Stand der Technik bekannte Teilungsverzugssterne nur Bewegungen horizontal, d.h. radial und/oder tangential zu einer Ebene, genauer eines Umfangs, des (Teilungsverzugs)Sterns oder bezogen auf eine Längsrichtung des Kunststoffbehältnisses, ausführen, nicht jedoch in anderen Ebenen wie vertikal, d.h. senkrecht zu einer Ebene des (Teilungsverzugs)Sterns bezogen auf den Umfang des (Teilungsverzugs)Sterns oder bezogen auf die Längsrichtung des Kunststoffbehältnisses. Diese Bewegungen werden stattdessen üblicherweise an anderen bzw. den zusätzlichen Transporteinheiten durchgeführt.

Es wird demnach erfindungsgemäß vorgeschlagen, eine Übergabe von Kunststoffbehältnissen direkt von einem Teilungsverzugsstern an einen weiteren Teilungsverzugsstern vorzunehmen ohne, zusätzliche Einlauf- bzw. Auslaufsterne, welche zwischen den Teilungsverzugssternen bzw. der ersten und der zweiten Transporteinrichtung angeordnet sind. Dadurch entfallen innerhalb des Gehäuses der ursprüngliche Behandlungsstern für der Außenentkeimung und der vorgelagerte Einlaufstern. Es ergibt sich somit ein Behandlungsmodul, welches nur mit insgesamt drei (statt fünf) Transporteinrichtungen bzw. Sternen innerhalb des Gehäuses auskommt.

Bevorzugt ist der Behältnisbehandlungsvorrichtung eine Heizvorrichtung vorgeschaltet, welche die Kunststoffbehältnisse und insbesondere Kunststoffvorformlinge auf eine vorgegebene Temperatur erwärmt. Die erste Transporteinrichtung ist vorteilhaft stromabwärts der Heizvorrichtung angeordnet, so dass eine Übergabe der Kunststoffbehältnisse von der Heizvorrichtung an die Behältnisbehandlungsvorrichtung, d.h. von einem Auslaufstern (erste Transporteinrichtung) der Heizvorrichtung an einen Einlaufstern (zweite Transporteinrichtung) der Behältnisbehandlungsvorrichtung mit zwei Teilungsverzugssternen durchgeführt wird ohne, dass zwischen diesen Teilungsverzugssternen weitere Transportsterne oder Transporteinrichtungen angeordnet sind.

Das oben genannte Gehäuse weist bevorzugt eine Einhausung auf, innerhalb welcher bevorzugt ein Reinraum ausgebildet wird. Die erste Transporteinrichtung ist dabei bevorzugt außerhalb des Gehäuses und damit außerhalb des Reinraums angeordnet und die zweite Transporteinrichtung ist bevorzugt innerhalb des Gehäuses und insbesondere innerhalb des Reinraums angeordnet. Die erste Transporteinrichtung übergibt die Kunststoffbehältnisse demnach bevorzugt an die zweite Transporteinrichtung, welche innerhalb eines die Umgebung abschirmenden Gehäuses angeordnet ist. Die zweite Transporteinrichtung bzw. der zweite Teilungsverzugsstern ist demnach bevorzugt aseptisch ausgebildet.

Bei einer bevorzugten Ausführungsform weist die Behälterbehandlungsvorrichtung ein Gehäuse auf, wobei der Transportpfad wenigstens teilweise innerhalb des Gehäuses liegt.

Bei einer bevorzugten Ausführungsform weist die Behältnisbehandlungsvorrichtung eine Behältnisaußenflächenbehandlungseinrichtung zum Behandeln von Außenflächen der Kunststoffbehältnisse, insbesondere eine Behältnisaußenflächensterilisationseinrichtung und/oder eine Behältnisinnenflächenbehandlungseinrichtung zum Behandeln von Innenflächen der Kunststoffbehältnisse, insbesondere eine Behältnisinnenflächensterilisationseinrichtung auf.

Die Behältnisaußenflächenbehandlungseinrichtung und die Behältnisinnenflächenbehandlungseinrichtung sind dabei bevorzugt innerhalb des Gehäuses angeordnet. Bevorzugt sind demnach zumindest die zweite Transporteinrichtung, die Behältnisaußenflächenbehandlungseinrichtung und die Behältnisinnenflächenbehandlungseinrichtung innerhalb des Gehäuses angeordnet, so dass bevorzugt die Behandlung und insbesondere die Sterilisation der Kunststoffbehältnisse innerhalb des Gehäuses bzw. innerhalb des durch das Gehäuse gebildeten Reinraums stattfindet.

Die Behältnisaußenflächensterilisationseinrichtung und die Behältnisinnenflächensterilisationseinrichtung sterilisieren die Behältnisse und insbesondere Vorformlinge bevorzugt mittels Elektronenstrahlen. Die Behältnisaußenflächensterilisationseinrichtung weist hierzu bevorzugt wenigstens einen sogenannten Flächenstrahler auf, welcher bevorzugt entlang des Transportpfads der Behältnisse angeordnet ist und auf deren Außenflächen gerichtet ist und die Behältnisinnenflächensterilisationseinrichtung weist bevorzugt wenigstens eine und bevorzugt eine Vielzahl von Strahlfingern auf, welche in die Behältnisse eingeführt werden.

Bei einer weiteren bevorzugten Ausführungsform ist die Behältnisaußenflächenbehandlungseinrichtung an der zweiten Transporteinrichtung angeordnet. Die Behältnisaußenflächenbehandlungseinrichtung und insbesondere Behältnisaußenflächensterilisationseinrichtung ist demnach bevorzugt direkt an dem (zweiten) Teilungsverzugsstern angeordnet, so dass, wie im Stand der Technik üblich, ein extra für die Außensterilisation bzw. Außenbehandlung vorgesehener Behandlungsstern entfällt. Die Behältnisinnenflächenbehandlungseinrichtung und insbesondere die Behältnisinnenflächensterilisationseinrichtung ist bevorzugt an einem der zweiten Transporteinrichtung nachfolgenden Transportstern angeordnet.

Die erste Transporteinrichtung bzw. der stromabwärts der Heizvorrichtung angeordnete Teilungsverzugsstern übergibt die Kunststoffbehältnisse demnach bevorzugt direkt an einen weiteren Teilungsverzugsstern, an welchem auch direkt eine Außenbehandlung von Kunststoffbehältnissen vorgenommen wird. Im Stand der Technik ist dabei üblicherweise nach dem ersten Teilungsverzugsstern innerhalb des Gehäuses zuerst ein Einlaufstern vorgesehen, welcher die Behältnisse an den nachfolgenden Transportstern für die Außenentkeimung übergibt, welcher die Behältnisse wiederum nach der Außenbehandlung an den zweiten Teilungsverzugsstern übergibt.

Durch die erfindungsgemäße Behältnisbehandlungsvorrichtung bzw. die vorgeschlagene Übergabe von einem Teilungsverzugsstern an direkt einen nachfolgenden Teilungsverzugsstern, an welchen auch die Außenbehandlung vorgenommen wird, entfallen demnach der in dem Gehäuse angeordnete Einlaufstern und der Behandlungs-Transportstern, so dass das Gehäuse insbesondere auch insgesamt kleiner ausgeführt werden kann.

In einer bevorzugten Ausführungsform weist das Gehäuse ein Übergabefenster zum Einschleusen und/oder Übergeben der Kunststoffbehältnisse von der ersten Transporteinrichtung, welche außerhalb des Gehäuses angeordnet ist, an die zweite Transporteinrichtung, welche innerhalb des Gehäuses angeordnet ist, auf. Bevorzugt ist das Übergabefenster in einem Übergabeabschnitt angeordnet, in welchem die Kunststoffbehältnisse von der ersten Transporteinrichtung an die zweite Transporteinrichtung übergeben werden.

An dem Übergabefenster ist die Teilung der Kunststoffbehältnisse bevorzugt variabel, so dass der Teilungsverzug des ersten Teilungsverzugssterns optimal auf die Anforderungen des zweiten Teilungsverzugssterns ausgelegt ist bzw. an diesen anpassbar ist.

Bei einer bevorzugten Ausführungsform weist das Übergabefenster eine Breite auf, die zwischen 250 mm und 320 mm, bevorzugt zwischen 270 mm und 310 mm und besonders bevorzugt zwischen 285 mm und 300 mm liegt. Vorteilhaft ist das Übergabefenster möglichst klein ausgeführt, so dass eine eventuelle Sterilität im Inneren des Gehäuses gewahrt werden kann und gleichzeitigt das Austrittsfenster für mögliche aus dem Gehäuse austretenden Strahlung verringert wird. Bevorzugt ist das Übergabefenster variabel ausgestaltet, so dass dessen Breite veränderbar ist. Dies ist beispielsweise vorteilhaft, da das Übergabefenster so an verschiedene Vorformlingtypen und -größen anpassbar ist. Diese Übergabefenster können auch als Eingang bzw. Ausgang bezeichnet werden

Bei einer weiteren bevorzugten Ausführungsform weist die zweite Transporteinrichtung eine Hub- und Dreheinrichtung auf, welche eine Bewegung des Halteelements der zweiten Transporteinrichtung in vertikaler und/oder horizontaler Richtung bezüglich einer Längsachse des Kunststoffbehältnisses und eine Drehbewegung des Kunststoffbehältnisses und/oder des Halteelements entlang der Längsachse ermöglicht. Bevorzugt ist jedem Halteelement der zweiten Transporteinrichtung eine eigene Hub- und Dreheinrichtung zugeordnet, so dass die Kunststoffbehältnisse unabhängig voneinander gedreht werden können und/oder die Halteelemente unabhängig voneinander bewegt werden können. Jeder Hub- und Dreheinrichtung ist insbesondere zur Ausführung der Drehbewegung des Kunststoffbehältnisses bevorzugt eine Antriebseinrichtung zugeordnet, welche in bekannter Weise ausgeführt ist. Die Hubbewegung des Halteelements in vertikaler Richtung wird bevorzugt durch eine oder mehrere Hubkurven und wenigstens eine Führungskurve realisiert.

Um die Übergabe von der ersten Transporteinrichtung an die zweite Transporteinrichtung und insbesondere von dem ersten Teilungsverzugsstern and den zweiten Teilungsverzugsstern zu ermöglichen ist demnach zusätzlich eine Hub- und Dreheinrichtung an der zweiten Transporteinrichtung / dem zweiten Teilungsverzugsstern angeordnet, welche bevorzugt zur Aufnahme der Kunststoffbehältnisse geeignet und bestimmt ist.

Die Hub- und Dreheinrichtung wird dabei benötigt, um einerseits die Kunststoffbehältnisse aufzunehmen und innen halten zu können, wobei bevorzugt die Übergabe von den Halteelementen und insbesondere Halteklammern der ersten Transporteinrichtung auf die Halteelemente und insbesondere Haltedorne der zweiten Transporteinrichtung durch einen Bewegung der Halteelemente der zweiten Transporteinrichtung auf die Behältnisse zu und demnach bevorzugt in vertikaler Richtung bzw. in der Längsrichtung der Behältnisse erfolgt, so dass das Halteelement in Richtung des Behältnisses bewegt wird. Andererseits dient die Hub- und Dreheinrichtung dazu, die Behältnisse vor der Behältnisaußenflächenbehandlungseinrichtung und insbesondere Behältnisaußenflächensterilisationseinrichtung, um deren Längsachse zu rotieren, so dass eine am Umfang gleichmäßig verteilte Strahlungsleistung und dadurch resultierende gleichmäßige Entkeimungsleistung auf die Behältnisse aufgebracht werden kann.

Die Halteelemente der ersten Transporteinrichtung sind dabei bevorzugt Halteklammern, welche die Kunststoffbehältnisse an einer Außenwandung und insbesondere unterhalb oder oberhalb des Tragrings der Behältnisse halten. Die Haltelemente der zweiten Transporteinrichtung sind bevorzugt Haltedorne, welche in die Behältnisse eingeführt werden und diese an einer Innenwandung halten. Die Kunststoffbehältnisse werden demnach bevorzugt von einem Außen-Halteelement an ein Innen-Halteelement und insbesondere einer Außen-Halteklammer an einen Innen-Haltedorn übergeben.

Vorteilhaft ermöglicht die Hub- und Dreheinrichtung zudem auch das optimale Aufstecken des Behältnisses auf den Haltedorn. Der Haltedorn ist dabei bevorzugt drehbar ausgebildet, so dass eine Drehbewegung des Kunststoffbehältnisses insbesondere bei der Behandlung des Behältnisses eingeleitet werden kann.

Bei einer bevorzugten Ausführungsform ist zumindest abschnittsweise im Bereich des Übergabefensters eine Abschirmeinrichtung angeordnet, welche die Umgebung des Gehäuses gegenüber dem Innenraum des Gehäuses abschirmt. Insbesondere handelt es sich hierbei um eine Strahlungsabschirmungseinrichtung zur Abschirmung von Strahlung der Behältnisaußenflächenbehandlungseinrichtung und/oder der Behältnisinnenflächenbehandlungseinrichtung und insbesondere entstehender Röntgenstrahlung. Es erfolgt demnach bevorzugt eine Übergabe des Kunststoffbehältnisses in ein strahlungsabgeschirmtes Gehäuse.

Bevorzugt wird daher vorgeschlagen die Außensterilisation der Kunststoffbehältnisse und insbesondere Kunststoffvorformlinge an der zweiten Transporteinrichtung und bevorzugt dem zweiten Teilungsverzugsstern anzuordnen. Um dies zu ermöglichen wird bevorzugt die Behältnisaußenflächenbehandlungseinrichtung bzw. der Flächenstrahler an der zweiten Transporteinrichtung bzw. dem zweiten Teilungsverzugsstern angeordnet und die zweite Transporteinrichtung bzw. der zweite Teilungsverzugsstern mit einer zusätzlichen Hub- und Dreheinrichtung ausgestattet. Die Übergabe vom Auslaufstern des Ofens (erste Transporteinrichtung) in den strahlungsabgeschirmten Bereich wird demnach bevorzugt von dem ersten Teilungsverzugsstern vorgenommen. Der zweite Teilungsverzugsstern ist demnach innerhalb des strahlungsabgeschirmten Bereichs, d.h. innerhalb des Gehäuses angeordnet.

Bei einer weiteren bevorzugten Ausführungsform folgen und begleiten die erste Transporteinrichtung und/oder die Halteelemente der ersten Transporteinrichtung während der Übergabe der Kunststoffbehältnisse an die zweite Transporteinrichtung wenigstens teilweise bzw. abschnittsweise den Transportpfad der zweiten Transporteinrichtung und/oder der Halteelemente der zweiten Transporteinrichtungen. In anderen Worten wird das Kunststoffbehältnis bei bzw. während der Übergabe bevorzugt wenigstens zeitweise und abschnittsweise gleichzeitig von dem Halteelement, insbesondere der Halteklammer, der ersten Transporteinrichtung und dem Halteelement, insbesondere dem Haltedorn, der zweiten Transporteinrichtung gehalten.

Bei der Übergabe von der außerhalb des Gehäuses angeordneten ersten Transporteinrichtung an die zweite Transporteinrichtung innerhalb des strahlungsabgeschirmten Gehäuses begleitet die erste Transporteinrichtung bzw. das Halteelement der ersten Transporteinrichtung die Kunststoffbehältnisse bevorzugt wenigstens zeitweise. Die Übergabe ist demnach bevorzugt begleitend ausgeführt.

Um die Übergabe von dem ersten Teilungsverzugsstern (erste Transporteinrichtung) an den zweiten Teilungsverzugsstern (zweite Transporteinrichtung) zu verbessern bzw. das Aufstecken auf die Halteelemente und insbesondere Haltedorne des zweiten Teilungsverzugssterns zu erleichtern und sicherer zu gestalten begleitet der erste Teilungsverzugsstern die Übergabe der Kunststoffbehältnisse und insbesondere Kunststoffvorformlinge bevorzugt abschnittsweise auf einer Kreisbahn, welche der Kreisbahn des zweiten Teilungsverzugssterns entspricht.

Wie oben erwähnt, führt die Hub- und Dreheinrichtung dabei bevorzugt eine Hubbewegung des Halteelements bzw. Haltedorns aus, wobei bevorzugt der Haltedorn auf das Kunststoffbehältnis zu bewegt wird, und in das Behältnis eingeführt wird, um dieses zu halten. Um die Hubbewegung zum Aufstecken des Kunststoffbehältnisses auf den Haltedorn der Hub- und Dreheinrichtung zu ermöglichen, ist bevorzugt innerhalb des Gehäuses eine erste und eine zweite Hubkurve sowie eine Führungsrolle angeordnet, welche das Heben und Senken des Haltedorns ermöglichen.

Dabei verlässt bevorzugt der erste Teilungsverzugsstern seine eigentliche Transportbahn, bei welcher es sich bevorzugt um eine im Wesentlichen kreisförmige Bahn handelt, um die Transportbahn und bevorzugt Kreisbahn des zweiten Teilungsverzugssterns zu begleiten. Der zweite Teilungsverzugsstern bewegt sich bevorzugt auf einer im Wesentlichen exakten Kreisbahn, so dass eine Führungsrolle der Hub- und Dreheinrichtung ohne Querverschleiß bzw. ohne Rutschen auf der Hubkurve abrollen kann. Im Wesentlichen bedeutet dabei, dass die Transportbahn der zweiten Transporteinrichtung bzw. des zweiten Teilungsverzugssterns nur minimal von einem kreisförmigen Pfad abweicht, wobei sich dies auch durch die wechselnden Abstände der Halteelemente zueinander und der unterschiedlichen Abstände der Halteelemente zur Drehachse des Teilungsverzugssterns ergibt. Das Aufstecken des Behältnisses auf den Haltedorn erfolgt somit nicht punktuell, sondern begleitend.

Die Hubkurve ist bevorzugt innerhalb des Gehäuses an einer stationären Seitenwand des Gehäuses angeordnet. Ein unterer Bereich des Gehäuses ist vorteilhaft beweglich ausgebildet und bevorzugt komplett absenkbar, um den Zugang zu Einstell- und Wartungsarbeiten innerhalb des Gehäuses zu ermöglichen. Eine derartige Absenkung ist beispielsweise in DE 10 2013 109 794 A1 näher beschrieben. Die darin beschriebenen Ausführungsformen stellen jeweils auch bevorzugte Ausführungsformen des absenkbaren Gehäuseteils der vorliegenden Erfindung dar. Die Anmelderin behält sich ausdrücklich vor, Merkmale dieser Druckschrift auch zur Definition bevorzugter Ausführungsformen der vorliegenden Erfindung heranzuziehen

Zusätzlich ist innerhalb des Gehäuses und bevorzugt wenigstens teilweise oder abschnittsweise im Bereich des zweiten Teilungsverzugssterns und insbesondere zumindest in dem Bereich des Übergabefensters eine Abschirmungseinrichtung wie insbesondere eine Strahlenschutzwand angeordnet, welche bevorzugt die durch die Behältnisaußenbehandlungseinrichtung und/oder Behältnisinnenbehandlungseinrichtung entstehende schädliche Röntgenstrahlung gegenüber dem Außenbereich des Gehäuses abschirmt.

Bevorzugt ist dabei ein Abschnitt des Gehäuses und insbesondere ein oberer Abschnitt des Gehäuses stationär angeordnet und ein weiterer Abschnitt, insbesondere ein unterer Abschnitt, bei welchem es sich um einen Boden handelt, beweglich und insbesondere absenkbar angeordnet. Bei dem unteren Abschnitt handelt es sich bevorzugt ebenfalls um eine Abschirmungseinrichtung, wie insbesondere eine Strahlenschutzwand. Die Anordnung der beiden Abschirmungseinrichtungen ist dabei derart gestaltet, so dass sich die Hub- und Dreheinrichtung und insbesondere wenigstens eine Führungsrolle der Hub- und Dreheinrichtung durch einen Spalt zwischen den beiden Abschirmungseinrichtungen hindurch auf einer Laufbahn bewegen kann.

Im Bereich des Übergabefensters ist dabei bevorzugt ein Teil der unteren Abschirmungseinrichtung an der Hubkurve angeordnet. Dieser Teil ist demnach bevorzugt bei Einstellungsarbeiten an der Behälterübergabe demontierbar ausgeführt ohne, dass dazu die Befestigung der Hubkurve verstellt werden muss. Bevorzugt sind daher die untere Abschirmungseinrichtung und die Hubkurve gemeinsam demontierbar ausgestaltet. Das bedeutet, dass, um das Einstellen zu ermöglichen bzw. zu erleichtern der Strahlenschutz demontiert werden kann ohne, dass Teile der Übergaben demontiert werden müssen.

Denkbar wäre bevorzugt gemäß einer anderen Ausführungsform auch, dass der zweite Teilungsverzugsstern bei der Übergabe dem ersten Teilungsverzugsstern entgegenfährt bzw. beide gleichzeitig eine Bewegung aufeinander zu machen. Hierdurch könnte das Übergabefenster in dem Gehäuse minimiert werden. Bevorzugt könnten der erste Teilungsverzugsstern und der zweite Teilungsverzugsstern auch elektrisch gesteuert ausgeführt werden, beispielsweise über eine Langstatortechnik oder dergleichen.

Zur Lösung der oben gennannten Aufgabe ist die vorliegende Erfindung weiter auch auf ein Verfahren zum Transportieren von Kunststoffbehältnissen und insbesondere Kunststoffvorformlingen entlang eines vorgegebenen Transportpfads gerichtet, wobei die Kunststoffbehältnisse wenigstens mittels einer ersten Transporteinrichtung, welche eine Vielzahl von Halteelementen, insbesondere Halteklammern, zum Halten der Kunststoffbehältnisse während des Transport aufweist, und einer zweiten Transporteinrichtung, welche eine Vielzahl von Halteelementen, insbesondere Dornen aufweist, transportiert werden und von der ersten Transporteinrichtung an die zweite Transporteinrichtung übergeben werden.

Bei dieser Lösung - die jedoch auch eine Ergänzung zu weiteren im Rahmen dieser Erfindung dargestellten Lösungen und Ausführungsformen sein kann - handelt es sich bei der ersten Transporteinrichtung und bei der zweiten Transporteinrichtung jeweils um einen Teilungsverzugsstern, so dass die Kunststoffbehältnisse von einem Teilungsverzugsstern an einen nachfolgenden Teilungsverzugsstern übergeben werden. Bei der ersten Transporteinrichtung handelt es sich dabei bevorzugt um einen ersten Teilungsverzugsstern und bei der zweiten Transporteinrichtung um einen zweiten Teilungsverzugsstern.

Es wird demnach auch verfahrensseitig vorgeschlagen, eine Übergabe von Kunststoffbehältnissen direkt von einem Teilungsverzugsstern an einen weiteren und insbesondere direkt nachfolgenden Teilungsverzugsstern vorzunehmen ohne, dass zwischen diesen Teilungsverzugssternen weitere Transportsterne wie beispielsweise zusätzliche Einlauf- bzw. Auslaufsterne angeordnet sind.

Bei einem bevorzugten Verfahren ist die zweite Transporteinrichtung dazu geeignet und bestimmt, die Halteelemente (der zweiten Transporteinrichtung) bezogen auf eine Längsachse der Kunststoffbehältnisse entlang einer vertikalen und/oder horizontalen Richtung zu bewegen und die Kunststoffbehältnisse bzw. die Halteelemente um ihre Längsachse zu drehen.

Bei einem weiteren bevorzugten Verfahren wird an der zweiten Transporteinrichtung eine Behandlung und insbesondere Sterilisation von Außenflächen der Kunststoffbehältnissen vorgenommen.

Die oben beschriebenen Vorrichtungen und Verfahren sind dabei insbesondere auch dazu eingerichtet und dafür vorgesehen, dieses beschriebene Verfahren durchzuführen, d. h., dass alle für die obig beschriebenen Vorrichtungen und Verfahren ausgeführten Merkmale ebenso für das hier beschriebene Verfahren offenbart sind und umgekehrt.

Eine weitere Lösung der der vorliegenden Erfindung zugrundeliegenden Aufgabe besteht in einer Behältnisbehandlungsvorrichtung zum Transportieren von Kunststoffbehältnissen und insbesondere Kunststoffvorformlingen entlang eines vorgegebenen Transportpfads, wobei die Behältnisbehandlungsvorrichtung wenigstens eine Transporteinrichtung aufweist, welche eine Vielzahl von Halteelementen aufweist.

Bei dieser Lösung - die jedoch auch eine Ergänzung zu weiteren im Rahmen dieser Erfindung dargestellten Lösungen und Ausführungsformen sein kann - handelt es sich bei der Transporteinrichtung um einen Teilungsverzugsstern, welcher eine Bewegungseinrichtung aufweist, welche dazu geeignet und bestimmt ist eine Bewegung der Halteelemente in wenigstens zwei Ebenen zu ermöglichen.

Bei einer bevorzugten Ausführungsform weist die Behältnisbehandlungsvorrichtung wenigstens eine erste Transporteinrichtung und eine zweite Transporteinrichtung auf, wobei die Kunststoffbehältnisse von der ersten Transporteinrichtung an die zweite Transporteinrichtung übergeben werden.

Durch die Kombination einer direkten Übergabe von einem ersten Teilungsverzugsstern an einen zweiten Teilungsverzugsstern und einer Bewegung der Halteelemente in wenigstens zwei Ebenen wird bevorzugt Platz und Transportweg gespart und es sind zusätzliche Funktionen für den Teilungsverzugsstern möglich sowie zusätzliche Formen der Übergabe.

Auch ohne die Übergabe des oben erwähnten Teilungsverzugsstern an einen weiteren Teilungsverzugssterns kann durch die Bewegung der Halteelemente in mehreren und insbesondere zwei Ebenen eine zusätzliche Funktion auf dem Teilungsverzugsstern geschaffen werden, für welche bisher ein weiterer Transportstern notwendig war und somit der Transport verkürzt werden.

Bei der Bewegung in wenigstens zwei Ebenen handelt es sich bevorzugt um eine Bewegung des Halteelements in einer horizontalen und/oder vertikalen Richtung und/oder eine Rotation des Halteelements. Vertikal bedeutet dabei senkrecht zu der Transporteinrichtung bzw. den Teilungsverzugsstern bezogen auf einen Umfang der Transporteinrichtung oder einen Transportpfad der Kunststoffbehältnisse entlang der Transporteinrichtung oder bezogen auf eine Längsachse des Kunststoffbehältnisses und/oder des Halteelements, bei welchem es sich bevorzugt um einen Haltedorn handelt. Horizontal bedeutet radial und/oder tangential zu dem Umfang der Transporteinrichtung oder einem Transportpfad der Kunststoffbehältnisse entlang der Transporteinrichtung oder bezogen auf eine Längsachse des Kunststoffbehältnisses. Unter der Rotation wird dabei eine Rotation bzw. ein Rotieren des Halteelements um die eigene Längsachse des Halteelements verstanden, wobei durch diese Rotation insbesondere ein von dem Halteelement gehaltenes Kunststoffbehältnis in Rotation um dessen Längsachse versetzt wird.

Bei der Bewegungseinrichtung handelt es sich daher bevorzugt um die oben erwähnte Hub- und Dreheinrichtung. Alle oben genannten Merkmale sind daher auch auf diese Ausführungsform anwendbar bzw. mit dieser Ausführungsform kombinierbar und umgekehrt.

Bei einer bevorzugten Ausführungsform handelt es sich bei der ersten Transporteinrichtung und bei der zweiten Transporteinrichtung jeweils um einen Teilungsverzugsstern, so dass auch bei einer Bewegung des Halteelements in wenigstens zwei Ebenen an dem Teilungsverzugsstern eine Übergabe in der oben beschriebenen Weise von einem ersten Teilungsverzugsstern an einen zweiten Teilungsverzugsstern stattfindet.

Bei einer weiteren bevorzugten Ausführungsform weist dabei auch hier die Behältnisbehandlungsvorrichtung eine Behältnisaußenflächenbehandlungseinrichtung zum Behandeln von Außenflächen der Kunststoffbehältnisse, insbesondere eine Behältnisaußenflächensterilisationseinrichtung und/oder eine Behältnisinnenflächenbehandlungseinrichtung zum Behandeln von Innenflächen der Kunststoffbehältnisse, insbesondere eine Behältnisinnenflächensterilisationseinrichtung auf, wobei bevorzugt die Behältnisaußenflächenbehandlungseinrichtung an der zweiten Transporteinrichtung angeordnet ist.

Die Kombination einer direkten Übergabe von einem ersten Teilungsverzugsstern an einen zweiten Teilungsverzugsstern und einer Bewegung der Halteelemente in wenigstens zwei Ebenen ist dabei insbesondere für eine Außenbehandlung von Kunststoffbehältnissen vorteilhaft. Insbesondere der Teilungsverzug ist dabei notwendig, um die Abstände der Behältnisse für die Außenbehandlung zu optimieren. Insbesondere kann dadurch auch die vertikale Position des Behältnisses während des Umlaufs, zum Beispiel relativ zum Behandlungsfenster zur Optimierung der Bestrahlung, variiert werden.

In einer weiteren bevorzugten Ausführungsform ist die Bewegungseinrichtung dazu geeignet und bestimmt eine Bewegung der Halteelemente in drei Ebenen zu ermöglichen. Das Halteelement kann demnach bevorzugt Bewegungen in der oben beschriebenen horizontalen und vertikalen Richtung ausführen und rotieren.

Bei einer bevorzugten Ausführungsform handelt es sich bei der Vielzahl von Halteelementen um Haltedorne. Diese innengreifenden Dorne sind insbesondere in der Außenbehandlung vorteilhaft, da hierdurch alle Außenflächen für die Behandlung zugänglich sind und nicht durch Halteelemente verdeckt werden.

Bei einer weiteren bevorzugten Ausführungsform weist die Transporteinrichtung wenigstens eine Hubkurve und wenigstens eine Führungsrolle auf, welche gemeinsam dazu geeignet und bestimmt sind, wenigstens eine Bewegung des Halteelements in wenigstens einer Ebene zu ermöglichen. Bei dieser Bewegung handelt es sich dabei um die Bewegung in vertikaler Richtung. Bevorzugt dienen die wenigstens eine Hubkurve und die wenigstens eine Führungsrolle demnach dazu die Bewegung des Halteelements in vertikaler Richtung auszuführen. Die Führungsrolle wird dabei auf der Hubkurve geführt, welche zur Bewegung des Halteelements in vertikaler Richtung und insbesondere einer, vom Erdmittelpunkt aus gesehenen, auf und ab Bewegung des Halteelements entsprechend erhöhte und niedrigere Bereiche aufweist. Die Bewegung in horizontaler Richtung kann in der bisher bekannten Weise an dem Teilungsverzugsstern ausgeführt werden. Denkbar wäre auch, die Vertikalbewegung durch beispielsweise eine Antriebseinrichtung durchzuführen.

Zur Lösung der oben gennannten Aufgabe ist die vorliegende Erfindung dabei auch auf ein Verfahren zum Transportieren von Kunststoffbehältnissen und insbesondere Kunststoffvorformlingen entlang eines vorgegebenen Transportpfads gerichtet, wobei die Kunststoffbehältnisse wenigstens mittels einer Transporteinrichtung, welche eine Vielzahl von Halteelementen, insbesondere Haltedornen aufweist, transportiert werden.

Bei dieser Lösung - die jedoch auch eine Ergänzung zu weiteren im Rahmen dieser Erfindung dargestellten Lösungen und Ausführungsformen sein kann - handelt es sich bei der Transporteinrichtung um einen Teilungsverzugsstern, welcher eine Bewegung der Halteelemente in wenigstens zwei Ebenen ermöglicht.

Bei einem bevorzugten Verfahren ist die Transporteinrichtung dabei dazu geeignet und bestimmt, die Halteelemente in einer vertikalen Richtung und/oder einer horizontalen Richtung zu bewegen und/oder zu rotieren.

Bei einem weiteren bevorzugten Verfahren wird an der Transporteinrichtung eine Behandlung und insbesondere Sterilisation von Außenflächen der Kunststoffbehältnisse vorgenommen.

Die oben beschriebenen Vorrichtungen und Verfahren sind ist dabei insbesondere auch dazu eingerichtet und dafür vorgesehen, dieses beschriebene Verfahren durchzuführen, d. h., dass alle für die obig beschriebenen Vorrichtungen und Verfahren ausgeführten Merkmale ebenso für das hier beschriebene Verfahren offenbart sind und umgekehrt.

Die vorliegende Erfindung ist weiterhin auf eine Behälterbehandlungsvorrichtung und ein entsprechendes Verfahren gerichtet, bei denen eine Behandlung und insbesondere eine Sterilisation von Behältnissen auch unter Verwendung eines sogenannten Teilungsverzugssterns erfolgt. Die Erfindung wird wiederum unter Bezugnahme auf die Sterilisation von Behältnissen beschrieben, ist wird jedoch darauf hingewiesen, dass die Erfindung auch bei anderen Aggregaten wie beispielsweise Druckaggregaten, Etikettiereraggregaten oder Inspektionseinrichtungen angewendet werden kann.

Wie oben erwähnt, weisen die aus dem internen Stand der Technik der Anmelderin bekannten Sterilisationsmodelle teilweise eine hohe Zahl an Transporteinrichtungen beispielsweise fünf Transportsterne auf. Dadurch ergibt sich eine relativ lange Verweilzeit innerhalb des Sterilisationsmoduls.

Der Erfindung liegt daher die Aufgabe zugrunde, die Verweilzeit der Behältnisse im insbesondere im Rahmen der Sterilisation aber auch bei anderen Behandlungen zu reduzieren. Dies wird erfindungsgemäß durch die Gegenstände der unabhängigen Patentansprüche erreicht. Vorteilhafte Ausgestaltungen und Weiterbildungen sind Gegenstand der Unteransprüche.

Eine erfindungsgemäße Behältnisbehandlungsvorrichtung weist eine Transporteinrichtungen zum Transport des Behältnisses entlang eines vorgegebenen Transportpfads auf, wobei die Transportvorrichtung wenigstens eine Transporteinrichtung zum Transportieren des Behältnisses aufweist, sowie wenigstens eine Behältnisbehandlungseinrichtung zum Behandeln des Behältnisses in einer vorgegebenen Weise. Dabei weist diese Transporteinrichtung einen (bevorzugt drehbaren) Träger auf, an dem eine Vielzahl von Halteeinrichtungen zum Halten mindestens eines Behältnisses angeordnet ist. Anstelle dieses drehbaren Trägers kann der Träger auch als Langstator ausgebildet sein, der bevorzugt einen Bestandteil eines Linearmotors ausbildet, wobei die Halteeinrichtungen bevorzugt an gegenüber diesem Langstator bewegliche Shuttles ausgebildet sind (wobei diese Shuttles die Läufer des Linearmotors darstellen).

Bei einer ersten erfindungsgemäßen Ausführungsform sind diese Halteeinrichtungen gegenüber dem Träger derart beweglich, dass ein Abstand zwischen zwei auf dem Transportpfad unmittelbar aufeinanderfolgenden Behältnissen veränderbar ist. Weiterhin ist diese Behältnisbehandlungseinrichtung derart angeordnet, dass sie die von der Transporteinrichtungen transportierten Behältnisse behandeln kann.

In einer weiteren erfindungsgemäßen Ausgestaltung ist die Halteeinrichtung derart drehbar, dass das von dieser Halteeinrichtung gehaltene Behältnis bezüglich seiner Längsrichtung drehbar ist.

Bei einer weiteren erfindungsgemäßen Ausgestaltung wird, wie oben erwähnt anstelle einer Transporteinrichtung mit einem drehbaren Träger ein linearmotorischer Antrieb vorgesehen. Bei dieser Ausführungsform ist ein Langstator mit einer Vielzahl von magnetischen oder magnetisierbaren Elementen vorgesehen, dem gegenüber sich ein oder mehrere Shuttles bewegen können. Auch hier weist bevorzugt dieser Langstator einen im Wesentlichen geradlinigen Abschnitt auf, an dem besonders bevorzugt die Behandlungseinrichtung angeordnet ist. Mit anderen Worten weist bevorzugt die Transporteinrichtung einen als Stator ausgeführten insbesondere stationären Träger auf, an dem eine Vielzahl von Halteeinrichtungen, welche bevorzugt jeweils einen Läufer aufweisen (oder einen solchen ausbilden), zum Halten mindestens eines Behältnisses angeordnet ist.

Es wird darauf hingewiesen, dass durch die drei oben angegebenen erfindungsgemäßen Ausgestaltungen die oben erwähnte Aufgabe erreicht werden kann. Dennoch ist es auch möglich, diese Ausgestaltungen miteinander zu kombinieren, insbesondere die Veränderung eines Abstands zwischen den Halteeinrichtungen mit der Drehbarkeit der Halteeinrichtungen zu kombinieren.

Bei einer bevorzugten Ausführungsform sind die Behältnisse aus einer Gruppe von Behältnissen ausgewählt, welche Kunststoffvorformlinge, Kunststoffflaschen, Glasflaschen und dergleichen enthält. Insbesondere handelt es sich bei den Behältnissen um Kunststoffvorformlinge

Besonders bevorzugt sind die Halteeinrichtungen auf schwenkbaren und/oder linearverschieblichen Armen angeordnet. Besonders bevorzugt sind hier die Halteeinrichtungen oder Bestandteile der Halteeinrichtungen gegenüber diesen schwenkbaren Armen drehbeweglich. Besonders bevorzugt sind die Halteeinrichtungen gegenüber dem Träger schwenkbar. Bei einer weiteren bevorzugten Ausführungsform sind die Halteeinrichtungen auch in einer geradlinigen Richtung gegenüber dem Träger beweglich.

Bei einer bevorzugten Ausgestaltung handelt es sich bei der Transporteinrichtung um einen sogenannten Teilungsverzugsstern, d. h. eine Transporteinrichtung, welche die Teilung der transportierten Behältnisse ändern kann. Bevorzugt ist der Teilungsverzugsstern, in der im Weiteren oder obenstehend im Rahmen dieser Anmeldung geführten Weise ausgeführt.

Besonders bevorzugt, findet die Behandlung der Behältnisse in einem geradlinigen Abschnitt des Transportpfads statt. Besonders bevorzugt ist die Behältnisbehandlungseinrichtung stationär angeordnet und die Behältnisse bewegen sich an dieser vorbei.

Besonders bevorzugt ist der Abstand zwischen zwei auf dem Transportpfad unmittelbar aufeinanderfolgenden Behältnisse während der Bewegung der Behältnisse veränderbar. Besonders bevorzugt sind durch die Transporteinrichtung transportierte Behältnisse wenigstens abschnittsweise entlang einer geraden Linie transportierbar.

Bei einer weiteren vorteilhaften Ausführungsform ist die Behältnisbehandlungseinrichtung dazu geeignet und bestimmt, eine Außenoberfläche der Behältnisse zu behandeln. Bei dieser Ausgestaltung ist es möglich und bevorzugt, dass die Behandlungseinrichtung insbesondere seitlich neben einem Transportpfad der Behältnisse angeordnet ist.

Insbesondere ist die Behandlungseinrichtung seitlich neben dem Transportpfad aber mit einem geringen Abstand zu diesem angeordnet. Unter einem geringen Abstand wird dabei ein Abstand verstanden, der geringer ist als 10 cm, bevorzugt geringer als 8 cm, bevorzugt geringer als 6 cm und bevorzugt geringer als 5 cm.

Bei einer weiteren vorteilhaften Ausführungsform weist die Transportrichtung eine Antriebseinrichtung und bevorzugt eine Vielzahl von Antriebseinrichtungen auf, mittels derer die von den Halteeinrichtungen gehaltenen Behältnisse bezüglich ihrer Längsachse drehbar sind. Dabei kann es sich beispielsweise um einen elektromotorischen Antrieb handeln, es kämen jedoch auch andere Antriebe in Betracht.

Besonders bevorzugt erzeugt die wenigstens eine Antriebseinrichtung berührungslos die Drehbewegungen der Behältnisse und/oder deren Halteeinrichtungen. Dabei ist beispielsweise eine magnetische Kopplung denkbar, welche sich auch durch ein Gehäuse, innerhalb dessen sich die Anordnung befindet erstrecken kann. Dies wird unter Bezugnahme auf die Figuren genauer erläutert.

Besonders bevorzugt sind die Behältnisse und insbesondere die an den Halteeinrichtungen gehaltenen Behältnisse in ihrer Längsrichtung bewegbar. Auf diese Weise kann die Behandlung der Behältnisse verbessert werden. Dabei ist es möglich, dass die Behältnisse gemeinsam mit der sie haltenden Halteeinrichtung oder auch gegenüber der Halteeinrichtung bewegt werden können. Dabei ist es möglich, dass jedes der Behältnisse einzeln in seiner Längsrichtung bewegbar ist. Es wäre jedoch auch denkbar, dass eine gemeinsame Bewegung der Halteeinrichtungen und/oder der Behältnisse, beispielsweise mittels einer Hubkurve erfolgt. Daneben könnten jedoch auch linearmotorische Antriebseinrichtungen vorgesehen sein, um diese Bewegung in der Längsrichtung der Behältnisse zu erreichen.

Bei einer weiteren vorteilhaften Ausführungsform ist die Behältnisbehandlungseinrichtung aus einer Gruppe von Behältnisbehandlungseinrichtungen ausgewählt, welche Behältnisau-ßenflächenbehandlungseinrichtungen, insbesondere Behältnisaußenflächensterilisationseinrichtungen, Behältnisinnenflächenbehandlungseinrichtungen, insbesondere Behältnisinnenflächensterilisationseinrichtung, Behältnisinspektionseinrichtungen, Behältnisbedruckeinrichtungen, Behältnismarkiereinrichtungen und dergleichen enthält.

Im Folgenden wird insbesondere auf die Sterilisation eines zu befüllenden Behältnisses oder eines Kunststoffvorformlings (der insbesondere in ein solches Behältnis umgeformt wird) Bezug genommen. Die Sterilisation eines zu befüllenden Behältnisses ist neben dem eigentlichen Füllvorgang der zentrale Prozessschritt in einer aseptischen Abfüllanlage. Neuere Entwicklungen nutzen ionisierende Strahlung, insbesondere Elektronenstrahlung, um eine Keimreduzierung zu erreichen. Diese Strahlung besteht in den meisten Anwendungen aus beschleunigten Elektronen (daneben oder alternativ jedoch auch ultraviolette Strahlung), die in einer entsprechenden Anlage erzeugt werden und so in oder auf das zu sterilisierende Behältnis gelangen.

Aus dem Stand der Technik der Anmelderin bekannte Systeme, die zur Sterilisation verwendet werden, weisen eine Elektronenerzeugungsvorrichtung auf, sowie einen Strahlfinger zur Entkeimung der inneren Oberflächen und eine Elektronenerzeugungsvorrichtung und daneben auch einen Flächenstrahler insbesondere zum Entkeimen von äußeren Oberflächen.

Bei bekannten Konzepten ist das Entkeimen der äußeren Oberflächen der Entkeimung der inneren Flächen vorgelagert. Dies erfolgt teilweise auf Karussells, wie bereits oben erläutert. Dabei sind üblicherweise derartige Transporteinrichtungen bzw. Karusselle mit einem Teilungsverzugsstern verbunden. Die dabei entstehende Röntgenstrahlung sollte durch geeignete Abschirmungen von der Umwelt abgeschirmt werden. Dadurch sind die beiden Behandlungseinrichtungen in einer strahlungsabschirmenden Vorrichtung eingebettet bzw. eingehaust. Um die Strahlungsabschirmung auch an einem Auslaufstern zu gewährleisten istjeweils noch ein Einlaufstern vorgeschaltet bzw. ein Auslaufstern nachgeschaltet. Auf diese Weise ergibt sich im Stand der Technik der Anmelderin eine Gesamtvorrichtung, welche mit fünf Sternen bzw. Karussell ausgestattet ist.

Hieraus ergeben sich diverse Nachteile, die vor allem in dem überlangen Transfer beispielsweise von einem Ofen zu einer Blasmaschine liegen bzw. in der langen Verweilzeit in dem Behandlungsmodul. Diese ergibt sich durch den Aufbau mit den fünf Sternen. Auf diese Weise ergeben sich auch große Schwierigkeiten beim Ausformen von Kunststoffvorformlinge zu fertigen Behältnissen, insbesondere wenn die Kunststoffvorformlinge etwas ungünstige Verstreckungsverhältnisse aufweisen.

Zusätzlich liegt auch in häufigen Übergaben, insbesondere innerhalb eines nur schlecht zugänglichen strahlungsabschirmenden Gehäuses eine erhöhte Gefahr von Fehlübergaben. Insbesondere bei einem Übergang von dem Einlaufstern in ein Außenbehandlungsmodul findet im Stand der Technik der Anmelderin eine punktuelle Übergabe von einem Klammerstern in einen Aufsteckdorn statt. Fehlgriffe wie Schiefsetzer o. ä. können dann bei nachfolgenden Übergaben zu einem Verlust des Kunststoffvorformlings oder gar zu Beschädigung der Übergabeeinrichtung führen.

Bei höheren Leistungen erhöht sich auch der Footprint der Anlage, weil sämtliche Sterne oder Karusselle mit der höheren Leistung wachsen würden.

Die Erfindung schlägt nun vor die Außensterilisation der Kunststoffvorformlinge am Teilungsverzugsstern durchzuführen. Um dies zu ermöglichen wird die Behandlungseinrichtung und insbesondere ein Flächenstrahler an den Teilungsverzugsstern gesetzt und der Teilungsverzugsstern bevorzugt mit einer zusätzlichen Hub-Dreheinheit ausgerüstet.

Dadurch ist es möglich die Verweilzeit der Kunststoffvorformlinge an der Behandlungseinrichtung und bevorzugt an einem Fenster des Flächenstrahlers zu verlängern und somit die benötigte Strahlendosis zum Entkeimen der Kunststoffvorformlinge zu gewährleisten.

Um die Dosis gleichmäßig am Umfang des Kunststoffvorformlings verteilen zu können ist der Teilungsverzugsstern bevorzugt mit einer Hub-Drehvorrichtung ausgestattet, welche es ermöglicht eine Drehung des Kunststoffvorformlings vor der Behandlungseinrichtung und insbesondere vor dem Fenster des Flächenstrahlers durchzuführen. Die Drehung sollte bevorzugt einmal vollständig über 360° gehen aber auch vielfache Drehungen wären denkbar und erwünscht.

Es ist aber auch denkbar mit leicht geringeren Drehungen als 360° zu arbeiten, da der Flächenstrahler die komplette ihm zugewandte Seite des Kunststoffvorformlings, wenn auch nicht homogen, behandelt.

Durch die Verwendung eines Teilungsverzugssterns (TVS) zum Bewegen des Kunststoffvorformlings vor dem Flächenstrahler ist es einerseits möglich die Behandlungszeit bzw. die Packungsdichte vor dem Behandlungsfenster zu erhöhen und andererseits auch die Laufbahn der Kunststoffvorformlinge vor dem Flächenstrahler zu optimieren.

Das bedeutet, dass idealerweise auf einer geraden Bahn oder auf einer maximalen Kreisbahn vor dem Flächenstrahler vorbeigefahren werden kann und dass der Abstand an den Seiten des Strahlenfenster nicht über Gebühr erhöht wird. Dies ist vorteilhaft, da mit erhöhtem Abstand bei der Behandlung die Dosis auf dem Kunststoffvorformling abnimmt. Die Dosisverteilung über dem Umfang des Kunststoffvorformlings wäre somit nicht gleichmäßig.

Bei einer bevorzugten Ausführungsform weist der Flächenstrahler eine ebene Oberfläche bzw. Abstrahloberfläche auf.

Im Inneren des Moduls bzw. Gehäuse und insbesondere wenigstens teilweise um den inneren Anteil des Teilungsverzugssterns herum ist bevorzugt eine Strahlenschutzwand angebracht welche die schädliche Röntgenbremsstrahlung des Flächenstrahlers aber auch die Strahlung der Fingeremitter gegenüber dem Außenbereich abschottet.

Dabei ist bevorzugt ein oberer Teil fest an dem Gehäuse angeordnet und ein unterer Teil an dem absenkbaren Boden befestigt. Beide Strahlenschutzwände sind besonders bevorzugt überlappend angeordnet und der Spalt der beiden Schutzwände ist vorteilhaft so gestaltet, dass die Hub-Drehvorrichtung sich, insbesondere durch den Spalt zwischen den beiden im Betrieb feststehenden Abschirmungen hindurch, auf einer Laufbahn bewegen kann.

Die Strahlenschutzwände des Gehäuses sind vorteilhaft aus einem besonderen strahlungsabschirmenden Material gefertigt zum Beispiel aus mit Edelstahl eingehaustem Blei oder aus Wolfram bzw. aus einem Wolfram-Sinter Verbund oder mit einem Material mit ähnlichen Eigenschaften.

Gleichwohl sind bevorzugt Teile der Abschirmung auch mit Edelstahl oder mit Gussmaterial ausgeführt. Das Anpassen des abschirmenden Materials an eine bessere Strahlendichtigkeit bringt vor allem eine wesentlich geringere Wandstärke der Abschirmung und somit die Möglichkeit, sich mit der Hub-Dreheinheit zwischen den beiden Schutzwänden hindurch auf der Laufbahn des Teilungsverzugssterns zu bewegen bzw. die Abschirmung in die Hub-Drehvorrichtung ragen zu lassen, ohne sie über Gebühr verbreitern bzw. vergrößern zu müssen.

Die Schutzwände des Gehäuses folgen bevorzugt der Laufbahn des TVS bzw. sind gleich oder sehr ähnlich der Kunststoffvorformlinglaufbahn nachempfunden. Die Laufbahn des TVS kann auch noch so angepasst werden, um seitlich des Flächenstrahlers eine Bewegung zum Flächenstrahler hin zu ermöglichen um mit der Abschirmung, die der Laufbahn Kunststoffvorformlings (auch als Preform bezeichnet) folgt, die Strahlenabschirmung zu verbessern.

Somit wäre es möglich, einen großen Teil der Röntgenbremsstrahlung direkt am bzw. in der Nähe des Flächenstrahlers abzuschirmen ohne dass die schädliche Strahlung weit ins Gehäuse gelangen kann. Dadurch wäre es auch möglich die erforderlichen Wandstärken schnell zu reduzieren was vor allem Kosten und Gewicht einsparen würde.

Eine weitere Ausführungsform wäre eine Verwendung einer Art Langstatorantriebs mit Shuttles, welche den TVS ersetzen bzw. seine Funktionen übernehmen könnten. Vorteilhaft wäre hier das noch freiere Umsetzen des Fahrprofils und damit die längere Verweilzeit vor dem Flächenstrahler. Bei dieser Ausführungsform wird die Verwendung eines Linearmotors vorgeschlagen. Auch dieser könnte, wie oben beschrieben im Bereich der Behandlungseinrichtung einen geradlinigen Verlauf oder einen Verlauf mit einem sehr hohen Krümmungsradius aufweisen.

Die Einleitung der Drehbewegung an die Hub-Dreheinheit erfolgt bevorzugt unter Einhaltung aseptischer Gesichtspunkte und wird am vorteilhaftesten berührungslos übertragen.
Aber auch alle anderen Möglichkeiten bis hin zum angetriebenen Zahnriemen sind denkbar.

Ein wesentlicher Aspekt dieser erfindungsgemäßen Ausgestaltung ist das Anbringen des Flächenstrahlers am Teilungsverzugsstern und somit bevorzugt, dass Drei - Sterne Konzept erst zu ermöglichen. Damit ergeben sich erhebliche Vorteile durch die Verkürzung der Verweilzeit der Kunststoffvorformlinge im Modul bzw. in der Vorrichtung. Zusätzlich kann die Verweilzeit der Kunststoffvorformlinge vor der Behandlungseinrichtung, insbesondere vor dem Flächenstrahler erhöht werden, um auch hohe Entkeimungsleistungen fahren zu können.

Auch kann somit die Bahnkurve vor dem Flächenstrahler optimiert werden, um die Strahlungsdosis an den Seiten des Flächenstrahler zu erhöhen. Der Abstand vor dem Flächenstrahler kann somit möglichst nah am Strahlenfenster erfolgen bzw. wird an den Rändern nicht erhöht.

Bei einer bevorzugten Ausführungsform ist ein Abstand zwischen dem Flächenstrahler und dem Behältnis bzw. dem Kunststoffvorformlinge kleiner als 10cm, bevorzugt kleiner als 8cm, bevorzugt kleiner als 6cm, bevorzugt kleiner als 5cm, bevorzugt kleiner als 4cm, bevorzugt kleiner als 3cm und besonders bevorzugt kleiner als 2cm.

Wie erwähnt wird, wird insbesondere bei der Entkeimung von Kunststoffvorformlingen, durch Elektronenstrahler in einem Teilschritt die Außenseite entkeimt. Dabei dreht sich der Kunststoffvorformling vor einer Elektronenquelle um seine Längsachse, sodass alle Oberflächenpunkte der Außenseite des Kunststoffvorformlings eine möglichst gleichmäßige Bestrahlungsdosis erfahren.

Im Stand der Technik wird der Kunststoffvorformling vor einer Elektronenquelle rotiert, indem sein Träger, beispielsweise durch Riemen oder Ritzel angetrieben wird. Nachteilig sind hier Abrieb und Schmutz, die durch den kontaktbehafteten Antrieb unweigerlich auftreten. Gerade in einem aseptischen Umfeld stellen derartige potentielle Verunreinigungen ein großes Problem dar.

Aus der EP 3 431 402 ist ein magnetischer Antrieb bekannt, der eine Drehbewegung kontaktlos einleitet und deshalb nahezu ohne einen Antrieb auskommt. Nachteilig ist hier jedoch die Gleichmäßigkeit der Drehgeschwindigkeit, da durch die magnetische Kopplung stark variierende Drehmomente auftreten. Dies kann zur Folge haben, dass der Kunststoffvorformling nicht gleichmäßig vor der Strahlenquelle rotiert und die Dosisverteilung auf der Oberfläche der Außenseite des Kunststoffvorformlings nicht konstant ist.

Es wird damit vorgeschlagen, den Kunststoffvorformling nicht wie üblicherweise auf einem Transportstern mit konstanter Teilung vor dem Elektronenstrahler zu transportieren, sondern auf einem Teilungsverzugsstern (TVS). Dadurch wird eine variable Tangentialgeschwindigkeit an verschieden Punktes des Sterns ermöglicht.

Um den Kunststoffvorformling um seine Längsachse zu drehen wird vorgeschlagen, in eben diesen TVS Rotationsdorne zu integrieren, auf die der Kunststoffvorformling aufsteckbar ist und insbesondere von unten aufgesteckt wird. Die Dorne werden wenigstens im Bereich der Elektronenquelle um ihre Längsachse rotiert, um die Außenhülle des Kunststoffvorformlings möglichst gleichmäßig zu bestrahlen.

Als Antrieb der Rotationsdorne wird ein magnetischer Antrieb und/oder ein berührungsloser Antrieb eingesetzt.

Bei einer bevorzugten Ausführungsform weist der Antrieb eine Drehgeschwindigkeitsglättungseinrichtung auf, welche bewirkt, dass der Drehantrieb keinen hohen Drehgeschwindigkeitsschwankungen unterworfen ist. Dies wird untenstehend genauer erläutert.

Bei einer bevorzugten Ausführungsform weist die Behältnisbehandlungsvorrichtung ein Gehäuse auf, innerhalb dessen wenigstens eine Transporteinrichtung angeordnet ist und dieses Gehäuse bildet bevorzugt einen Reinraum aus, der einen Innenraum dieses Gehäuses gegenüber einer (insbesondere unsterilen) Umgebung abschirmt. Besonders bevorzugt ist der hier beschriebene Teilungsverzugsstern innerhalb dieses Gehäuses angeordnet. Bevorzugt sind auch weitere Transporteinrichtungen innerhalb dieses Gehäuses angeordnet.

Besonders bevorzugt ist dieses Gehäuse wenigstens abschnittsweise als Strahlenschutzgehäuse ausgebildet, insbesondere zum Schutz gegen Beta- und/oder Gammastrahlung, aber auch gegen Röntgenstrahlung.

Bei einer weiteren bevorzugten Ausführungsform verläuft wenigstens eine Wandung des Gehäuses parallel zu einem Abschnitt des Transportpfads der Behältnisse. Durch diesen Verlauf kann insgesamt die Gehäusegröße verringert werden. Insbesondere handelt es sich hier um eine entlang des Transportpfads der Kunststoffvorformlinge geradlinig verlaufende Wandung.

Bei einer weiteren vorteilhaften Ausführungsform weist die Behältnisbehandlungsvorrichtung wenigstens eine weitere Transporteinrichtung zum Transportieren der Behältnisse auf, und bevorzugt wenigstens zwei weitere Transporteinrichtungen und besonders bevorzugt genau zwei weitere Transporteinrichtungen, die zum Transportieren der Behältnisse und insbesondere Kunststoffvorformlinge dienen.

Bei einer weiteren bevorzugten Ausführungsform weist die Behältnisbehandlungsvorrichtung wenigstens eine zweite Behältnisbehandlungseinrichtung auf, wobei diese zweite Behältnisbehandlungseinrichtung besonders bevorzugt eine Behältnisinnenflächenbehandlungseinrichtung und insbesondere eine Behältnisinnenflächensterilisationseinrichtung ist.

Wie oben erwähnt, weist diese zweite Behandlungseinrichtung bevorzugt eine Vielzahl von Strahlerfingern auf, welche in die Behältnisse einführbar sind, um diese mit Elektronenstrahlung zu beaufschlagen. Besonders bevorzugt sind jeweils Elektronenbeschleunigungseinrichtungen vorgesehen sowie auch Austrittsfenster zum Austritt der Elektronenstrahlen, die bevorzugt aus Titan ausgebildet sind.

Bei einer weiteren vorteilhaften Ausführungsform erzeugt die Antriebseinrichtung die Drehbewegung der Halteeinrichtungen mittels magnetischer Kräfte. Wie unten genauer erläutert, kann an der Halteeinrichtung ein Rotor mit einer Vielzahl von Magneten angeordnet sein, dessen Drehbewegung von einem Stator, der ebenfalls eine Vielzahl von Magneten oder magnetischen oder magnetisierbaren Elementen aufweist, erzeugt wird.

Bei einer weiteren vorteilhaften Ausführungsform weist die Behältnisbehandlungsvorrichtung eine Überwachungseinrichtung zum Überwachen der Drehbewegung der Behältnisse auf. Besonders bevorzugt weist dabei diese Überwachungseinrichtung eine Bildaufnahmeeinrichtung oder wenigstens eine Spule auf. So können beispielsweise in einer Spule Ströme oder Spannungen induziert werden, welche wiederum ein Maß für die Überwachung der Drehbewegung sind. Dabei wäre es beispielsweise möglich, dass eine Vielzahl von Spulen vorgesehen ist, welche zwischen Magneten beispielsweise innerhalb eines Stators angeordnet ist. Bevorzugt ist die Überwachungseinrichtung dazu geeignet und bestimmt, einen Stillstand der Drehbewegung der Kunststoffvorformlinge und/oder eine Abweichung der Drehgeschwindigkeiten der Kunststoffvorformlinge (bzw. der sie haltenden Halteeinrichtungen zu erkennen.

Bei einer besonders bevorzugten Ausführungsform weist die Antriebseinrichtung einen mit der Halteeinrichtung gekoppelten Rotor auf, an dem eine Vielzahl von Magneten angeordnet ist, sowie einen Stator, an dem bevorzugt ebenfalls eine Vielzahl von Magneten oder magnetischen Elementen angeordnet ist. Auf diese Weise erfolgt eine Übertragung der Drehbewegung durch magnetische Kräfte zwischen einem Stator und einem Rotor. Dabei wäre es möglich, dass dieser Stator außerhalb oder innerhalb des Gehäuses angeordnet ist und dass die Übertragung der magnetischen Kräfte durch eine Gehäusewandung erfolgt.

Bei einer bevorzugt Ausführungsform ist der Stator geradlinig ausgebildet und/oder die magnetischen Elemente des Stators erstrecken sich entlang einer geradlinigen Richtung. Besonders bevorzugt ist der Rotor kreisförmig ausgebildet. Bei dieser Ausgestaltung wirkt daher ein geradliniger Stator mit einem kreisförmigen Rotor zusammen.

Besonders bevorzugt erstrecken sich die Magneten oder magnetischen Elemente (oder eine Längsrichtung dieser Magnete oder magnetischen Elemente des Rotors und/oder des Stators wenigstens abschnittsweise schräg bezüglich einer Richtung, welche parallel zur Drehachse der Halteeinrichtung verläuft.

Bei der aus der EP 3 431 402 dargestellten vertikalen Anordnung von Magneten schwanken die Anziehungskräfte entlang der Transportrichtung stark, weil sich an einer Position die Magnete genau gegenüberstehen. In diesem Fall wird kein Drehmoment übertragen und an einer anderen Position kommt es zu einem Abstoßen und zu einem Anziehen und dadurch zu einem hohen Drehmoment. Diese hohe Varianz in dem Drehmoment führt zu (nicht gewollten) Geschwindigkeitsschwankungen. Dieses Problem steigt mit zunehmender Transversalgeschwindigkeit der Rollen, d. h. mit steigenden Produktionsleistungen. Ab einer bestimmten Transversalgeschwindigkeit führen die Drehmomentschwankungen dazu, dass die Rolle nicht mehr in die Magnetteilung synchronisiert werden kann und damit keine kontinuierliche Drehbewegung mehr ausgeführt wird.

Die hier beschriebene verbesserte Form der Permanentmagnete insbesondere an einen Stator, der besonders bevorzugt als feststehende Leiste ausgebildet ist und einem Rotor (welcher beispielsweise als Magnetrolle ausgeführt ist) kann einen glättenden Effekt auf den Drehmomentverlauf entlang des Transportwegs haben. So können beispielsweise schräg verzahnte Magnetanordnungen vorgesehen sein.

Bei einer bevorzugten Ausführungsform verlaufen die Magnete und/oder magnetischen Elemente geradlinig jedoch schräg und/oder windschief bezüglich der genannten Drehachse. Bei einer weiteren bevorzugten Ausführungsform verlaufen die Magnete und/oder magnetischen Elemente abgewinkelt gegenüber der Drehachse. Bei einer weiteren bevorzugten Ausführungsform verlaufen die Magnete oder magnetischen Elemente gekrümmt.

Bei einer weiteren bevorzugten Ausgestaltung verlaufen die sind die einzelnen Magnete in der Transportrichtung der Kunststoffvorformlinge voneinander beabstandet.

Bei einer bevorzugten Ausführungsform trägt nur einer der beiden Magnetpartner Magnete und der andere weist ein Material mit hoher magnetischer Permeabilität auf, wie zum Beispiel Eisen. Durch eine geeignete Führung der magnetischen Flüsse können Drehmomentschwankungen in günstiger Weise beeinflusst werden. Dieses Prinzip ist teilweise bei Reluktanzmotoren bekannt, bei denen das Drehmoment im Rotor ausschließlich durch die Reluktanzkraft erzeugt wird und nicht zu wesentlichen Anteilen durch die Lorentzkraft.

Bei einer bevorzugten Ausführungsform wird das Material eines Trägers der Magneten (nicht das Magnetmaterial selbst) so gewählt, dass es einen möglichst geringen spezifischen elektrischen Widerstand aufweist. So kann beispielsweise der Magnetträger aus Aluminium bestehen.

Bei einer weiteren vorteilhaften Ausführungsform weist die Antriebseinrichtung einen mit der Halteeinrichtung gekoppelten Rotor auf sowie einen Stator, wobei entweder der Rotor oder der Stator aus einem Material mit einer hohen magnetischen Permeabilität besteht oder ein solches Material aufweist, wobei dieses Material bevorzugt aus einer Gruppe von Materialien ausgewählt ist welche Eisen, Mumetall (NiFe), nanokristalline Metalle und amorphe Metalle enthält) und/oder die Permeabilitätszahl des Materials größer ist als 200, bevorzugt größer als 300.

Wenn die Tangentialgeschwindigkeit ungleich einer idealen Abrollgeschwindigkeit an der Leiste bzw. dem Stator ist, induzieren die gegenüberliegenden Permanentmagnete eine Spannung in das leitfähige Trägermaterial und verursachen Wirbelströme. Diese bremsen oder beschleunigen die Drehbewegung einer Rolle und drängen sie in Richtung der Idealdrehzahl.

Diese Idee ist ähnlich dem Prinzip einer Wirbelstrombremse, d. h. auch hier wird der Umfang der Rolle gebremst und damit die Rolle selbst in Drehung versetzt.

Besonders bevorzugt weist die Vorrichtung eine Abdeckung zum Abdecken der Magnete auf. Diese kann beispielsweise aus einem elektrisch gut leitenden Material bestehen, um den Wirbelstromeffekt hier zu erzeugen. So kann die Abdeckung beispielsweise aus Aluminium bestehen.

Wie erwähnt, sind bei einer bevorzugten Ausführungsform in wenigstens eines der Antriebselemente, d. h. den Stator oder den Rotor und insbesondere in eine feststehende Magnetleiste Spulen eingesetzt, welche bevorzugt in axialer Richtung in Richtung des Rotors, d. h. der Rolle zeigen.

Sofern sich der Rotor mit einer idealen Geschwindigkeit dreht und wie gewünscht an der Leiste "abrollt", wird nur wenig Spannung in den Spulen induziert. Rutscht eine Rolle jedoch durch oder schwingt sie in der Drehgeschwindigkeit, dann wird durch die große Flussdichteänderung relativ viel Spannung in den Spulen induziert.

Die induzierten Spannungen werden von einer elektronischen Steuerung (beispielsweise SPS) gemessen und können entweder in der Spule oder als Reihenschaltung der Spulen ausgewertet werden.

Die vorliegende Erfindung ist weiterhin auf ein Verfahren zum Behandeln von Behältnissen gerichtet, wobei die Behältnisse mit einer Transportvorrichtung entlang eines vorgegebenen Transportpfads transportiert werden und wobei die Transportvorrichtung wenigstens eine Transporteinrichtung zum Transportieren des Behältnisses aufweist und wobei wenigstens eine Behältnisbehandlungsvorrichtung die Behältnisse in einer vorgegebenen Weise behandelt, wobei diese Transporteinrichtung einen drehbaren Träger aufweist, an dem eine Vielzahl von Halteeinrichtungen diese (Vielzahl der) Behältnisse hält.

Bei einer erfindungsgemäßen Ausgestaltung werden diese Halteeinrichtungen gegenüber einem Träger derart bewegt, dass ein Abstand zwischen zwei auf dem Transportpfad unmittelbar aufeinanderfolgenden Behältnisse verändert wird. Weiterhin behandelt die Behältnisbehandlungseinrichtung die von Transporteinrichtung transportierten Behältnisse.

Bei einem weiteren erfindungsgemäßen Verfahren werden die von den Halteeinrichtungen gehaltenen Behältnisse wenigstens zeitweise und bevorzugt während dieser Behandlung bezüglich einer Längsrichtung der Behältnisse und insbesondere während der Behandlung gedreht, wobei besonders bevorzugt eine Überwachungseinrichtung die Drehbewegung der Behältnisse überwacht und insbesondere hinsichtlich einer Drehgeschwindigkeit überwacht.

Es wird daher auch verfahrensseitig vorgeschlagen, dass die Behandlung während einer Drehung der Behältnisse erfolgt. Bevorzugt handelt es sich bei den Behältnissen um Kunststoffvorformlinge.

Bei einem bevorzugten Verfahren behandelt die Behältnisbehandlungseinrichtung eine Au-ßenoberfläche der Behältnisse. Insbesondere sterilisiert die Behältnisbehandlungseinrichtung die Außenoberfläche der Behältnisse. Besonders bevorzugt wird zu diesem Zweck ein Flächenstrahler vorgesehen, der Strahlung und insbesondere Elektronenstrahlung auf die Außenoberfläche der Behältnisse richtet. Dabei kann dieser Flächenstrahler derart an einem Gehäuse der Vorrichtung angeordnet sein, dass die Strahlung durch eine Öffnung des Gehäuses hindurch auf die Behältnisse trifft.

Besonders bevorzugt behandelt die Behältnisbehandlungseinrichtung die Behältnisse, während diese Behältnisse entlang eines im Wesentlichen geradlinigen Transportpfadabschnitts transportiert werden. Alternativ kann es sich auch um einen Transportpfadabschnitt handeln, dessen Krümmungsradius wesentlich größer ist, als ein geometrischer Krümmungsradius, der entstehen würde, wenn alle Halteeinrichtungen auf der Transporteinrichtungen gleichartig ausgerichtet werden. Besonders bevorzugt ist dieser Krümmungsradius wenigstens zweimal, bevorzugt wenigstens dreimal, bevorzugt wenigstens viermal und bevorzugt wenigstens fünfmal so groß.

Besonders bevorzugt werden die Behältnisse auch mit einer zweiten Behandlungseinrichtung behandelt. Dabei wird insbesondere eine Innensterilisation vorgenommen, wie oben beschrieben. Dabei können beispielsweise Elektronenstrahleinrichtungen in die Behältnisse eingeführt werden.

Bevorzugt werden die Behältnisse an den stationär angeordneten Behandlungseinrichtungen vorbeibewegt. Bei einem weiteren bevorzugten Verfahren wird die Drehung der an den Halteeinrichtungen angeordneten Behältnisse durch magnetische Kräfte übertragen.

Bevorzugt erfolgt die Übertragung der Drehbewegung derart, dass eine Drehgeschwindigkeit der Behältnisse und/oder Kunststoffvorformlinge geglättet wird.

Bei einem bevorzugten Verfahren wird die Drehbewegung der Behältnisse geglättet und/oder eine Schwingungsdämpfung mittels Wirbelströmen vorgenommen.

Die vorliegende Erfindung bezieht sich weiterhin auf eine Vorrichtung zum Behandeln von Kunststoffvorformlingen, insbesondere zum Sterilisieren von Kunststoffvorformlingen.

Derartige Vorrichtungen sind aus dem Stand der Technik seit langem bekannt. Dabei werden Kunststoffvorformlinge in einem Gehäuse, in welchem eine Behandlung, insbesondere eine Sterilisation, durchgeführt wird, durch einen Eingang zugeführt. In diesen derartigen Vorrichtungen sind üblicherweise Transporteinrichtungen angebracht, die die Kunststoffvorformlinge entlang eines Transportpfades transportieren. Nach einer Behandlung erfolgt eine Entfernung der Kunststoffvorformlinge über einen Ausgang der Vorrichtung.

Allerdings weist der Stand der Technik einen Nachteil auf. Um mangelhafte Kunststoffvorformlinge oder Kunststoffvorformlinge, die bestimmte Erfordernisse nicht erfüllen, welche beispielsweise nicht ausreichend behandelt und insbesondere sterilisiert wurden, entfernen zu können, müsste das ganze Verfahren gestoppt und das Gehäuse geöffnet werden. Jedoch könnten durch das Öffnen der Vorrichtung Kunststoffvorformlinge, die die Erfordernisse erfüllen, wieder kontaminiert werden. Auch gehen bei einem Öffnen des Gehäuses dessen Reinraumeigenschaften verloren und es muss erneut das Gehäuse sterilisiert werden.

Die vorliegende Erfindung liegt daher die Aufgabe zugrunde, dass Kunststoffvorformlinge oder allgemein Behältnisse entfernbar sind, ohne den Betriebsprozess zu unterbrechen und/oder das Gehäuse zu öffnen.

Diese Aufgaben werden erfindungsgemäß durch die Gegenstände der unabhängigen Ansprüche erreicht. Vorteilhafte Ausführungsformen und Weiterbildungen sind Gegenstand der Unteransprüche.

Eine erfindungsgemäße Vorrichtung zum Behandeln von Behältnissen, insbesondere von Kunststoffbehältnissen und insbesondere Kunststoffvorformlingen, insbesondere zum Sterilisieren von Kunststoffvorformlingen, weist ein Gehäuse auf, welches die Vorrichtung umgibt, wobei innerhalb des Gehäuses die Behältnisse bzw. Kunststoffvorformlinge behandelt werden. Innerhalb dieses Gehäuses ist wenigstens eine Transporteinrichtung zum Transportieren der Kunststoffvorformlinge entlang eines vorgegebenen Transportpfads vorgesehen. Dabei weist die Vorrichtung einen Eingang auf, durch den die Kunststoffvorformlinge in das Gehäuse einführbar sind, und einen Ausgang, durch den die Kunststoffvorformlinge aus dem Gehäuse ausführbar sind, auf.

Erfindungsgemäß weist die Vorrichtung eine Schleuseneinrichtung und/oder eine Entnahmeeinrichtung auf, mittels derer die Kunststoffvorformlinge aus dem Gehäuse entfernbar sind.

Bei einer weiteren vorteilhaften Ausführungsform weist die Vorrichtung Halteeinrichtungen zum Halten der Kunststoffvorformlinge. Dabei kann es sich um Haltedorne handeln, die in den Mündungen der Kunststoffvorformlinge eingreifen.

Es wird darauf hingewiesen, dass die hier vorliegende Erfindung auch mit weiteren Aspekten, die im Rahmen dieser Anmeldung beschrieben wurden, kombinierbar sind, beispielsweise mit dem Vorhandensein genau dreier Transporteinrichtungen innerhalb des Gehäuses oder mit dem Vorhandensein eines Teilungsverzugssterns oder dergleichen.

Bei einer weiteren vorteilhaften Ausführungsform weist die Transporteinrichtung einen drehbaren Träger auf, an dem die Halteeinrichtungen angeordnet sind. Dabei kann es sich um ein Transportrad handeln.

Vorteilhafterweise ist die Transporteinrichtung ein Teilungsverzugsstern, welcher geeignet ist, einen Abstand aufeinanderfolgender Kunststoffvorformlinge zu verändern und insbesondere zu erhöhen. Denkbar wäre anstelle des Teilungsverzugsstern bevorzugt auch ein Langstator, welcher die Teilung der Kunststoffvorformlinge ändert.

Bevorzugterweise sind die Kunststoffvorformlinge während eines Betriebes der Vorrichtung entfernbar, insbesondere während eines Sterilisationsbetriebes, insbesondere auch während eines Transports der übrigen Kunststoffvorformlinge.

Bei einer weiteren vorteilhaften Ausführungsform sind die Behältnisse und insbesondere Kunststoffvorformlinge über einen weiteren Ausgang entfernbar, der bevorzugt zwischen dem Eingang und dem Ausgang angebracht ist. Besonders bevorzugt ist ein weiterer Ausgang näher an dem Ausgang als dem Eingang angeordnet. Bei einer weiteren bevorzugten Ausführungsform weist die Vorrichtung mehrere Transporteinrichtungen auf und der weitere Ausgang ist besonders bevorzugt in einem Bereich der letzten dieser Transporteinrichtungen angeordnet.

Bei einer weiteren vorteilhaften Ausführungsform entfernt die Vorrichtung ausgewählte und/oder fehlerhafte Kunststoffvorformlinge. Dabei kann die Vorrichtung bevorzugterweise beschädigte Kunststoffvorformlinge oder solche, die herabgefallen sind, entfernen. Daneben können auch Behältnisse und insbesondere Kunststoffvorformlinge, die fehlerhaft sterilisiert wurden, entfernt werden.

Bei einer weiteren vorteilhaften Ausführungsform weist die Vorrichtung eine Inspektionseinrichtung zum Inspizieren der Behältnisse und insbesondere der Kunststoffvorformlinge auf. Dabei werden die Behältnisse und insbesondere die Kunststoffvorformlinge auf Fehler inspiziert und/oder werden untersucht, ob sie bestimmte Erfordernisse erfüllen. Bevorzugterweise sendet diese Inspektionseinrichtung der Schleuseneinrichtung ein Signal, ob ein Behältnis und insbesondere ein Kunststoffvorformling entfernt werden soll.

Bei einer weiteren vorteilhaften Ausführungsform weist die Vorrichtung eine Sterilisationseinrichtung zum Sterilisieren der Kunststoffvorformlinge auf. Dabei ist besonders bevorzugt eine Elektronenstrahlsterilisationseinrichtung angebracht.

Bevorzugterweise weist die Vorrichtung eine Behältnisinnenflächenbehandlungseinrichtung und/oder eine Behältnisaußenflächenbehandlungseinrichtung. Wie oben erwähnt, ist die Behältnisaußenflächenbehandlungseinrichtung bevorzugt als Flächenstrahler ausgebildet und insbesondere als Elektronenflächenstrahler.

Bei einer weiteren bevorzugten Ausführungsform ist die Behältnisinnenflächenbehandlungseinrichtung in der obigen Weise ausgebildet, weist also insbesondere in die Kunststoffvorformlinge einführbare stangenartige Körper auf, welche die Innenwandung der Kunststoffvorformlinge mit Elektronenstrahlen sterilisieren.

In einer weiteren vorteilhaften Ausführungsform ist ein Auslaufstern vorgesehen, mittels dessen die Kunststoffvorformlinge aus der Vorrichtung ausführbar sind.

Bei einer weiteren vorteilhaften Ausführungsform ist das Gehäuse, welches die Vorrichtung umgibt, ein Isolator. Dabei ist bevorzugterweise ein Reinraum vorgesehen, welcher besonders bevorzugt unter einem Überdruck (z.B. an Sterilluft) gehalten ist. Das Gehäuse und/oder die Gehäusewandungen sind bevorzugterweise strahlungsabschirmend ausgebildet.

Erfindungsgemäß kann auch bei einer Entnahme von Kunststoffvorformlingen durch eine Schleuseneinrichtung die Reinraumfunktion erhalten werden.

Vorteilhafterweise ist die Schleuseneinrichtung abtrennbar und insbesondere von dem Gehäuse abtrennbar. So ist es möglich, dass ein Bereich der gesamten Vorrichtung bzw. des Elektronenstrahlmoduls von dem Gehäuse abtrennbar ist. Insbesondere ist dieser Bereich und/oder die Schleuseneinrichtung abtrennbar, ohne dass hierbei die Reinraumeigenschaften zerstört werden. Bevorzugt ist diese Schleuseneinrichtung auch in einem laufenden Betrieb der Vorrichtung abtrennbar.

Bei einer bevorzugten Ausführungsform ist die Schleuseneinrichtung von einem Bediener manuell abtrennbar. Die Schleuseneinrichtung und/oder besagte Bereich ist bevorzugt so abtrennbar, dass in einem Abtrennbereich weder Strahlung noch bei der Produktion entstehende Gase in das Gehäuse ein oder aus diesem austreten können oder die Sterilität der restlichen Maschine gefährdet ist.

Bei einer bevorzugten Ausführungsform weist die Vorrichtung eine Transporteinrichtung und insbesondere einen Auslaufstern auf, mittels dessen die Behältnisse und insbesondere Kunststoffvorformlinge aus der Vorrichtung ausgeführt werden. Diese Transporteinrichtung und/oder die Vorrichtung kann dabei eine Strahlenschutzeinrichtung aufweisen, welche ein Austreten von Strahlung und insbesondere Röntgenstrahlung aus dem Gehäuse verhindert.

In einem laufenden Betrieb der Vorrichtung ist es möglich, dass Behältnisse und insbesondere Kunststoffvorformlinge in den Bereich der Schleuseneinrichtung geleitet werden, insbesondere wenn diese Schleuseneinrichtung gerade nicht von der Vorrichtung getrennt ist bzw. sich an der Vorrichtung bzw. dem Gehäuse befindet.

Bei einer weiteren vorteilhaften Ausführungsform weist die Vorrichtung eine Fördereinrichtung auf, welche Behältnisse und insbesondere Kunststoffvorformlinge in die Schleuseneinrichtung und insbesondere in den erwähnten abtrennbaren Teil der Vorrichtung fördert. Dabei kann eine derartige Fördereinrichtung beispielsweise Schrägflächen aufweisen, welche die Behältnisse bzw. Kunststoffvorformlinge in die Schleuse fördern.

Durch die beschriebene Vorgehensweise ist eine Ausschleusung von Behältnissen bzw. Kunststoffvorformlingen in während einer laufenden Produktion möglich bzw. die Produktion muss nicht unterbrochen werden. Die Kunststoffvorformlinge können auf diese Weise für weitere Untersuchungen entnommen werden.

Bei einer bevorzugten Ausführungsform ist die Schleuseneinrichtung an einer Wandung des Gehäuses angeordnet und insbesondere an einer Öffnung dieser Wandung, über welche Kunststoffvorformlinge von dem Gehäuse in die Schleuseneinrichtung gelangen können.

In diesem der Wandung des Gehäuses kann eine bewegbares Wandungselement vorgesehen sein, mit dem diese Öffnung verschlossen werden kann.

Bei einer weiteren vorteilhaften Ausführungsform ist in dem Gehäuse eine weitere Transporteinrichtung und bevorzugt sind wenigstens zwei weitere Transporteinrichtungen angeordnet.

Die vorliegende Erfindung ist weiterhin auf ein Verfahren zum Behandeln von Kunststoffvorformlingen, insbesondere zum Sterilisieren von Kunststoffvorformlingen gerichtet. Dabei werden die Kunststoffvorformlinge innerhalb eines Gehäuses behandelt und insbesondere sterilisiert und dabei mit einer Transporteinrichtung entlang eines vorgegebenen Transportpfades transportiert. Die Kunststoffvorformlinge werden über einen Eingang in das Gehäuse eingeführt und am Ende eines Betriebes über einen Ausgang wieder aus dem Gehäuse ausgeführt.

Erfindungsgemäß wird ein vorgegebener Anteil der Kunststoffvorformlinge über eine Schleuseneinrichtung aus dem Gehäuse entfernt.

Bei einem weiteren bevorzugten Verfahren werden die Kunststoffvorformlinge mit einer Sterilisationseinrichtung behandelt und insbesondere sterilisiert. Dabei werden die Kunststoffvorformlinge besonders bevorzugt mit einer Behältnisinnenflächenbehandlungseinrichtung und/oder mit einer Behältnisaußenflächenbehandlungseinrichtung behandelt und insbesondere sterilisiert.

Bevorzugt wird die Schleuseneinrichtung wenigstens zeitweise von dem Gehäuse getrennt oder abgenommen.

Zusammengefasst können die Kunststoffvorformlinge entlang einer geraden Linie an der Behandlungseinrichtung vorbeitransportiert werden. Auf diesen Abstand bleibt auch der Abstand zwischen dem Kunststoffvorformling bzw. Behältnis und der Behandlungseinrichtung, insbesondere einem Flächenstrahler im Wesentlichen gleich. Weiterhin werden bevorzugt die Kunststoffvorformlinge zwischen der Behandlungseinrichtung und einem Strahlenschutz bewegt. Dieser Strahlenschutz kann dabei aus einem speziell gewählten Material, beispielsweise Wolfram bestehen.

Der Teilungsverzugsstern ermöglicht ist, dass die Kunststoffvorformlinge verhältnismäßig länger an der Behandlungseinrichtung bzw. dem Flächenstrahler vorbeibewegt werden. Bevorzugt kann auch die Packungsdichte an Kunststoffvorformlingen vor dem Flächenstrahler erhöht werden.

Der Strahlenschutz (innerhalb und/oder außerhalb des Gehäuses weist bevorzugt eine Kontur auf, welche ein Austreten von Strahlung aus dem Gehäuse bzw. Isolator verhindert. Durch den Teilungsverzugsstern können die Kunststoffvorformlinge der Kontur des Strahlungsschutzes folgen. Wie oben erwähnt kann anstelle eines Teilungsverzugsstern auch ein Langstator mit Shuttles bzw. ein linearmotorischer Antrieb gewählt werden.

Die vorliegende Erfindung betrifft weiter eine Behältnisbehandlungsvorrichtung mit einem Modul zum Sterilisieren mit einer Strahlung und einem Rinser und ein entsprechendes Verfahren.

Im Bereich der Getränke herstellenden Industrie ist es seit langem bekannt, dass die abzufüllenden Behältnisse, insbesondere vor ihrer Befüllung, sterilisiert werden. Dabei hat es sich als vorteilhaft erwiesen, nicht erst die expandierten Behältnisse, sondern bereits die Vorformlinge vor der Umformung zu Behältnissen zu sterilisieren, da die Vorformlinge wesentlich kleiner sind als die hieraus entstehenden Behältnisse.

Der Begriff "Behältnis" umfasst daher insbesondere auch einen Vorformling, insbesondere einen Kunststoffvorformling.

Als Methode der Sterilisation kommt im Stand der Technik einerseits die Sterilisation mittels sterilisierenden Gasen und insbesondere mittels Wasserstoffperoxid vor. Um den Einsatz von Chemikalien bei der Sterilisation von Behältnissen zu reduzieren, sind im Stand der Technik auch Vorrichtungen und Verfahren bekannt, welche die Behältnisse mit anderen Maßnahmen sterilisieren, wie beispielsweise mittels ultravioletter Strahlung oder Elektronenstrahlen.

Zur Reinigung von Behältnissen ist es außerdem bekannt, die Behältnisse mit einem fließförmigen Medium zu beaufschlagen, so dass insbesondere Stäube und andere sich in den Behältnissen befindliche Verunreinigungen aus dem Innenraum der Behältnisse entfernt werden, um eine Verunreinigung des Produktes beim späteren Füllvorgang zu vermeiden.

Nach dem derzeitigen Stand der Technik erweist sich eine Kombination der verschiedenen Reinigungsmethoden häufig als schwierig.

Der Erfindung liegt daher die Aufgabe zugrunde, eine Vorrichtung und ein Verfahren zur Verfügung zu stellen, durch die ein möglichst gutes Reinigungs- und Sterilisationsergebnis der Behältnisse erzielt wird.

Eine erfindungsgemäße Behältnisbehandlungsvorrichtung, insbesondere eine Behältnissterilisationsvorrichtung, weist mehrere Transporteinrichtungen zum Transport eines Behältnisses entlang eines vorgegebenen Transportpfads innerhalb eines Gehäuses auf, wobei mindestens eine der Transporteinrichtungen eine Behältnisaußenflächenbehandlungseinrichtung ist und mindestens eine der Transporteinrichtungen eine Behältnisinnenflächenbehandlungseinrichtung ist, welche jeweils mindestens eine Halteeinrichtung zum Halten mindestens eines Behältnisses während einer Behältnisbehandlung und/oder des Transports entlang des Transportpfads aufweisen.

Erfindungsgemäß weist die Behältnisaußenflächenbehandlungseinrichtung und/oder die Behältnisinnenflächenbehandlungseinrichtung wenigstens eine Strahlungsquelle auf und die Behältnisbehandlungsvorrichtung weist erfindungsgemäß wenigstens eine Beaufschlagungseinrichtung aufweist, welche das Behältnis mit einem fließfähigen Medium, insbesondere ionisierter Luft, beaufschlagt.

Bei einer bevorzugten Ausführungsform handelt es sich bei der Strahlungsquelle um eine Elektronenstrahlungsquelle. Bevorzugt weisen die Behältnisaußenflächenbehandlungseinrichtung und die Behältnisinnenflächenbehandlungseinrichtung jeweils eine Strahlungsquelle, bevorzugt eine Elektronenstrahlungsquelle auf. Es wird daher vorgeschlagen, dass sowohl zur Behältnisinnensterilisation als auch zur Behältnisaußensterilisation jeweils Strahlung und insbesondere Elektronenstrahlung eingesetzt wird.

Bei einer bevorzugten Ausführungsform weist die Behältnisinnenflächenbehandlungseinrichtung eine Behältnisinnenflächenbeaufschlagungseinrichtung auf. Vorteilhaft beaufschlagt die Behältnisinnenflächenbeaufschlagungseinrichtung eine Behältnisinnenfläche mit Elektronenstrahlung. Bevorzugt ist die Behältnisinnenflächenbeaufschlagungseinrichtung in das Innere des zu sterilisierenden Behältnisses, insbesondere durch einen Mündungsbereich, einführbar. Vorteilhaft ist die Behältnisinnenflächenbeaufschlagungseinrichtung dazu geeignet, im Inneren des Behältnisses während eines vorgegebenen Zeitraums Strahlung, insbesondere Elektronenstrahlung zu emittieren.

Bevorzugt ist wenigstens eine Halteeinrichtung dazu geeignet und vorgesehen, das Behältnis während der Behältnisinnenflächensterilisation zu halten. Vorteilhaft sind die Behältnisinnenflächenbeaufschlagungseinrichtung und/oder die Halteeinrichtung beweglich. Vorteilhaft sind die Behältnisinnenflächenbeaufschlagungseinrichtung und/oder die Halteeinrichtung zumindest auch in einer Längsrichtung des Behältnisses beweglich. Bevorzugt sind die Behältnisinnenflächenbeaufschlagungseinrichtung und/oder die Halteeinrichtung zumindest auch während der Behältnisinnenflächensterilisation in einer Längsrichtung des Behältnisses beweglich. Vorteilhaft kann das Behältnis gegenüber der Behältnisinnenflächenbeaufschlagungseinrichtung in einer Längsrichtung des Behältnisses während eines vorgegebenen Zeitraums wenigstens zeitweise mit einer relativen Bewegungsgeschwindigkeit bewegt werden.

Bei einer bevorzugten Ausführungsform weist die Behältnisaußenflächenbehandlungseinrichtung eine Behältnisaußenflächenbeaufschlagungseinrichtung auf. Vorteilhaft ist die Behältnisaußenflächenbehandlungseinrichtung stets außerhalb der zu sterilisierenden Behältnisse angeordnet, beispielsweise seitlich entlang eines Transportpfades der Behältnisse. Bevorzugt ist die Behältnisaußenflächenbehandlungseinrichtung stationär gegenüber dem Transportpfad der Behältnisse angeordnet. Auf diese Weise werden die einzelnen Behältnisse an der Behältnisaußenflächenbehandlungseinrichtung vorbeigeführt. Vorteilhaft weist die Behältnisaußenflächenbeaufschlagungseinrichtung eine Strahlquelle auf, deren Austrittsfenster die Strahlung gleichmäßig auf eine große Fläche verteilt, insbesondere auf die Außenfläche der zu sterilisierenden Behältnisse.

Bei einer vorteilhaften Ausführungsform handelt es sich bei der Beaufschlagungseinrichtung bevorzugt um einen sogenannten Rinser bzw. eine Rinsereinheit. Bei dem fließfähigen Medium handelt es sich bevorzugt um ein gasförmiges Medium, besonders bevorzugt um Druckluft und insbesondere gereinigte, aufbereitete und/oder sterilisierte Druckluft. Vorteilhaft kann es sich bei dem fließfähigen Medium auch um ein flüssiges Medium handeln. Denkbar ist dabei auch, dass es sich bei dem fließfähigen Medium um ein steriles Medium handelt, so dass keine weiteren Kontaminationen an den Behältnissen verursacht werden.

Die Kombination einer Beaufschlagungseinrichtung mit einer Behältnisaußenflächenbehandlungseinrichtung und/oder einer Behältnisinnenflächenbehandlungseinrichtung wirkt sich besonders vorteilhaft auf die Entkeimungsergebnisse und den zu erzielenden log-Raten aus. Dies gilt besonders, wenn die Behältnisaußenflächenbehandlungseinrichtung und/oder die Behältnisinnenflächenbehandlungseinrichtung eine Strahlungsquelle, insbesondere eine Elektronenstrahlungsquelle aufweist.

Bei einer bevorzugten Ausführungsform weist die Behältnisbehandlungsvorrichtung eine weitere Transporteinrichtung auf, welche die Behältnisse während des Beaufschlagungsvorgangs transportiert. Bei dieser Transporteinrichtung handelt es sich bevorzugt um einen Sägezahnstern.

Bei einer bevorzugten Ausführungsform weist die Beaufschlagungseinrichtung wenigstens eine Rinserdüse auf. Bevorzugt kann es sich dabei um eine statische bzw. stationäre Düse handeln. Bei einer statischen Anordnung der Rinserdüse werden die Vorformlinge bevorzugt unter der Beaufschlagungseinrichtung hinwegbewegt und so gereinigt. Bei einer derartigen stationären Rinserdüse werden die Behältnisse, insbesondere die Kunststoffvorformlinge, bevorzugt in einer aufrechtstehenden Position, d.h. mit ihren Mündungen nach oben, transportiert.

Bei einer weiteren bevorzugten Ausführungsform ist die wenigstens eine Rinserdüse wenigstens zeitweise während des Transports der Behältnisse mit den Behältnissen mitbewegbar. Durch eine mitfahrende Düse sind, im Vergleich zu einer stationären Düse, erheblich längere Prozess- bzw. Rinserzeiten möglich, was insgesamt zu besseren Rinserergebnissen führt.

In einer weiteren besonders bevorzugten Ausführungsform ist die wenigstens eine Rinserdüse mit den Behältnissen mitbewegbar und wird in einen Mündungsbereich der Behältnisse eingeführt. Durch eine derartige mitfahrende und eintauchende Rinserdüse können, insbesondere bei sehr feinen und leichten Partikeln, höchste Reinigungsergebnisse erreicht werden.

Die Düsenstellung beim Einführen der Rinserdüse in die Behältnisse wird dabei bevorzugt über eine mechanische, hydraulische, pneumatische oder ähnliche hierfür geeignete Einrichtung gesteuert. Vorzugsweise wird dabei insbesondere die Höhe des Düsenkopfes beim Prozessstart, vor dem eigentlichen Prozess und während des Prozesses erfasst. Bevorzugt wird dabei die Eintauchtiefe der Düsen in das Behältnis und bevorzugt die Mündung des Behältnisses gezielt eingestellt um, je nach Behältnistyp, ein optimales Reinigungsergebnis zu erreichen und gewährleisten zu können.

Im Vergleich zu statischen Rinserdüsen kann der Reinigungsgrad der Behältnisse bei mitfahrenden Düsen demnach enorm erhöht werden, da hier ein Behältnis durch eine Düse längere Zeit gereinigt wird. Sowohl bei Verwendung einer statischen Düse als auch bei Verwendung einer mitfahrenden Düse erfolgt die Reinigung der Behältnisse bevorzugt während des Transports der Behältnisse.

Bevorzugt werden die Behältnisse, wenn es sich um eine mitfahrende Düse bzw. eine mitfahrende und eintauchende Düse handelt, über Kopf, d.h. mit der Mündung nach unten transportiert.

Bei einer bevorzugten Ausführungsform ist die Beaufschlagungseinrichtung außerhalb des Gehäuses angeordnet. Hierdurch kann das Gehäuse vorteilhaft möglichst klein ausgebildet sein.

Bei einer weiter bevorzugten Ausführungsform ist die Beaufschlagungseinrichtung entlang des Transportpfades stromaufwärts der Behältnisaußenflächenbehandlungseinrichtung und/oder der Behältnisinnenflächenbehandlungseinrichtung angeordnet. Besonders bevorzugt ist die Beaufschlagungseinrichtung sowohl stromaufwärts der Behältnisaußenflächenbehandlungseinrichtung als auch der Behältnisinnenflächenbehandlungseinrichtung angeordnet. Bevorzugt wird ein Behältnis somit zuerst von der Beaufschlagungseinrichtung mit einem fließfähigen Medium beaufschlagt, bevor es anschließend von der Behältnisaußenflächenbehandlungseinrichtung und der Behältnisinnenflächenbehandlungseinrichtung behandelt, insbesondere sterilisiert wird. Dies ist besonders deshalb vorteilhaft, weil durch die Beaufschlagungseinrichtung eine Vorreinigung des Behältnisses erfolgen kann, bevor die Au-βen- und/oder Innenfläche des Behältnisses sterilisiert wird.

Bevorzugt ist die Beaufschlagungseinrichtung nicht unmittelbar vor der Behältnisaußenflächenbehandlungseinrichtung und/oder der Behältnisinnenflächenbehandlungseinrichtung angeordnet. Vorteilhaft können zwischen der Beaufschlagungseinrichtung und der Behältnisau-βenflächenbehandlungseinrichtung und/oder der Behältnisinnenflächenbehandlungseinrichtung sowohl Transporteinrichtungen als auch weitere Behältnisbehandlungseinrichtungen vorgesehen sein.

Bei einer vorteilhaften Ausführungsform weist die Behältnisbehandlungsvorrichtung eine Heizvorrichtung zum Erwärmen des Behältnisses auf. Bevorzugt weist die Heizvorrichtung wenigstens eine Heizeinrichtung, besonders bevorzugt mehrere Heizeinrichtungen auf. Vorteilhaft kann es sich bei der Heizeinrichtung um eine Strahlungsquelle handeln, beispielsweise für Infrarot- oder Mikrowellenstrahlung.

Bevorzugt ist die Heizvorrichtung entlang des Transportpfads stromaufwärts der der Behältnisaußenflächenbehandlungseinrichtung und/oder der Behältnisinnenflächenbehandlungseinrichtung angeordnet. Besonders bevorzugt ist die Heizvorrichtung sowohl stromaufwärts der Behältnisaußenflächenbehandlungseinrichtung als auch der Behältnisinnenflächenbehandlungseinrichtung angeordnet. Bevorzugt wird ein Behältnis somit zuerst von der Heizvorrichtung erwärmt, bevor es von der Behältnisaußenflächenbehandlungseinrichtung und der Behältnisinnenflächenbehandlungseinrichtung behandelt, insbesondere sterilisiert wird.

Bei einer bevorzugten Ausführungsform ist die Beaufschlagungseinrichtung entlang des Transportpfades stromaufwärts der Heizvorrichtung angeordnet und/oder die Beaufschlagungseinrichtung ist unmittelbar vor der Heizvorrichtung angeordnet. Besonders bevorzugt ist die Beaufschlagungseinrichtung in die Heizvorrichtung integriert. Bevorzugt wird ein Behältnis somit zuerst von der Beaufschlagungseinrichtung mit einem fließfähigen Medium beaufschlagt, bevor es anschließend von der Heizvorrichtung erwärmt wird. Diese Reihenfolge ist besonders dann vorteilhaft, wenn es sich bei dem fließfähigen Medium um ein flüssiges Medium handelt. Dieses kann auf diese Weise durch die Erwärmung des Behältnisses in der Heizvorrichtung besonders gut verdunsten.

Bei einer bevorzugten Ausführungsform weist die Behältnisbehandlungsvorrichtung eine weitere Transporteinrichtung auf, die zwischen der Heizvorrichtung und der Behältnisaußenflächenbehandlungseinrichtung und/oder der Behältnisinnenflächenbehandlungseinrichtung angeordnet ist. Vorteilhaft handelt es sich um exakt eine Transporteinrichtung, die zwischen der Heizvorrichtung und der Behältnisaußenflächenbehandlungseinrichtung bzw. der Behältnisinnenflächenbehandlungseinrichtung angeordnet ist. Ob diese Transporteinrichtung zwischen der Heizeinrichtung und der Behältnisaußenflächenbehandlungseinrichtung oder zwischen der Heizeinrichtung und der Behältnisinnenflächenbehandlungseinrichtung angeordnet ist, hängt davon ab, ob in Transportrichtung die Behältnisaußenflächenbehandlungseinrichtung oder die Behältnisinnenflächenbehandlungseinrichtung stromaufwärts angeordnet ist.

Besonders bevorzugt ist eine Ausführungsform, bei der in Transportrichtung zunächst eine Beaufschlagungseinrichtung angeordnet ist, anschließend eine Heizvorrichtung, darauffolgend eine, insbesondere eine einzige, Transporteinrichtung und darauf folgend eine Behältnisaußenflächenbehandlungseinrichtung und eine Behältnisinnenflächenbehandlungseinrichtung. Vorteilhaft wird die Beaufschlagungseinrichtung somit nahe an der Behältnisaußenflächenbehandlungseinrichtung und der Behältnisinnenflächenbehandlungseinrichtung angeordnet. Hierdurch wird die Möglichkeit einer Wiederverschmutzung des Behältnisses zwischen der Beaufschlagungseinrichtung und der Behältnisaußenflächenbehandlungseinrichtung und der Behältnisinnenflächenbehandlungseinrichtung minimiert. Hierdurch können noch bessere Entkeimungsergebnisse erzielt werden.

Bevorzugt ist die Transporteinrichtung, die zwischen der Heizvorrichtung und der Behältnisaußenflächenbehandlungseinrichtung bzw. der Behältnisinnenflächenbehandlungseinrichtung angeordnet ist, außerhalb des Gehäuses angeordnet.

Vorzugsweise sind innerhalb des Gehäuses zusätzlich zu der Behältnisaußenflächenbehandlungseinrichtung und der Behältnisinnenflächenbehandlungseinrichtung höchstens zwei weitere Transporteinrichtungen, besonders bevorzugt lediglich eine weitere Transporteinrichtung angeordnet.

Zusätzlich zu den bereits genannten Vorteilen führt dies vorteilhaft zu einer größeren geometrischen Flexibilität. Im Stand der Technik sind üblicherweise fünf Sterne im Gehäuse angeordnet, insbesondere zwei Behandlungskarusselle und drei Transfersterne. Dies führt im Stand der Technik dazu, dass im Wesentlichen nur eine räumliche Anordnung der Heizvorrichtung zur Umformungseinrichtung möglich ist, bei der die Heizvorrichtung und die Umformungseinrichtung im Wesentlichen in einem 90°-Winkel angeordnet sind.

Eine 0°-Aufstellung von Heizvorrichtung und Umformungseinrichtung kann im Stand der Technik nicht realisiert werden. Dies kann zu erheblichen Einschränkungen der Layoutplanung kundenseitig führen. Ebenso ist es bei der im Stand der Technik bekannten Ausführungsform schwierig, eine Beaufschlagungseinrichtung, die in den Einlauf der Heizvorrichtung integriert ist, zu bestücken, da es aus Mangel von vorhandenem Bauraum hier zu Kollision kommen würde.

Durch die Reduzierung der Transporteinrichtungen im Gehäuse wird die Positionierung der Transporteinrichtungen bzw. der Behältnisaußenflächenbehandlungseinrichtung und der Behältnisinnenflächenbehandlungseinrichtung zueinander vereinfacht und es ergibt sich dadurch ein höherer Freiheitsgrad bei der Anordnung dieser. Auf diese Weise kann vorteilhaft eine Anordnung von Heizvorrichtung und Umformungseinrichtung in einer 0°-Stellung realisiert werden. Auch alle anderen Winkelpositionen zwischen 0° und 90° sind denkbar.

Hierdurch wird auch reichlich Bauraum geschaffen, um eine integrierte Beaufschlagungseinrichtung in der Heizvorrichtung zu bestücken.

Bei einer vorteilhaften Ausführungsform handelt es sich bei der Transporteinrichtung zwischen Heizvorrichtung und der Behältnisaußenflächenbehandlungseinrichtung bzw. der Behältnisinnenflächenbehandlungseinrichtung um einen Teilungsverzugsstern. Dies lässt wiederum eine geschicktere Anordnung der einzelnen vor- und nachgelagerten Maschinen-Modulen zu.

Die vorliegende Erfindung ist weiterhin auf ein Verfahren zum Behandeln und insbesondere zum Sterilisieren von Behältnissen, insbesondere von Kunststoffvorformlingen, gerichtet. Dabei wird ein Behältnis entlang eines vorgegebenen Transportpfads, welcher wenigstens teilweise innerhalb eines Gehäuses liegt, von mehreren Transporteinrichtungen transportiert, wobei das Behältnis an mindestens einer Transporteinrichtung an seiner Behältnisaußenfläche behandelt und insbesondere sterilisiert wird, wobei das Behältnis an mindestens einer Transporteinrichtung an seiner Behältnisinnenfläche behandelt und insbesondere sterilisiert wird und wobei das Behältnis während einer Behältnisbehandlung und/oder des Transports entlang des Transportpfads von Halteeinrichtungen gehalten wird.

Erfindungsgemäß wird das Behältnis im Rahmen der Behandlung an der Behältnisaußenfläche und/oder der Behältnisinnenfläche mit einer Strahlung, insbesondere einer Elektronenstrahlung, beaufschlagt und /oder das Behältnis wird erfindungsgemäß von einer Beaufschlagungseinrichtung mit einem fließfähigen Medium beaufschlagt.

Dabei ist die beschriebene Vorrichtung insbesondere dazu eingerichtet und dafür vorgesehen, dieses beschriebene Verfahren durchzuführen, d.h. dass alle für die obig beschriebene Vorrichtung ausgeführten Merkmale ebenso für das hier beschriebene Verfahren offenbart sind und umgekehrt.

Bei einem vorteilhaften Verfahren erfolgt die Beaufschlagung des Behältnisses mit einem fließfähigen Medium zeitlich vor der Behandlung der Behältnisaußenfläche und/oder der Behältnisinnenfläche mit einer Strahlung. Insbesondere wird das Behältnis bevorzugt zuerst mit einem fließfähigen Medium vorgereinigt und anschließend mit Elektronenstrahlung entkeimt. Hierdurch können besonders gute Entkeimungsergebnisse erzielt werden.

Bevorzugt erfolgt die Beaufschlagung des Behältnisses mit einem fließfähigen Medium zeitlich vor einer Erwärmung des Behältnisses. Hierdurch können eventuell vorhandene Reste des fließfähigen Mediums besser verdunsten.

Vorteilhaft wird das Behältnis von der Beaufschlagungseinrichtung unmittelbar an die Heizvorrichtung übergeben. Hierdurch wird zum einen vorteilhaft das Risiko einer Verunreinigung vermindert, da ein möglichst kurzer Weg zwischen der Beaufschlagungseinrichtung und der Behältnisaußenflächenbehandlungseinrichtung und der Behältnisinnenflächenbehandlungseinrichtung realisiert wird. Zum anderen wird ermöglicht, dass die Beaufschlagungseinrichtung in die Heizvorrichtung integriert sein kann.

Die vorliegende Erfindung auf eine Verbesserung insbesondere beim Transport von Behältnissen gerichtet. Im Bereich der getränkeherstellenden Industrie ist es seit langem bekannt, dass der Herstellungsprozess von Behältnissen eine Vielzahl von Behältnisbehandlungseinrichtungen und Transporteinrichtungen umfasst. Ebenso ist bekannt, dass für den Transport der Behältnisse lineare und/oder rotierende Transporteinrichtungen verwendet werden.

Im Stand der Technik ist eine Vielzahl an Halteeinrichtungen bekannt, welche die Behältnisse während einer Behandlung und/oder während des Transports halten. Beispielsweise sind solche Halteeinrichtungen bekannt, welche die Behältnisse unterhalb des Tragrings greifen, wobei dieser locker auf der Halteeinrichtung aufliegt. Weiterhin sind solche Halteeinrichtungen bekannt, welche die Behältnisse im Bereich einer Mundstücksnut (schmaler Bereich zwischen Verschlussring und Tragring) greifen oder solche Halteeinrichtungen, welche die Behältnisse im Bereich der Mündung, beispielsweise durch Haltedorne, greifen.

Die vorliegende Erfindung wird hier unter Bezugnahme auf die Sterilisation von Behältnissen beschrieben, was auch eine besonders bevorzugte Anwendung der Erfindung darstellt. Es wird jedoch darauf hingewiesen, dass die Erfindung auch bei anderen Vorrichtungen, in denen Halteeinrichtungen zum Halten von Behältnissen zum Einsatz kommen, anwendbar ist.

Im Rahmen der bekannten Herstellungsprozesse von Behältnissen müssen diese immer wieder zwischen zwei Transporteinrichtungen und/oder Behältnisbehandlungseinrichtungen, genauer gesagt zwischen deren Halteeinrichtungen übergeben werden, beispielsweise zwischen zwei Transportsternen.

Hierfür öffnet am Überlappungspunkt die Halteeinrichtung (Klammer) des einen Transportsterns und lässt das Behältnis los, während die Halteeinrichtung des anderen Transportsterns schließt und somit das Behältnis klemmt oder zumindest umschließt und weiter transportiert. Hierfür ist es nötig, dass das Behältnis an unterschiedlichen Positionen von der Halteeinrichtung (Klammern) gegriffen wird. Dabei unterscheidet man Halteeinrichtungen (Klammern), bei denen der Tragring aufliegt und das Behältnis etwas unterhalb am Durchmesser geklemmt wird und Halteeinrichtungen (Klammern), die zwischen dem Verschlussring und dem Tragring, der so genannten Mundstücksnut, greifen. Der Abstand zwischen der Unterkante des Tragrings und der Mundstücksnut ist sehr gering, wodurch bei der Übergabe nur sehr wenig Platz zwischen den Halteeinrichtungen (Klammerpaaren) ist, was die Gestaltung der Halteeinrichtungen (Klammern) sehr einschränkt.

Die im Stand der Technik bekannten Halteeinrichtungen zum Greifen des Behältnisses unterhalb des Tragrings weisen den Nachteil auf, dass der Tragring des Behältnisses nur aufliegt und nicht wie bei einer Haltereinrichtung mit einer Mundstücksnut-Klammer die Ausrichtung durch den Verschlussring und den Tragring sichergestellt ist. Insbesondere bei der Verwendung von Behältnissen mit einem kleineren Tragring oder Ähnlichem kann es dazu kommen, dass das Behältnis nicht gerade in der Halteeinrichtung sitzt.

Neben möglichen Problemen bei der Übergabe zwischen zwei Transporteinrichtungen können auch Probleme bei der Behandlung der Behältnisse, beispielsweise bei der Innensterilisation durch Elektronenstrahlen auftreten, bei welcher ein Strahlrohr in das Behältnis eingetaucht wird. Steht das Behältnis schräg, so stößt das Rohr an und es kann die Sterilität des Behältnisses nicht gewährleistet werden, wodurch dieses aus der Produktionskette genommen werden muss. Außerdem kann durch eine Kollision auch das Strahlrohr und damit der gesamte Elektronenstrahlemitter zerstört werden.

Der vorliegenden Erfindung liegt daher die Aufgabe zugrunde, geeignete Halteeinrichtungen bereit zu stellen, die gleichzeitig ein stabiles Greifen der Behältnisse gewährleisten und gleichzeitig eine sichere Übergabe der Behältnisse zwischen zwei Transporteinrichtungen zu gewährleisten. Diese Aufgabe wird erfindungsgemäß durch eine Vorrichtung und ein Verfahren nach den unabhängigen Ansprüchen erreicht. Vorteilhafte Ausführungsformen und Weiterbildungen sind Gegenstand der Unteransprüche.

Eine erfindungsgemäße Behältnisbehandlungsvorrichtung, insbesondere eine Behältnissterilisationsvorrichtung weist eine Transportvorrichtung zum Transport eines Behältnisses entlang eines vorgegeben Transportpfads auf, wobei die Transportvorrichtung wenigstens eine Behältnisbehandlungseinrichtung, bevorzugt eine Behältnisaußenflächenbehandlungseinrichtung und/oder besonders bevorzugt eine Behältnisinnenflächenbehandlungseinrichtung, wobei das Behältnis, insbesondere ein Kunststoffvorformling oder eine Kunststoffflasche, einen Kopfbereich mit einer Mündung, einem Verschlussring, einer Mundstücksnut und einem Tragring aufweist.

Daneben weist die Transportvorrichtung wenigstens eine erste Transporteinrichtung und wenigstens eine zweite Transporteinrichtung auf, wobei die erste Transporteinrichtung wenigstens eine erste Halteeinrichtung zum Halten eines Behältnisses und die zweite Transporteinrichtung wenigstens eine zweite Halteeinrichtung zum Halten eines Behältnisses während einer Behältnisbehandlung und/oder Transports entlang des Transportpfads aufweist.

Erfindungsgemäß ist der Tragring des Behältnisses insbesondere die Außenfläche des Tragrings und/oder bevorzugt die Unterseite des Tragrings und/oder besonders bevorzugt die Oberseite des Tragrings durch die wenigstens eine erste Halteeinrichtung und die Mundstücksnut des Behältnisses, insbesondere direkt an der Unterseite des Verschlussrings anliegend durch die wenigstens eine zweite Halteeinrichtung erfassbar.

Die vorgeschlagenen Halteeinrichtungen bieten den Vorteil, dass ein auf diese Weise gehaltenes Behältnis fixiert wird, also der Tragring bzw. die Mundstücksnut eingeklemmt ist und somit ein seitliches Verkippen bzw. eine schräge Anordnung des Behältnisses in der Halteeinrichtung effektiv vermieden werden kann.

Bevorzugt umfasst eine Behältnisbehandlungsvorrichtung, insbesondere eine Behältnissterilisationsvorrichtung, wenigstens eine Behältnisbehandlungseinrichtung, besonders bevorzugt eine Vielzahl von Behältnisbehandlungseinrichtungen. Bevorzugt ist die wenigstens eine Behältnisbehandlungseinrichtung ausgewählt aus einer Gruppe von Behältnisbehandlungseinrichtungen umfassend eine Behältnisaußenflächenbehandlungseinrichtung (insbesondere eine Behältnisaußenflächensterilisationseinrichtung), eine Behältnisinnenflächenbehandlungseinrichtung (insbesondere eine Behältnisinnenflächensterilisationseinrichtung), eine Erwärmungseinrichtung, eine Umformungseinrichtung, eine Abfülleinrichtung, eine Verschlusseinrichtung, eine Inspektionseinrichtung und dergleichen.

Bevorzugt umfasst die Transportvorrichtung wenigstens eine erste Transporteinrichtung und wenigstens eine zweite Transporteinrichtung. Bei einer vorteilhaften Ausführungsform weist die wenigstens eine erste und/oder zweite Transporteinrichtung einen rotierbaren Träger auf und ist bevorzugt ein Transportstern.

Es wäre jedoch auch hier der Einsatz von einem Langstator möglich, an dem eine Vielzahl von Shuttles angeordnet ist, wie oben beschrieben.

Bei einer weiteren vorteilhaften Ausführungsform ist die wenigstens eine erste und/oder zweite Transporteinrichtung einer Behältnisbehandlungseinrichtung zugeordnet, welche dazu geeignet und bestimmt ist, das Behältnis während des Transports zu behandeln. Besonders bevorzugt ist die wenigstens eine erste und/oder zweite Transporteinrichtung Teil einer Behältnisbehandlungseinrichtung.

Bevorzugt handelt es sich bei dem zu behandelnden Behältnis um eine Kunststoffflasche und besonders bevorzugt um einen Kunststoffvorformling. Das Behältnis weist bevorzugt einen Kopfbereich auf, welche eine Mündung, ein (Außen-)Gewinde, ein Verschlussring, eine Mundstücksnut und einen Tragring umfasst.

Bevorzugt weist der Verschlussring einen geringeren Durchmesser als der Tragring auf. Besonders bevorzugt weist das Behältnis zwischen dem Verschlussring und dem Tragring eine (sogenannte) Mundstücksnut auf.

Bevorzugt weist eine erste Transporteinrichtung wenigstens eine erste Halteeinrichtung zum Halten eines Behältnisses und besonders bevorzugt eine Vielzahl solcher Halteeinrichtungen auf. Bevorzugt weist eine zweite Transporteinrichtung wenigstens eine zweite Halteeinrichtung zum Halten eines Behältnisses und besonders bevorzugt eine Vielzahl solcher Halteeinrichtungen auf. Besonders bevorzugt unterschieden sich die erste und die zweite Halteeinrichtung durch einen zu erfassenden Bereich des Behältnisses und/oder durch Kontakt- oder Aufnahmebereiche, die zum Erfassen dieses Bereichs des Behältnisses geeignet und bestimmt sind.

Bevorzugt ist die wenigstens eine erste und/oder zweite Halteeinrichtung dazu geeignet und bestimmt, ein Behältnis (fest) zu greifen und in der Lage ist, das Behältnis zu stabilisieren, und besonders bevorzugt in der Lage, ein seitliches Verkippen des Behältnisses zu verhindern. Bevorzugt umschließt die wenigstens eine erste und/oder zweite Halteeinrichtung wenigstens abschnittsweise das Behältnis, insbesondere einen Teil des Kopfbereichs des Behältnisses.

Bei einer vorteilhaften Ausführung ist die wenigstens eine erste und/oder die wenigstens eine zweite Halteeinrichtung klammerartig ausgebildet.

Bevorzugt ist die wenigstens erste und/oder zweite Halteeinrichtung zweiteilig aufgebaut, wobei bevorzugt zwei Halteelemente bzw. Klammerelemente (bzw. Klammerarme) an einer gemeinsamen Schwenkachse beweglich angeordnet sind. Bevorzugt kann die wenigstens erste und/oder zweite Halteeinrichtung in einer geschlossen bzw. greifenden Stellung betrieben werden und/oder in einer geöffneten Stellung, wobei bevorzugt der Abstand zwischen den beiden Enden der Halteelemente bzw. Klammerelemente in dieser Stellung größer ist als der Durchmesser des Behältnisses und/oder ein Außendurchmesser eines Mündungsbereichs des Behältnisses.

Insbesondere ist die wenigstens eine erste Halteeinrichtung dazu geeignet und bestimmt, den Tragring des Behältnisses zu erfassen. Insbesondere ist die wenigstens eine zweite Halteeinrichtung dazu geeignet und bestimmt, die Mundstücksnut des Behältnisses zu erfassen. Bei einer vorteilhaften Ausführung weist die erste Halteinrichtung eine Haltenut zur Erfassung des Tragring des Behältnisses auf. Bevorzugt erfasst die wenigstens eine erste Halteinrichtung die Unterseite des Tragrings des Behältnisses wenigstens abschnittsweise und/oder die Außenseite des Tragrings wenigstens abschnittsweise und/oder die Oberseite des Tragrings wenigstens abschnittsweise. Besonders bevorzugt umfasst die wenigstens eine erste Halteeinrichtung die Unterseite, die Oberseite und die Außenseite des Tragrings zumindest abschnittsweise. Dies bietet den Vorteil, dass das Behältnis in der wenigsten einen ersten Halteeinrichtung (optimal) fixiert wird und gegen ein seitliches Verkippen gesichert ist.

Bevorzugt ist die wenigstens eine zweite Halteeinrichtung dazu geeignet und bestimmt, das Behältnis im Bereich der Mundstücksnut zu greifen. Besonders bevorzugt wird der obere Bereich der Mundstücksnut erfasst, wobei die wenigstens eine zweite Halteeinrichtung an der Unterseite des Verschlussrings des Behältnisses anliegt.

Bei einer vorteilhaften Ausführungsform sind die wenigstens eine erste Transporteinrichtung und die wenigste eine zweite Transporteinrichtung innerhalb der Behältnisbehandlungsvorrichtung aneinander anschließend angeordnet.

Bei einer vorteilhaften Ausführungsform weist die Behältnisbehandlungsvorrichtung wenigstens einen Übergabebereich auf, in welchem das Behältnisses zwischen der wenigstens einen ersten Transporteinrichtung und der wenigstens einen zweiten Transporteinrichtung übergebbar ist.

Bevorzugt ist der Übergabebereich derart gestaltet, dass die wenigstens eine erste und die wenigstens eine zweite Transporteinrichtung tangential zueinander verlaufen. Besonders bevorzugt überschneiden sich der Transportpfad der an der wenigstens einen ersten Transporteinrichtung angeordneten wenigstens einen ersten Halteeinrichtung und der Transportpfad der an der wenigstens einen zweiten Transporteinrichtung angeordneten wenigstens einen zweiten Halteeinrichtung.

Bei einer vorteilhaften Ausführungsform sind die wenigstens eine erste Halteeinrichtung und die wenigstens eine zweite Halteeinrichtung im Übergabebereich überlappend zueinander und/oder im Wesentlichen senkrecht zu einer Längsachse des Behältnisses anordenbar.

Dies bedeutet, dass sich in dem Übergabebereich, in welchem sich die Transportpfade der wenigstens einen ersten und zweiten Halteeinrichtungen überschneiden, die beiden Halteeinrichtungen (im Wesentlichen) parallel zueinander und (im Wesentlichen) senkrecht zur Längsachse des Behältnisses befinden. Da die beiden Halteeinrichtungen sowohl in den Tragring als auch den direkt daran anschließenden Bereich der Mundstücksnut eingreifen kommen sich die beiden Halteeinrichtungen sehr nahe, was mit den derzeit im Stand der Technik üblichen Halteeinrichtungen nicht möglich wäre.

Besonders bevorzugt überlappen sich Abschnitte der ersten Halteeinrichtung und der zweiten Halteeinrichtung in einem Bereich der Übergabe der Behältnisse.

In einer vorteilhaften Ausführungsform ist das dem Behältnis zugewandte Ende der wenigstens einen ersten und der wenigsten einen zweiten Halteeinrichtung verjüngt, insbesondere komplementär zueinander ausgebildet.

Dies bietet den Vorteil, dass die erste und die zweite Halteinrichtung sehr nahe übereinander angeordnet werden können, ohne sich zu berühren. Durch die Verwendung von Halteinrichten gemäß der vorliegenden Erfindung ist es möglich, ein Behältnis zwischen zwei Transporteinrichtungen zu übergeben, welche beide das Behältnis fest greifen und das Behältnis gegen seitliches Verkippen schützen.

Im Stand der Technik ist für eine solche Übergabe (zwischen einer Transporteinrichtung mit einer Halteeinrichtung, die den Tragring greift und einer Transporteinrichtung mit einer Halteinrichtung, die die Mundstücksnut greift) eine zusätzliche Transporteinrichtung, beispielsweise ein weiterer Transportstern nötig, welche einen anderen Bereich des Behältnisses, beispielsweise unterhalb des Tragrings greift.

Die Verwendung der vorgeschlagenen Halteeinrichtungen bietet den Vorteil, dass dadurch auf einen weiteren Transportstern verzichtet werden kann und somit der technische Aufwand und die räumliche Ausdehnung der Anlage deutlich reduziert werden kann.

Die vorliegende Erfindung ist weiterhin gerichtet auf ein Verfahren zur Behandlung von Behältnissen. Das vorgeschlagene Verfahren kann dabei alle im Zusammenhang mit der obig beschriebenen Behältnisbehandlungsvorrichtung beschriebenen Merkmalen einzeln oder in Kombination miteinander aufweisen und/oder diese ausführen.

Weiterhin ist die vorliegende Erfindung auf ein Verfahren zur Behandlung von Behältnissen mit einer Behältnisbehandlungsvorrichtung, insbesondere mit einer Behältnissterilisationsvorrichtung gerichtet. Die Behältnisbehandlungsvorrichtung weist eine Transportvorrichtung auf, welche ein Behältnis entlang eines vorgegebenen Transportpfads transportiert, wobei die Transportvorrichtung wenigstens eine Behältnisbehandlungseinrichtung aufweist und das Behältnis einen Kopfbereich mit einer Mündung, einem Verschlussring, einer Mundstücksnut und einem Tragring aufweist. Die Transportvorrichtung weist wenigstens eine erste Transporteinrichtung und wenigstens eine zweite Transporteinrichtung auf, wobei die erste Transporteinrichtung wenigstens eine erste Halteeinrichtung zum Halten eines Behältnisses und die zweite Transporteinrichtung wenigstens eine zweite Halteeinrichtung zum Halten eines Behältnisses während einer Behältnisbehandlung und/oder des Transports entlang des Transportpfads aufweist.

Erfindungsgemäß erfasst die wenigstens eine erste Halteinrichtung den Tragring des Behältnisses, insbesondere die Außenfläche des Tragrings und/oder bevorzugt die Unterseite des Tragrings und/oder besonders bevorzugt die Oberseite des Tragrings und die wenigstens eine zweite Halteeinrichtung die Mundstücksnut, insbesondere direkt an der Unterseite des Verschlussrings des Behältnisses.

Das vorgeschlagene Verfahren bietet den Vorteil, dass sowohl die erste als auch die zweite Halteeinrichtung ein festes Greifen des Behältnisses ermöglicht und gleichzeitig einen Schutz gegen seitliches Verkippen bietet.

Bei einer vorteilhaften Ausführungsform erfolgt die Übergabe des Behältnisses zwischen der wenigstens einen ersten Transporteinrichtung und der wenigstens einen zweiten Transporteinrichtung in einem Übergabebereich, wobei die wenigstens eine erste Halteeinrichtung und die wenigstens eine zweite Halteeinrichtung während der Übergabe überlappend angeordnet sind. Insbesondere sind die erste und zweite Halteeinrichtung in einer senkrecht zu dem Transportpfad der Behältnisse stehenden Richtung (insbesondere überpappend) angeordnet

Bei einem bevorzugten Verfahren sind zum Zeitpunkt der Übergabe die wenigstens eine erste Halteeinrichtung und die wenigstens eine zweite Halteeinrichtung direkt übereinander angeordnet, während das Behältnis durch eine der beiden Halteeinrichtungen gehalten wird.

Bei einer vorteilhaften Ausführungsform läuft die Übergabe des Behältnisses zwischen der wenigstens einen ersten Transporteinrichtung und der wenigstens einen zweiten Transporteinrichtung synchronisiert ab und/oder erfolgt ohne Berührung der wenigstens einen ersten Halteeinrichtung und der wenigstens einen zweiten Halteeinrichtung miteinander.

Bevorzugt erfolgt das Greifen der wenigstens einen ersten Halteeinrichtung bzw. der wenigstens einen zweiten Halteeinrichtung und das Öffnen (Loslassen) der wenigstens zweiten Halteeinrichtung bzw. der wenigstens einen ersten Halteeinrichtungen zeitgleich und bevorzugt synchronisiert zueinander ab. Besonders bevorzugt ist die Übergabe des Behältnisses (Greifen bzw. Öffnen) mit der Bewegung der wenigsten einen ersten Transporteinrichtung und/oder der wenigstens einen zweiten Transporteinrichtung synchronisiert.

Weiterhin ist die vorliegende Erfindung auf eine Halteanordnung zum Halten eines Behältnisses während einer Behältnisbehandlung und/oder eines Transports entlang eines vorgegebenen Transportpfads, insbesondere mit wenigstens einer ersten und mit wenigstens einer zweiten Transporteinrichtung, gerichtet, insbesondere in einem Übergabebereich zwischen der wenigsten einen ersten und der wenigstens einen zweiten Transporteinrichtung.

Die Halteanordnung weist wenigstens eine erste Halteeinrichtung und wenigstens eine zweiten Halteeinrichtung auf, wobei bevorzugt die wenigstens eine erste Halteeinrichtung der wenigstens einen ersten Transporteinrichtung und die wenigstens eine zweite Halteeinrichtung der wenigstens einen zweiten Transporteinrichtung zugeordnet ist, wobei die wenigstens eine erste und die wenigstens eine zweite Halteeinrichtung jeweils wenigstens zwei Halteelemente, bevorzugt Klammerelemente aufweist, welche dazu geeignet und bestimmt sind, einen Kopfbereich eines Behältnisses zu greifen.

Der Kopfbereich des Behältnisses weist bevorzugt eine Mündung, einen optionalen Verschlussring, eine Mundstücksnut und einen Tragring auf. Die jeweils wenigstens zwei Halteelemente sind über eine gemeinsame Schwenkachse schwenkbar gelagert und dazu geeignet und bestimmt, in einer das Behältnis greifenden Position oder in einer das Behältnis freigebenden Position angeordnet zu werden.

Erfindungsgemäß weisen die jeweils wenigstens zwei Halteelemente jeweils einen abschließenden Bereich auf, welcher dem zu haltenden Behältnis zugewandt ist und verjüngt ausgebildet ist, wobei die wenigstens eine erste und die wenigstens eine zweite Halteeinrichtung überlappend anordenbar sind.

Bevorzugt erfolgt eine Übergabe des Behältnisses von der wenigstens einen ersten Transporteinrichtung an die wenigstens eine zweite Transporteinrichtung bzw. in umgekehrter Reihenfolge in einer Übergabeposition, in der die wenigstens eine erste und die wenigstens eine zweite Halteeinrichtung direkt übereinander (überlappend) angeordnet sind. Bevorzugt überlappen in dieser Übergabeposition die wenigstens eine erste Halteeinrichtung und die wenigstens eine zweite Halteeinrichtung dahingehend, dass der verjüngte Bereich der Halteelemente der einen Halteeinrichtung mit einem nicht-verjüngten Bereich der anderen Halteeinrichtung übereinander liegen. Besonders bevorzugt berühren sich die wenigstens eine erste und die wenigstens eine zweite Halteeinrichtung, insbesondere deren Halteelemente niemals.

Bei einer vorteilhaften Ausführung weisen die verjüngten abschließenden Bereiche der Halteelemente der wenigstens einen ersten und der wenigstens einen zweiten Halteeinrichtung aufeinander zu und sind komplementär zueinander ausgebildet.

Bei einer vorteilhaften Ausführung sind die wenigstens eine erste und die wenigstens eine zweite Halteeinrichtung baugleich und können bevorzugt an der wenigsten einen ersten Transporteinrichtung und/oder der wenigstens einen zweiten Transporteinrichtung angeordnet werden und besonders bevorzugt in zwei verschiedenen Positionen angeordnet werden.

Die beiden Positionen unterscheiden sich formal durch eine Spiegelung an einer horizontalen Ebene. Bevorzugt kann somit der verjüngte Bereich der Halteelemente entweder nach oben oder nach unten zeigen.

Bevorzugt sind die wenigstens eine erste und die wenigstens eine zweite Halteeinrichtung, insbesondere deren Halteelemente (Klammern) derart ausgestaltet, dass sowohl ein vorstehender Teil des Behältnisses, insbesondere der Tragring des Behältnisses erfasst werden kann als auch ein flacher Abschnitt des Behältnisses, insbesondere die Mundstücksnut bevorzugt direkt unterhalb des Verschlussrings gegriffen werden kann.

Weitere Vorteile und Ausführungsformen ergeben sich aus den beigefügten Zeichnungen.

Darin zeigen:
- Fig. 1: eine schematische Aufsicht auf eine Behältnisbehandlungsvorrichtung in einer beispielhaften Ausführungsform;
- Fig. 2: eine weitere schematische Aufsicht auf eine Behältnisbehandlungsvorrichtung in einer weiteren beispielhaften Ausführungsform mit einer senkrechten Projektion eines Transportpfads;
- Fig. 3: eine schematische Darstellung von Teilen zweier Behältnisflächenbehandlungseinrichtungen;
- Fig. 4: eine schematische Darstellung eines beispielhaften Profils der Verschiebung einer Halteeinrichtung und/oder eines Behältnisses entlang der Höhenrichtung auf einer Behältnisflächenbehandlungseinrichtung;
- Fig. 5a: eine beispielhafte Zuordnung verschiedener Sektoren einer Kreisbahn zu ver-schiedenen Prozessschritten bei einer Behandlungseinrichtung gemäß dem Stand der Technik;
- Fig. 5b: eine beispielhafte Zuordnung verschiedener Sektoren einer Kreisbahn zu verschiedenen Prozessschritten bei einer Behandlungseinrichtung gemäß der bevorzugten Ausführungsform der vorliegenden Erfindung;
- Fig. 6: eine schematische Darstellung einer Übergabe von der ersten Transporteinrichtung an die zweite Transporteinrichtung;
- Fig. 7: eine weitere schematische Darstellung einer Übergabe von der ersten Transporteinrichtung an die zweite Transporteinrichtung;
- Fig. 8: eine Detaildarstellung einer Übergabe von der ersten Transporteinrichtung an die zweite Transporteinrichtung;
- Fig. 9: eine Schnittdarstellung einer Übergabe von der ersten Transporteinrichtung an die zweite Transporteinrichtung;
- Fig. 10: eine weitere Darstellung einer Transporteinrichtung mit einer Außensterilisationseinrichtung;
- Fig. 11: eine Schrägansicht der in Fig. 10 gezeigten Vorrichtung;
- Fig. 12: eine Detaildarstellung der Außenbehandlungseinrichtung;
- Fig. 13: eine Schnittdarstellung der Außenbehandlungseinrichtung;
- Fig. 14: eine Darstellung des Transports der Kunststoffvorformlinge;
- Fig. 15: eine Darstellung einer Hub- Dreheinrichtung mit einem Kunststoffvorformling;
- Fig. 16: eine Darstellung der Hub- Dreheinrichtung;
- Fig. 17: eine Darstellung zur Erzeugung von Drehbewegungen der Halteeinrichtung;
- Fig. 18: eine Darstellung zur magnetischen Übertragung von Drehbewegungen in einer ersten Ausgestaltung;
- Fig. 19: eine Darstellung zur magnetischen Übertragung von Drehbewegungen in einer zweiten Ausgestaltung;
- Fig. 20: eine Darstellung zur magnetischen Übertragung von Drehbewegungen in einer dritten Ausgestaltung;
- Fig. 21: eine Darstellung zur magnetischen Übertragung von Drehbewegungen in einer vierten Ausgestaltung;
- Fig. 22: eine Darstellung zur magnetischen Übertragung von Drehbewegungen in einer vierten Ausgestaltung;
- Fig. 23: eine Darstellung eines Zusammenhangs zwischen einer Drehzahl und einer Erzeugung von Wirbelströmen;
- Fig. 24: eine Darstellung zur magnetischen Übertragung von Drehbewegungen in einer fünften Ausgestaltung;
- Fig. 25: eine Darstellung zur magnetischen Übertragung von Drehbewegungen in einer sechsten Ausgestaltung;
- Fig. 26: eine Darstellung einer vorteilhaften Vorrichtung mit einer Schleuseneinrichtung;
- Fig. 27: eine weitere Darstellung einer vorteilhaften Vorrichtung mit einer Schleuseneinrichtung;
- Fig. 28: eine Darstellung einer vorteilhaften Vorrichtung mit einem Rinser;
- Fig. 29: eine Darstellung einer Halteeinrichtung zum Halten von Kunststoffvorformlingen; und
- Fig. 30: eine weitere Darstellung einer Halteeinrichtung zum Halten von Kunststoffvorformlingen.

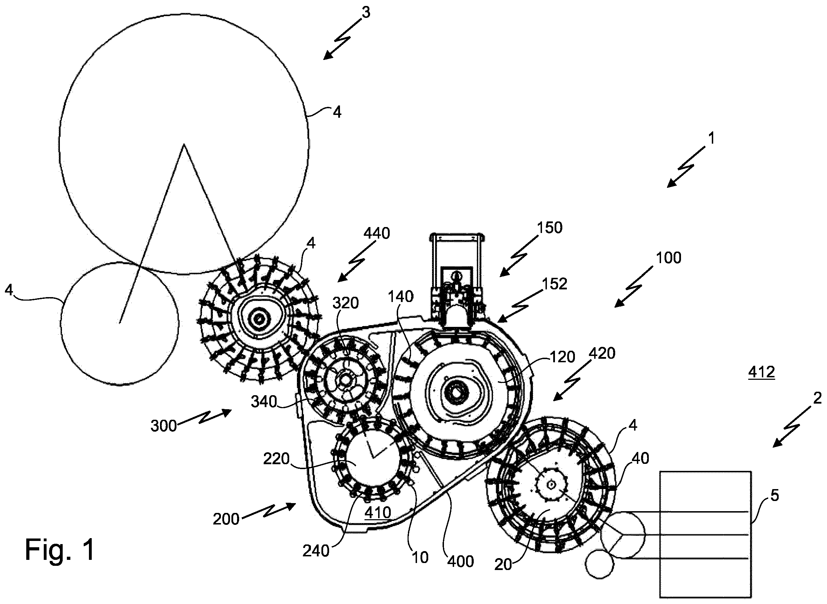

Figur 1 zeigt eine schematische Aufsicht auf eine Behältnisbehandlungsvorrichtung 1 in einer beispielhaften Ausführungsform. Die gezeigte Behältnisbehandlungsvorrichtung 1, bei der es sich im gezeigten Beispiel um eine Behältnissterilisationsvorrichtung handelt, weist mehrere Transporteinrichtungen 100, 200, 300 zum Transport eines Behältnisses 10 entlang eines in dieser Darstellung nicht hervorgehobenen Transportpfads innerhalb eines Gehäuses 400 auf. Eine dieser Transporteinrichtungen 100, 200, 300 ist eine Behältnisaußenflächenbehandlungseinrichtung 100 und eine andere eine Behältnisinnenflächenbehandlungseinrichtung 200. Diese weisen jeweils eine Vielzahl von Halteeinrichtungen 140, 240 zum Halten mindestens eines Behältnisses 10 während einer Behältnisbehandlung und/oder des Transports entlang des Transportpfads auf.

Zumindest eine Halteeinrichtung 140 der Behältnisaußenflächenbehandlungseinrichtung 100 kann zumindest zeitweise in den Bereich einer Öffnung 420 der Gehäusewandung 400 gebracht werden. Dadurch wird ermöglicht, dass dieses Halteelement 140 aus einem Raum 412 außerhalb des Gehäuses 400 von einer dort stromaufwärts bezüglich des Transportpfads angeordneten Transporteinrichtung 4 ein Behältnis aufnehmen kann. Durch diese Ausgestaltung kann auf eine zusätzliche Transporteinrichtung innerhalb des Gehäuses verzichtet werden, die das Behältnis von außerhalb aufnimmt und an die Behältnisaußenflächenbehandlungseinrichtung 100 weiterleitet.

Die Transporteinrichtung 4 ist in der gezeigten Ausführungsform stromabwärts einer Heizvorrichtung 2 angeordnet, die mindestens eine Heizeinrichtung 5 wie beispielsweise einen Ofen oder eine (beispielsweise Infrarot- oder Mikrowellen-) Strahlungsquelle umfasst. Die Transporteinrichtung 4 weist zur Aufnahme der Behältnisse 10 eine Vielzahl von Halteelementen 40 auf, die gemeinsam auf einem rotierbaren Träger 20 angeordnet sind.

Teil der dargestellten Behältnisaußenflächenbehandlungseinrichtung 100 ist eine Behältnisaußenflächenbeaufschlagungseinrichtung 150, die im gezeigten Beispiel eine Strahlungsquelle 150 ist. Denkbar wäre jedoch auch deren Ausgestaltung als Düse oder Düsenanordnung, mittels welcher ein fluides Medium wie eine Sterilisationslösung oder ein sterilisierendes Gas auf die zu sterilisierende Behältnisfläche aufgebracht wird.

In der Behältnisaußenflächenbehandlungseinrichtung 100 werden die Behältnisse durch die Haltelemente 140 in einem Behältnisaußenflächenbeaufschlagungsbereich 152 an der Behältnisaußenflächenbeaufschlagungseinrichtung 150 vorbeigeführt. Während dieses Transports werden Sie mit dem Sterilisierenden Medium - in diesem Fall sterilisierende Strahlung - beaufschlagt. Vorteilhaft werden die Behältnisse 10 dabei nicht nur entlang des Transportpfads bewegt, sondern wie an anderer Stelle beschrieben ist in mindestens einer weiteren Richtung bewegt. Diese Bewegung kann eine Rotation (um eine Behältnislängsachse und/oder eine Behältnisquerachse) und/oder eine Verschiebung senkrecht zur Zeichenebene (also in Höhenrichtung H) umfassen. Durch eine solche individuelle Bewegung des zu behandelnden Behältnisses ist es möglich, alle zu sterilisierenden (Außen-) Flächen mit einer einzigen Behältnisaußenflächenbeaufschlagungseinrichtung 150 zu behandeln. Dauerhafte Abschattungen einzelner (Außen-) Oberflächen des Behältnisses 10 durch andere Abschnitte desselben Behältnisses können vermieden werden.

Bei der in Figur 1 dargestellten Ausführungsform wird ein Behältnis 10 nach der Behandlung durch die Behältnisaußenflächenbehandlungseinrichtung 100 unmittelbar an eine Behältnisinnenflächenbehandlungseinrichtung 200 übergeben. Diese umfasst ebenfalls eine Vielzahl von Halteeinrichtungen 240, die an einem rotierenden Träger 220 angeordnet sind. Ebenso ist eine Behältnisinnenflächenbeaufschlagungseinrichtung vorgesehen. Die Behältnisinnenflächenbeaufschlagungseinrichtung ist in der gezeigten Darstellung nicht deutlich erkennbar, da sie als eine Vielzahl von Strahlfingern ausgeführt ist, die sich jeweils mit einer Position zur Aufnahme eines Behältnisses 10 überdecken. Jedem Haltelement 240 ist somit ein solcher Strahlfinger zugeordnet. Während der Rotation des Trägers 220 werden die Behältnisse relativ zu demjenigen Strahlfinger bewegt, der dem von diesem Behältnis 10 besetzten Halteelement 240 zugeordnet ist. Diese Bewegung erfolgt in einem Abschnitt zwischen der Aufnahme deines Behältnisses 10 durch die Behältnisinnenflächenbehandlungseinrichtung 200 und der Abgabe an eine entlang des Transportpfads folgende Transporteinrichtung 300.

Vorzugsweise handelt es sich bei der dieser Transporteinrichtung 300 um einen Transportstern 300 mit einem rotierbaren Träger 320 und einer Vielzahl daran angeordneter Haltelemente 340. Dieser Transportstern 300 nimmt die von der Behältnisinnenflächenbehandlungseinrichtung 200 behandelten Behältnisse 10 auf und übergibt sie an eine außerhalb dieses Gehäuses 400 angeordnete weitere Transporteinrichtung 4. Vorzugsweise erfolgt auch der weitere Transport unter Bedingungen, die eine Kontamination der durch die Behältnisbehandlungsvorrichtung 1 behandelten Behältnisse 10 verhindert, beispielsweise in einem Reinraum.

Wie in dem in Fig. 1 gezeigten Beispiel dargestellt ist, ist der vom Gehäuse 400 umschlossene Bereich 410 besonders klein, was unter anderem mit der lediglich einen weiteren Transporteinrichtung 300 zusätzlich zu der Behältnisaußenflächenbehandlungseinrichtung 100 und der Behältnisinnenflächenbehandlungseinrichtung 200 innerhalb des Gehäuses 400 begründet werden kann.

Als der der Behandlung in der Behältnisbehandlungsvorrichtung 1 nachgeschaltete weitere Behandlung der Behältnisse kann beispielsweise deren Umformung zu anderen Behältnissen 10, wie beispielsweise Flaschen, durch eine lediglich schematisch angedeutete Umformungseinrichtung 3 erfolgen. Ergänzend oder alternativ dazu wäre das Befüllen (und gegebenenfalls Verschließen) der durch die Behältnisbehandlungsvorrichtung 1 behandelten Behältnisse 10 denkbar.

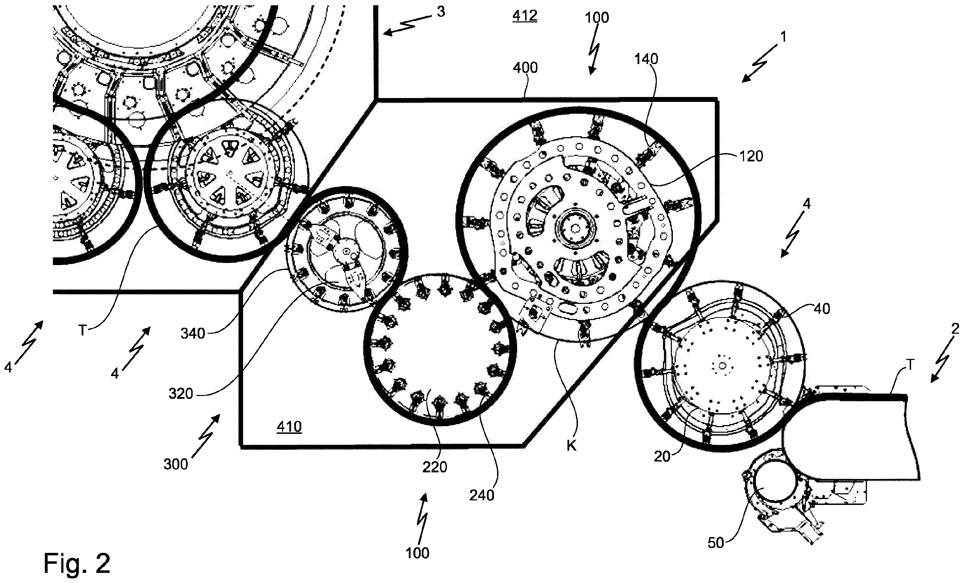

Fig. 2 stellt eine weitere schematische Aufsicht auf eine Behältnisbehandlungsvorrichtung 1 in einer weiteren beispielhaften Ausführungsform dar. Anders als in Fig. 1 ist ein Transportpfad T dargestellt, entlang dessen die (in dieser Figur nicht dargestellten) Behältnisse 10 während ihres Transports geführt werden. Der Transportpfad T ist in einer senkrechten Projektion dargestellt. Verschiebungen der Behältnisse während des Transports in der Höhenrichtung H, senkrecht zur Zeichenebene (und damit auch zu der Projektion des Transportpfads), sind daher in dieser Darstellung nicht erkennbar.

In dem gezeigten Ausführungsbeispiel erstreckt sich der dargestellte Abschnitt des Transportpfads T von einer Heizvorrichtung 2 über eine Transporteinrichtung 4 bis in die Behandlungseinrichtung 1 und von dieser über eine weitere Transporteinrichtung 4 zunächst bis zu einer Umformungseinrichtung 3 und anschließend weiter über eine weitere Transporteinrichtung 4 zum Abführen der Behältnisse von der Umformungseinrichtung 3. Entlang des Transportpfads wird ein Behältnis durch Halteeinrichtungen 40, 140, 240, 340 geführt, die jeweils an rotierbaren Trägern 20, 120, 220, 320 angeordnet sind. Der Abstand zweier sich entlang des Transportpfads unmittelbar aufeinander folgender Behältnisse 10 beziehungsweise Halteeinrichtungen 40, 140, 240, 340 kann mindestens einmal, bevorzugt mehrfach geändert werden. Insbesondere ist dies bevorzugt im Bereich einer (in Fig. 2 nicht im Detail dargestellten Beaufschlagungseinrichtung) und/oder im Bereich der Durchführung durch eine Wandung des Gehäuses 400, da bei einer in diesen Bereichen verlangsamten Bewegung entlang der Transportrichtung die Behandlungszeit verlängert werden kann, beziehungsweise das Fenster in der Gehäusewandung kleiner ausgebildet sein kann, jeweils bei gleicher Rotationsgeschwindigkeit des Trägers. Details dazu sind an anderer Stelle beschrieben.

Im Inneren des Gehäuses 400 sind auch in dem in Figur 2 dargestellten Ausführungsbeispiel genau drei Transporteinrichtungen 100, 200 und 300 angeordnet, nämlich die Behältnisaußenflächenbehandlungseinrichtung 100, die Behältnisinnenflächenbehandlungseinrichtung 200 und ein Transportstern 300. Anders als in Fig. 1 sind die Halteelemente 140 der Behältnisaußenflächenbehandlungseinrichtung 100 als außengreifende Klammern 140 und nicht als Dorne ausgebildet. Die Ausbildung der Halteelemente 140 kann den jeweiligen Bedürfnissen angepasst werden. Bevorzugte Ausgestaltungen der Halteelemente 140 der Behältnisaußenflächenbehandlungseinrichtung 100 sind ebenfalls an anderer Stelle detaillierter beschrieben. Wie auch der Fig. 2 zu entnehmen ist, kann es möglich sein, dass die Halteelemente 140 der Behältnisaußenflächenbehandlungseinrichtung 100 zumindest zeitweise und abschnittsweise außerhalb des Gehäuses 400 in der Umgebung 412 des Gehäuses 400 angeordnet sind, um ein Behältnis außerhalb des Gehäuses 400 aufzunehmen und dann in das Gehäuseinnere 410 einzubringen.

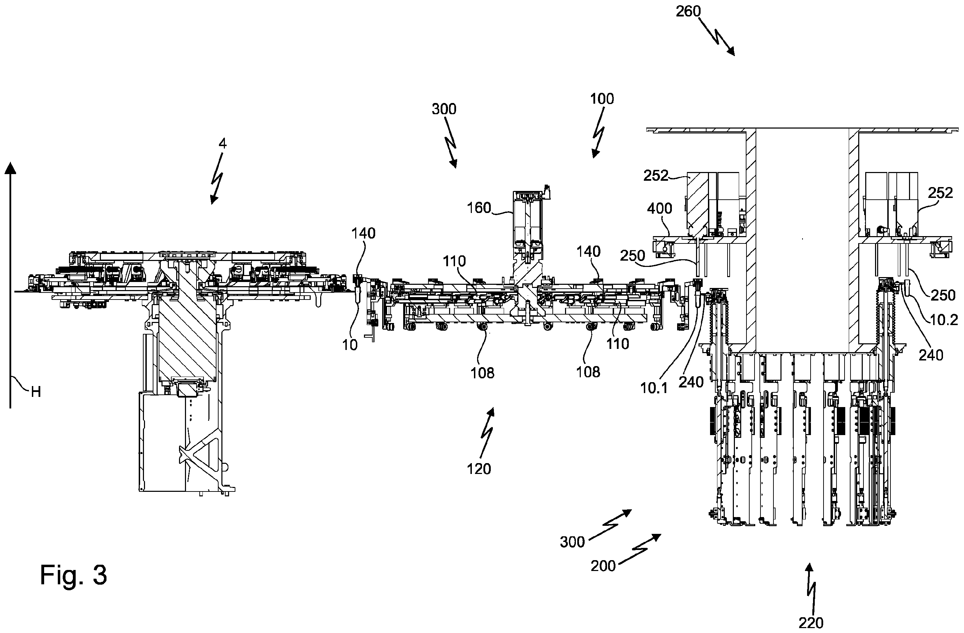

Fig. 3 zeigt eine schematische Darstellung von Teilen von Transporteinrichtungen 4, 100, 200, 300 zwischen denen ein Behältnis 10 bei dessen Bewegung entlang des Transportpfads übergeben wird. Bei den Transporteinrichtungen 300 handelt es sich im gezeigten Beispiel um eine Behältnisaußenflächenbehandlungseinrichtung 100 und eine Behältnisinnenflächenbehandlungseinrichtung 200. Die Behältnisaußenflächenbeaufschlagungseinrichtung beziehungsweise Strahlungsquelle der Behältnisaußenflächenbehandlungseinrichtung 100 ist in dieser Darstellung nicht gezeigt. Die Transporteinrichtung 4 ist eine außerhalb des (nicht dargestellten) Gehäuses angeordnete Transporteinrichtung, welche die Behältnisse an die Behältnisaußenflächenbehandlungseinrichtung 100 übergibt. Auf diese Transporteinrichtung 4 wird in der Beschreibung der Darstellung gemäß Fig. 3 nicht im Detail eingegangen.

Die Antriebseinrichtungen 160 und 260 der beiden (innerhalb des nicht gezeigten Gehäuses angeordneten) Behältnisflächenbehandlungseinrichtungen 100, 200 sind bezüglich des nicht eingezeichneten Transportpfads der Behältnisse 10 auf der der Antriebseinrichtung 6 der Transporteinrichtung 4 gegenüberliegenden Seite angeordnet. So sind die Antriebseinrichtung 160 der Behältnisaußenflächenbehandlungseinrichtung 100 und die Antriebseinrichtung 260 der Behältnisinnenflächenbehandlungseinrichtung 200 oberhalb des Transportpfads angeordnet und die Antriebseinrichtung 6 der Transporteinrichtung 4 unterhalb des Transportpfads. Bevorzugt befinden sie sich außerhalb des nur abschnittsweise dargestellten Gehäuses 400 um deren Wartung durchführen zu können, ohne das Gehäuse 400 öffnen zu müssen.

Die "hängende" Anordnung des Trägers 120 der Behältnisaußenflächenbehandlungseinrichtung 100 mit den daran angeordneten Halteeinrichtungen 140 an der Antriebseinrichtung ermöglicht es, einen vergleichsweise großen freien Raum innerhalb des nicht dargestellten Gehäuses unterhalb des Trägers 120 bereitzustellen. In diesen kann beispielsweise eine nicht gezeigte Steuerkurve angeordnet werden, auf welcher Führungsrollen 108 der Behältnisaußenflächenbehandlungseinrichtung 100 entlangrollen können. Der oben beschriebene Raum ist insbesondere vorteilhaft, um einfachen Zugang zu der Steuerkurve erhalten zu können und diese einstellen zu können. Ebenso wäre es beispielsweise bei einem Wechsel der zu behandelnden Behältnisse 10 denkbar, eine Steuerkurveneinheit aus dem Gehäuse zu entfernen und durch eine andere Steuerkurveneinheit zu ersetzen. Da keine anderen Bauteile der Behältnisaußenflächenbehandlungseinrichtung 100 dauerhaft in dem Bereich der Steuerkurveneinheit unterhalb des Trägers angeordnet sind, kann eine solche Steuerkurveneinheit ohne sie zerlegen zu müssen herausgenommen werden. Dies ermöglicht einen schnellen Wechsel, insbesondere, wenn wiederholt zwischen mehreren Behältnissen gewechselt werden soll. Die jeweiligen Steuerkurveneinheiten könnten dann unmittelbar eingesetzt werden. Eine erneute Einstellung auf das zu behandelnde Behältnis kann - bis auf eine eventuell notwendige Feinjustierung - entfallen.

Neben den Führungsrollen 108, die wie oben beschrieben unter Nutzung eine Steuerkurve eine Verschiebung der Halteeinrichtung 140 und damit auch der davon abschnittsweise geführten Behältnisse 10 in der vertikalen Richtung, also entlang der Höhenrichtung H, ermöglicht, umfasst die Behältnisaußenflächenbehandlungseinrichtung 100 auch einen Verschiebemechanismus 110. Mittels diesem ist es möglich, eine einzelne Halteeinrichtung 140 senkrecht zu der Höhenrichtung H zu verschieben. Diese Verschiebung kann beispielsweise eine Verschiebung in Umfangsrichtung oder eine Verschiebung in radialer Richtung bezüglich des Trägers 120 umfassen. Natürlich sind auch Verschiebungen denkbar und in einigen Anwendungen vorteilhaft, die sowohl eine Komponente in Umfangsrichtung als auch eine in radialer Richtung aufweisen. Zusätzlich dazu und unabhängig davon ist selbstverständlich auch die wie oben beschrieben Verschiebung entlang der horizontalen Richtung H möglich, beispielsweise durch die Wechselwirkung der Führungsrollen 108 mit der Steuerkurve. Insbesondere sind Kombinationen von Verschiebungen in mindestens zwei Richtungen im Bereich der (nicht gezeigten) Behältnisaußenflächenbeaufschlagungseinrichtung 150 vorteilhaft, um ein Behältnis 10 trotz der Krümmung der Umlaufbahn des rotierenden Trägers 120 möglichst linear in einem definierten Abstand an der Behältnisaußenflächenbeaufschlagungseinrichtung 150 entlang führen zu können. Gegebenenfalls ist bei dieser Führung eine weitere Bewegung des Behältnisses möglich, beispielsweise eine Rotation um eine horizontale (Kippen) oder vertikale (Drehen) Achse um dauerhafte Abschattungen bestimmter Flächen durch andere Teile des Behältnisses vermeiden zu können.

Bei der Behältnisinnenflächenbehandlungseinrichtung 200 handelt es sich ebenfalls um eine Transporteinrichtung 300, da das Behältnis während der Behandlung durch die Behältnisinnenflächenbehandlungseinrichtung 200 ebenfalls entlang des Transportpfads transportiert wird. Dazu wird zunächst ein Behältnis 10 von der stromaufwärts befindlichen Transporteinrichtung 300, hier der Behältnisaußenflächenbehandlungseinrichtung 100 übernommen. Dies erfolgt auf einem ersten Höhenniveau. Dieses niedrige Höhenniveau ist erforderlich, da eine innengreifende Halteeinrichtung 140 der Behältnisaußenflächenbehandlungseinrichtung 100 zwischen dem Strahlfinger 250 der Behältnisinnenflächenbehandlungseinrichtung 200 und dem Behältnis angeordnet ist.

Im gezeigten Beispiel wird das zu übernehmende Behältnis 10 durch die Behältnisinnenflächenbehandlungseinrichtung 200, genauer durch eine deren Halteeinrichtungen 240 aufgenommen und anschließend auf ein niedrigeres Höhenniveau abgesenkt. Dies ist vorteilhaft, um das Behältnis von der innengreifenden Halteeinrichtung 140 der Behältnisaußenflächenbehandlungseinrichtung 100 abzuziehen. Alternativ oder ergänzend dazu wäre auch das Anheben der Halteeinrichtung 140 der Behältnisaußenflächenbehandlungseinrichtung 100 denkbar, wobei der dazu zur Verfügung stehende Raum durch den Strahlfinger 250 limitiert ist.

Nachdem das Behältnis 10 durch die Halteeinrichtungen 240 der Behältnisinnenflächenbehandlungseinrichtung 200 abgezogen wurde, befindet sich auf einem niedrigeren Höhenniveau, wie es für das Behältnis 10.1 dargestellt ist. Anschließend wird das Behältnis 10 wieder angehoben, dabei bewegt es sich relativ zu einer Behältnisinnenflächenbeaufschlagungseinrichtung 250, bevorzugt einem Strahlfinger 250. Die Relativbewegung erfolgt derart, dass der Strahlfinger 250 zumindest abschnittsweise in das Innere des Behältnisses 10 hineinragt. Dadurch ist eine Beaufschlagung der inneren Oberflächen des Behältnisses 10 aus kurzer Distanz möglich, was besonders effizient ist.

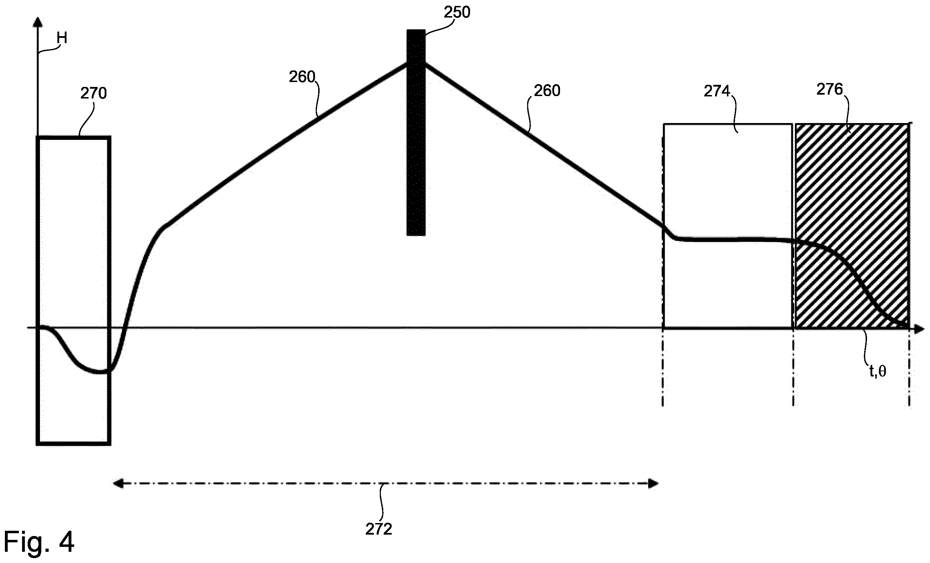

Vorzugsweise erfolgt die Relativbewegung zwischen Behältnis 10 und Strahlfinger 250 durch eine Bewegung des Behältnisses auf den Strahlfinger 250 zu. Dabei kann der Strahlfinger 250 beziehungsweise die Behältnisinnenflächenbeaufschlagungseinrichtung 250 bezüglich des Trägers 220 in der Position unverändert verbleiben. Dies hat den Vorteil, dass lediglich das vergleichsweise leichte und kostengünstige Behältnis bewegt werden muss und nicht der empfindliche Strahlfinger 250 mit der Strahlungserzeugungseinrichtung 252 und eventuell vorhandenen Anschlüssen und/oder Zuleitungen. Diese Strahlungserzeugungseinrichtung 252 und eventuell vorhandene Anschlüsse und/oder Zuleitungen befinden sich vorzugsweise - wie im gezeigten Beispiel dargestellt - außerhalb des lediglich abschnittsweise dargestellten Gehäuses 400, um die Wartung auch bei geschlossenem Gehäuse zu ermöglichen. Die Bewegung eines Behältnisses 10 beziehungsweise einer Halteeinrichtung 240 während einer Umdrehung des Trägers 220 der Behältnisinnenflächenbehandlungseinrichtung 200 ist im Detail in Zusammenhang mit Fig. 4 beschrieben.

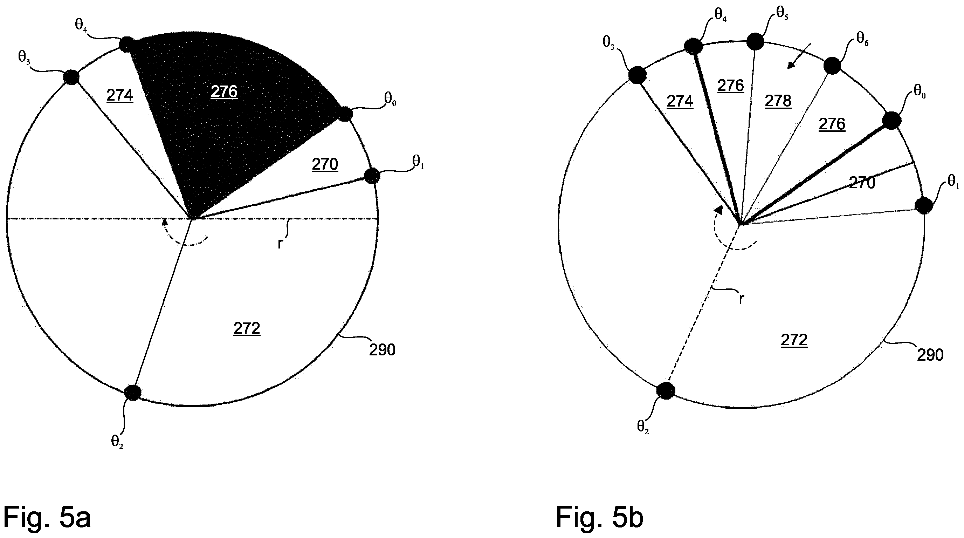

Die Abgabe des behandelten Behältnisses 10.2 an eine nicht dargestellte, entlang des Transportpfads folgende Transporteinrichtung erfolgt vorzugsweise unmittelbar nachdem der Strahlfinger 250 das Behältnis 10.2 vollständig verlassen hat. Eine Absenkung des Behältnisses 10.2 auf das erste Höhenniveau, auf dem die Aufnahme des Behältnisses 10.1 stattgefunden hat, ist nicht notwendig, sondern würde den für die Behältnisbehandlung zur Verfügung stehenden Sektor reduzieren, wie im Zusammenhang mit den Figuren 4 und 5 beschrieben ist.

Fig. 4 zeigt eine schematische Darstellung eines beispielhaften Profils 280 der Verschiebung einer Halteeinrichtung 240 und/oder eines Behältnisses 10 entlang der Höhenrichtung H auf einer Behältnisinnenflächenbehandlungseinrichtung 200. Im Bereich 270 wird das Behältnis 10 durch die Halteeinrichtung 240 der Behältnisinnenflächenbehandlungseinrichtung 200 aufgenommen. Wie oben beschrieben wird es zunächst abgesenkt, um es von der innengreifenden Halteeinrichtung 140 der Behältnisaußenflächenbehandlungseinrichtung 100 abzuziehen. Kurzzeitig befindet sich das Behältnis 10 somit auf einem Höhenniveau welches unterhalb des Höhenniveaus liegt, in welchem der erste Kontakt mit der Halteeinrichtung 240 erfolgt ist. Die Abszissenachse ist sowohl mit t als auch mit θ gekennzeichnet. Dadurch soll symbolisiert werden, dass das Profil sowohl zeitabhängig als auch winkelabhängig sein kann. Da die Rotation des Trägers ohnehin eine bestimmte Zeit in Anspruch nimmt, wird das Profil bei Verwendung eines rotierenden Trägers 220 üblicherweise zeitabhängig und winkelabhängig sein.

Sobald das Behältnis 10 von der Halteeinrichtung 240 übernommen wurde und sich beides durch die Rotation des rotierenden Trägers 220 von der Behältnisaußenflächenbehandlungseinrichtung 100 ausreichend entfernt hat, kann der eigentliche Behandlungsprozess 272 beginnen. Dazu wird das Behältnis angehoben, also entlang der Höhenrichtung H verschoben. Am höchsten Punkt befindet sich die Behältnisinnenflächenbeaufschlagungseinrichtung 250, die schematisch als Strahlfinger 250 dargestellt ist, abschnittsweise im Inneren des Behältnisses. Dort und auf dem Weg dorthin und davon weg kann sie Behältnisinnenflächen mit einem Medium beziehungsweise Strahlung beaufschlagen. Sobald das Behältnis soweit abgesenkt ist, dass die Behältnisinnenflächenbeaufschlagungseinrichtung 250 vollständig außerhalb dieses Behältnisses angeordnet ist, wird das Behältnis im Bereich 274 an eine stromabwärts angeordnete Transporteinrichtung abgegeben. Diese Abgabe erfolgt auf einem anderen, insbesondere höheren Höhenniveau als die Aufnahme des Behältnisses im Bereich 270.

Die notwendige Verschiebung der dann nicht von einem Behältnis besetzten Halteeinrichtung 240 auf das erste Höhenniveau, nämlich das Höhenniveau für die Behältnisaufnahme, erfolgt in einem Zeitabschnitt, beziehungsweise Winkelbereich 276, in dem keine Behandlung des Behältnisses 10 stattfinden kann. Somit ist dieses Zeitintervall 276, das üblicherweise als "Totzeit" bezeichnet wird, nicht mehr ungenutzt, sondern kann aktiv zur Vorbereitung auf den nächsten Behandlungsschritt beziehungsweise für die nächste Behältnisaufnahme 270 beitragen. Wie insbesondere durch die Figuren 5a und 5b veranschaulicht ist, kann so die für den eigentlichen Behandlungsprozess verfügbare Zeit 272 beziehungsweise der Behältnisbehandlungssektor 272 vergrößert werden. Bei vorgegebener Behandlungsdauer, beziehungsweise der dafür zur genutzten Kreisbogenlänge kann der Umfang des durch die Halteelemente überstrichenen Kreises verkleinert werden. Dies ermöglicht einen geringeren Radius und somit kleiner Dimensionierung des rotierenden Trägers 220.

Fig. 5a zeigt eine beispielhafte Zuordnung verschiedener Sektoren einer Kreisbahn zu verschiedenen Prozessschritten bei einer Behandlungseinrichtung gemäß dem Stand der Technik. Im Sektor 270 erfolgt die Übernahme eines Behältnisses durch eine Halteeinrichtung. Der zu diesem Sektor gehörende Kreisbogen muss zwangsläufig eine Mindestlänge aufweisen, da das Einführen des Strahlfingers erst dann erfolgen kann, wenn die Halteeinrichtung 140 der stromaufwärts angeordneten Transporteinrichtung vollständig aus dem Bewegungsbereich der Halteeinrichtung 240 und des von diesem aufgenommenen Behältnisses 10 entfernt sein muss. Erst nach Verlassen dieses Sektors kann daher an dem mit θ₁ gekennzeichneten Punkt mit dem Einführen des Strahlfingers in das Behältnis begonnen werden.

Der sich daran anschließende Sektor 272 steht der Behältnisbehandlung zur Verfügung. Während der Behandlung wird das Behältnis entlang des Kreisbogens 290 geführt. Der in diesem Sektor liegende Punkt θ₂ kennzeichnet den Wendepunkt, ab welchem der Strahlfinger aus dem Behältnis herausgeführt wird. Der Sektor 272 erstreckt sich bis zum Punkt θ₃ bei welchem der Strahlfinger vollständig aus dem Behältnis entfernt ist. Sobald dies gewährleistet ist, kann im Sektor 274 die Abgabe des behandelten Behältnisses 10 an eine stromabwärts angeordnete Transporteinrichtung 300 erfolgen.

Da die stromaufwärts angeordnete Transporteinrichtung und auch die stromabwärts angeordnete Transporteinrichtung 300 jeweils einen gewissen Raum einnehmen, ist der Sektor des Totvolumens 276 nicht beliebig verkleinerbar. Je nach Dimension der benachbarten Transporteinrichtungen überspannt dieser Sektor üblicherweise einen Bereich von etwa 75° - 90°. Der Punkt θ₄ liegt somit üblicherweise bezüglich des Punkts der des Beginns der Aufnahme des Behältnisses θ₀ bei etwa 270° - 285°. Dieser Bereich kann nicht für eine Behältnisbehandlung genutzt werden.

Fig. 5b zeigt eine beispielhafte Zuordnung verschiedener Sektoren einer Kreisbahn zu verschiedenen Prozessschritten bei einer Behandlungseinrichtung gemäß einer bevorzugten Ausführungsform der vorliegenden Erfindung. Sektoren und Prozessschritte, in denen zu den in Fig. 5a dargestellten analog sind, sind mit gleichen Bezugszeichen gekennzeichnet. Dementsprechend erfolgt auch bei einer erfindungsgemäßen Behältnisinnenflächenbehandlungseinrichtung 200 die Übernahme eines Behältnisses 10 im Sektor 270, beginnend an Punkt θ₀. Da das Absenken der Halteeinrichtung 240 bevorzugt ohnehin ein diesem Sektor erfolgt, in dem ein Anheben der Halteeinrichtung 240 zur Vermeidung von Kollisionen mit der Halteeinrichtung 140 der stromaufwärts angeordneten Transporteinrichtung 100 noch nicht möglich ist, liegt der Startpunkt θ₁ der Behandlung trotz der Absenkung ähnlich wie bei dem Beispiel aus dem Stand der Technik.

Im Sektor 272 findet die Behältnisbehandlung statt. Der Punkt θ₂ kennzeichnet auch hier den Wendepunkt, ab welchem der Strahlfinger aus dem Behältnis herausgeführt wird. Nur der direkte Vergleich zeigt, dass der Sektor 272 weiter ist als der zur Behandlung zur Verfügung stehende gemäß Fig. 5a. Die größere Weite des Sektors 272 kann erreicht werden, da die Übergabe an eine folgende Transporteinrichtung 300 in Sektor 274 unmittelbar dann erfolgt, wenn die Halteeinrichtung so weit abgesenkt wurde, dass kein Abschnitt des Strahlfingers mehr im Inneren des Behältnisses 10 ist. Die Rückführung des Halteelements auf das Höhenniveau, welches am Punkt θ₀ vorliegen muss, erfolgt erst in einem Sektor 278, welcher ein Ausschnitt des Sektors 276 ist, der die Totzeit kennzeichnet. Im gezeigten Beispiel erstreckt sich der Sektor 278 über einen Winkel von 25°, nämlich von θ₅ = 310° bis θ₆ = 335° (jeweils bezüglich θ₀).

Da bei einer (beispielsweise durch die Rotationsgeschwindigkeit und die Behandlungszeit) vorgegebenen Länge L des Kreisbogens 290 kann der Radius r gemäß der Formel L = 2π •r•θ/360 bei steigendem Winkel θ reduziert werden. Gemäß der oben genannten Formel ermöglicht eine Vergrößerung des Winkels um 10° bei gleichbleibender Kreisbogenlänge L eine Reduzierung des Radius r um nahezu 20%. Da der Radius sogar mit r² in die für den Träger 220 notwendige Kreisfläche einfließt, ergibt sich bereits bei geringen Vergrößerungen des zur Behandlung nutzbaren Sektors 272 beziehungsweise des Winkels zwischen θ₁ und θ₃ eine große Flächenersparnis und auch Material- und Gewichtsersparnis für die Behältnisinnenflächenbehandlungseinrichtung 200.

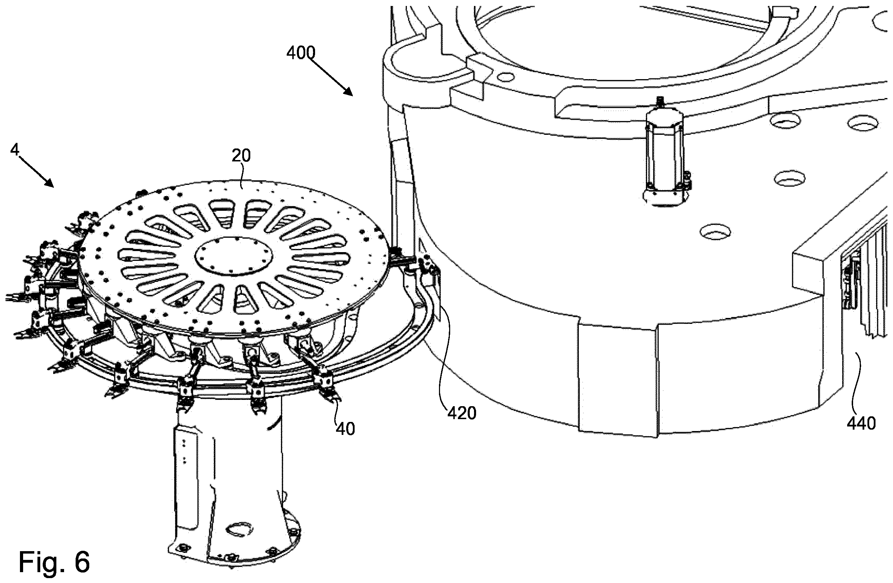

Figur 6zeigt eine schematische Darstellung einer Übergabe von der ersten Transporteinrichtung 4 an die zweite Transporteinrichtung. Die zweite Transporteinrichtung ist dabei in dieser Darstellung innerhalb des Gehäuses 400 angeordnet und daher nicht erkennbar. Bei der ersten Transporteinrichtung 4 handelt es sich dabei um einen ersten Teilungsverzugsstern, welcher dazu ausgebildet ist, eine Teilung zwischen benachbart transportierten Kunststoffbehältnissen zu verändern. Dies wird durch die schwenkbare Lagerung der Halteelemente 40 ermöglicht.

Die erste Transporteinrichtung 4 weist dabei einen rotierbaren Träger auf und übergibt die Kunststoffbehältnisse über ein (Übergabe-) Fenster 420 an die innerhalb des Gehäuses 400 angeordnete zweite Transporteinrichtung. Das Bezugszeichen 152 kennzeichnet den Behältnisaußenflächenbeaufschlagungsbereich, in welchem die Außenbehandlung der Kunststoffbehältnisse durchgeführt wird.

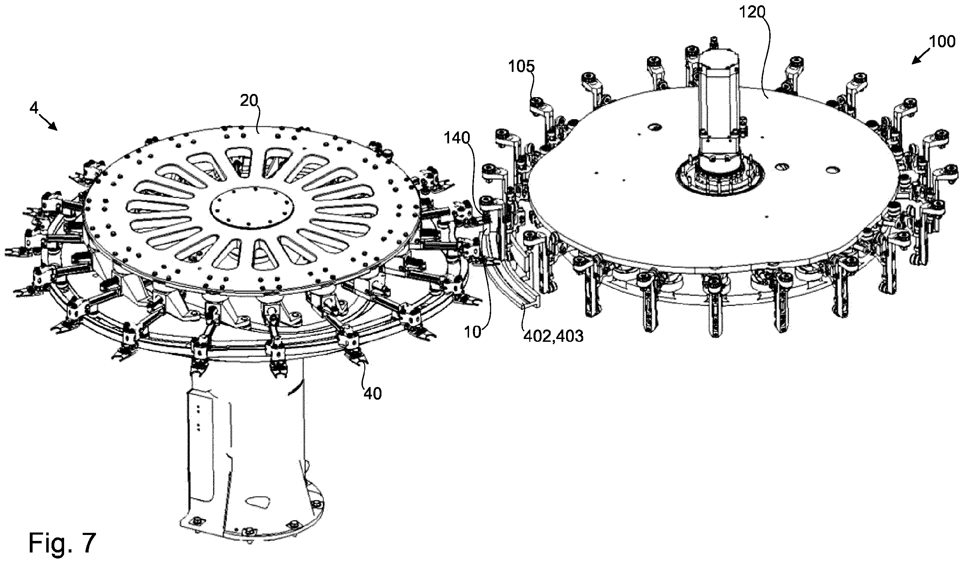

Figur 7 zeigt eine weitere schematische Darstellung einer Übergabe von der ersten Transporteinrichtung 4 an die zweite Transporteinrichtung 100. Zur genaueren Darstellung dieser Übergabe ist diese in der Figur 7 ohne das Gehäuse dargestellt. Dargestellt ist dabei insbesondere der Augenblick der Übergabe, bei welcher das Kunststoffbehältnis 10 sowohl von dem Halteelement 40 der ersten Transporteinrichtung 4 als auch von dem Halteelement 140 der zweiten Transporteinrichtung 100 gehalten wird. Bei der zweiten Transporteinrichtung 100 handelt es sich dabei um einen zweiten Teilungsverzugsstern, welcher ebenfalls dazu ausgebildet ist, eine Teilung zwischen benachbart transportierten Kunststoffbehältnissen zu verändern.

Die zweite Transporteinrichtung 100 weist dabei zumindest im Bereich der eigentlichen Übergabe eine Hubkurve 402, 403 auf, entlang derer eine Führungsrolle 406 geführt wird, so dass eine sichere Übergabe ermöglich wird. Das Bezugszeichen 105 kennzeichnet eine Hub- und Dreheinrichtung, welche während der Übergabe eine Hubbewegung des Halteelements 140 auf das Kunststoffbehältnis 10 zu ermöglicht, wodurch das Halteelement 140 in das Kunststoffbehältnis 10 eingeführt wird, um dieses zu halten. Das Bezugszeichen 120 kennzeichnet den rotierbaren Träger der zweiten Transporteinrichtung 100.

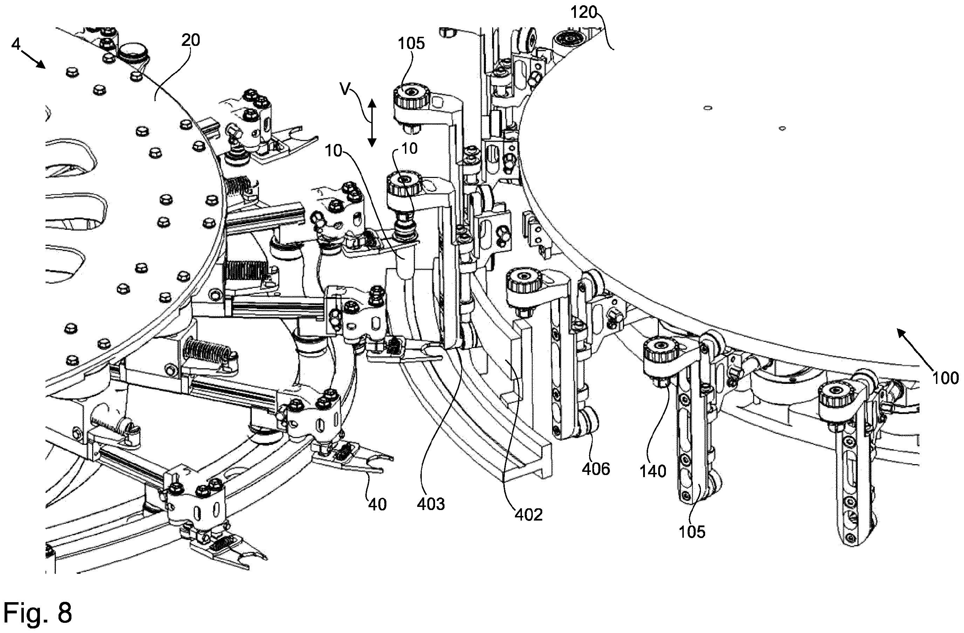

Figur 8 zeigt eine Detaildarstellung einer Übergabe von der ersten Transporteinrichtung 4 an die zweite Transporteinrichtung 100. Wie auch in der Figur 7 wird das Kunststoffbehältnis 10 dabei in dieser Darstellung sowohl von dem Halteelement 40 der ersten Transporteinrichtung 4 als auch von dem Halteelement 140 der zweiten Transporteinrichtung 100 gehalten.

Gut erkennbar sind dabei in dieser Darstellung die erste Hubkurve 402 und die zweite Hubkurve 403 zwischen denen eine Führungsrolle 406 der Hub- und Dreheinrichtung 105 geführt wird. Die Hub- und Dreheinrichtung 105 ist dabei dazu geeignet und bestimmt, das Halteelement 140 entlang der vertikalen Richtung v in Richtung des Behältnisses zu bewegen.

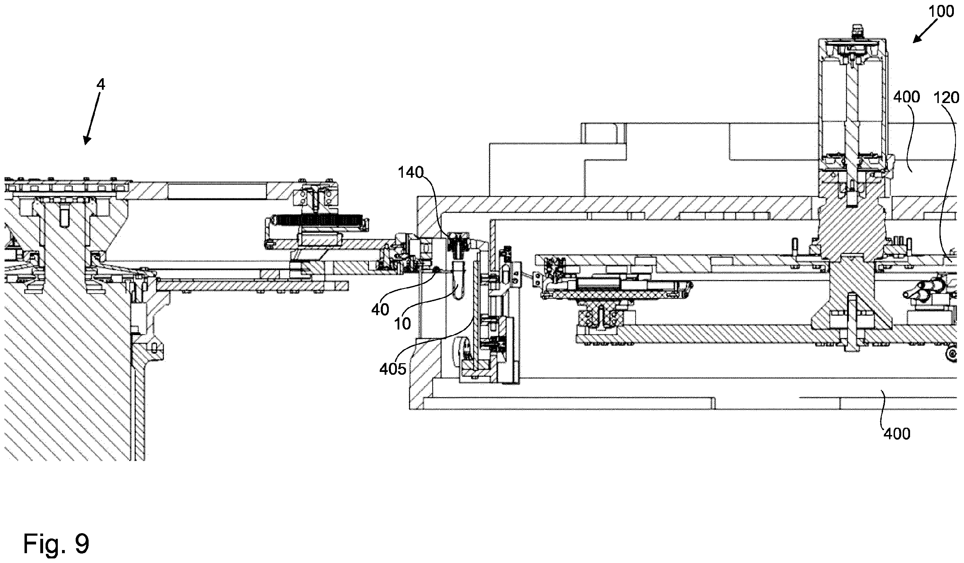

Figur 9 eine Schnittdarstellung einer Übergabe von der ersten Transporteinrichtung 4 an die zweite Transporteinrichtung 100, wobei auch hier wieder der in den Figuren 7 und 8 gezeigte Zeitpunkt der Übergabe dargestellt ist. Das Bezugszeichen 405 kennzeichnet eine Abschirmungseinrichtung, welche die Umgebung gegenüber dem inneren des Gehäuses 400 abschirmt, so dass beispielsweise bei der Sterilisation der Behältnisse entstehende Strahlung und insbesondere Röntgenstrahlung nicht an die Umgebung gelangt.

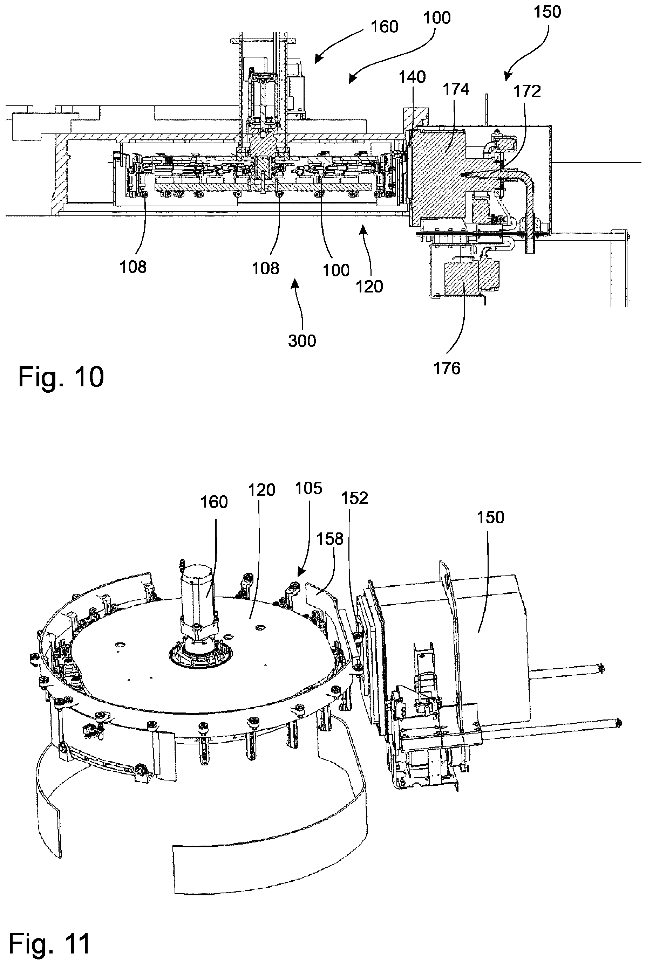

Fig. 10 zeigt eine weitere Ausgestaltung der erfindungsgemäßen Vorrichtung. Dabei ist wiederum die (innerhalb des Gehäuses) angeordnete Transporteinrichtung mit dem drehbaren Träger 120 dargestellt. Zur Vermeidung von Wiederholungen wird auf die obige Beschreibung zu Fig. 3 verwiesen.

Das Bezugszeichen 150 kennzeichnet die Behältnisaußenflächenbeaufschlagungseinrichtung in ihrer Gesamtheit. Diese weist eine Elektronenerzeugungseinrichtung 172 auf, die in einer Vakuumkammer 174 endet. Das Bezugszeichen 178 kennzeichnet eine Vakuumpumpe, insbesondere zur Erreichung eines Grobvakuums. Das Bezugszeichen 140 kennzeichnet eine Halteeinrichtung wie einen Haltedorn zum Halten der Kunststoffvorformlinge.

Fig. 11 zeigt eine weitere Darstellung einer bevorzugten Ausführungsform der Erfindung. Hierbei ist die Antriebseinrichtung 160 vorgesehen, sowie eine Wandung 158. Das Bezugszeichen 152 kennzeichnet den Behältninsaußenflächenbeaufschlagungsbereich, in dem hier eine Außensterilisation des Behältnisses vorgenommen wird. Das Bezugszeichen 105 kennzeichnet wieder die Hub- und Dreheinrichtung. Die Bezugszeichen 182 und 183 beziehen sich auf die obere und untere Abschirmung innerhalb des (nicht gezeigten Gehäuses). Wie oben erwähnt ist dabei die obere Abschirmung 183 stationär und die untere Abschirmung 182 absenkbar.

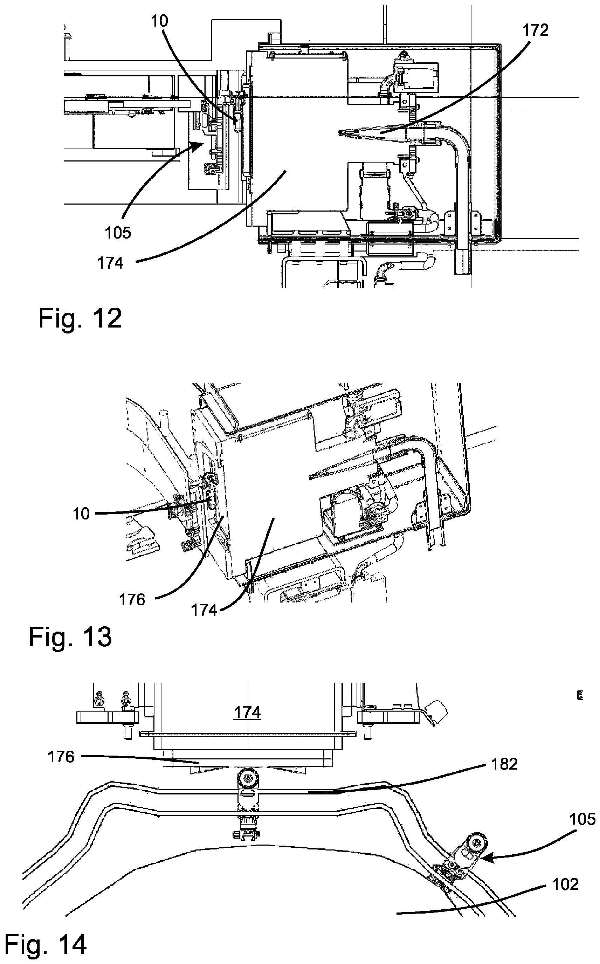

Fig. 12 zeigt eine weitere Darstellung der in Fig. 11 gezeigten Ausführungsform. Hier ist auch ein Kunststoffvorformling 10 dargestellt, der von der Hub- und Dreheinrichtung gehalten und an seiner Außenoberfläche sterilisiert wird. Man erkennt, dass der Kunststoffvorformling sehr nahe an der Außensterilisationseinrichtung vorbeigeführt wird.

Fig. 13 zeigt eine weitere Schnittdarstellung der in Fig. 12 gezeigten Ausführungsform. Dabei kennzeichnet das Bezugszeichen 176 die Strahlerfläche des Flächenstrahlers 150 (d.h. der Außenflächensterilisationseinrichtung). Man erkennt, dass die Gesamte Außenflächensterilisationseinrichtung innerhalb eines Gehäuses angeordnet ist, wobei es sich bevorzugt um ein strahlungsabschirmendes Gehäuse handelt.

Fig. 14 veranschaulicht die Außensterilisation der Kunststoffvorformling. Dabei ist ein Bereich einer Abschirmung 182 vorgesehen, der bewirkt, dass die Kunststoffvorvorformlinge im Bereich der Strahlerfläche geradlinig entlang der Linie G transportiert werden, um so die Sterilisationswirkung zu verbessern.

Bevorzugt ist dieser Teil der Abschirmung, der nicht dargestellten Laufbahn des Kunststoffvorformlingwegs nachempfunden. Durch die beidseitigen Verengungen, die zum Flächenstrahler gerichtet sind, ist hier eine Verbesserung der Strahlenabschirmung zu erreichen.

Daneben wäre es auch möglich, dass in diesem Bereich die Kunststoffvorformlinge langsamer bewegt werden als in anderen Bereichen des Transportpfads. Gleichzeitig werden in diesem Abschnitt die Kunststoffvorformlinge bezüglich ihrer Längsachsen gedreht.

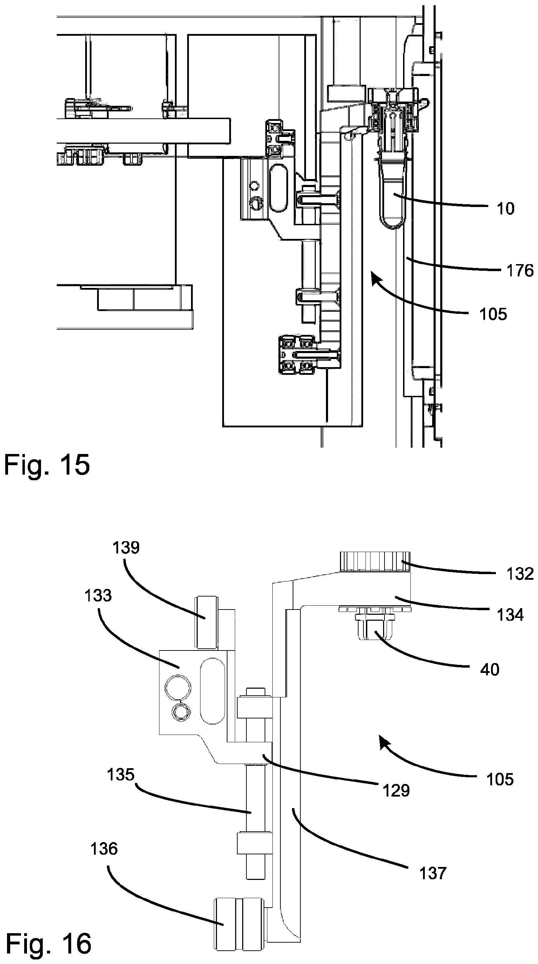

Fig. 15 zeigt eine Detaildarstellung der in Fig. 14 gezeigten Darstellung. Hier erkennt man auch wieder den Kunststoffvorformling, der, gehalten von der Hub- und Dreheinrichtung an der Strahlerfläche vorbeibewegt wird. Daneben ist der Kunststoffvorformling auch bezüglich seiner Längsrichtung L drehbar gelagert bzw. wird bezüglich dieser Längsrichtung L gedreht.

Zu erkennen ist auch die Anordnung der in Figur 11 gezeigten oberen und der unteren Strahlenabschirmung, welche zueinander beabstandet sind, damit die Hub- und Dreheinrichtung mit dem Träger der Dreheinheit hindurchfassen kann.

Fig. 16 zeigt eine detailliertere Darstellung der Hub- und Dreheinrichtung 105. Diese weist einen Rotor 132 auf, der drehfest mit dem Haltedorn 30 gekoppelt ist und drehbar an dem Arm bzw. Ausleger 134 gelagert ist.

Das Bezugszeichen 137 kennzeichnet einen Träger, an dem der Ausleger 134 angeordnet ist. Dieser Träger 135 ist linearbeweglich gegenüber eine Führungseinrichtung 135 gelagert und an einer Halterung 133 befestigt. Das Bezugszeichen 136 kennzeichnet eine Kurvenrolle, welche gemeinsam mit einer (nicht gezeigten) Führungskurve die Linearbewegung des Haltedorns auslösen kann.

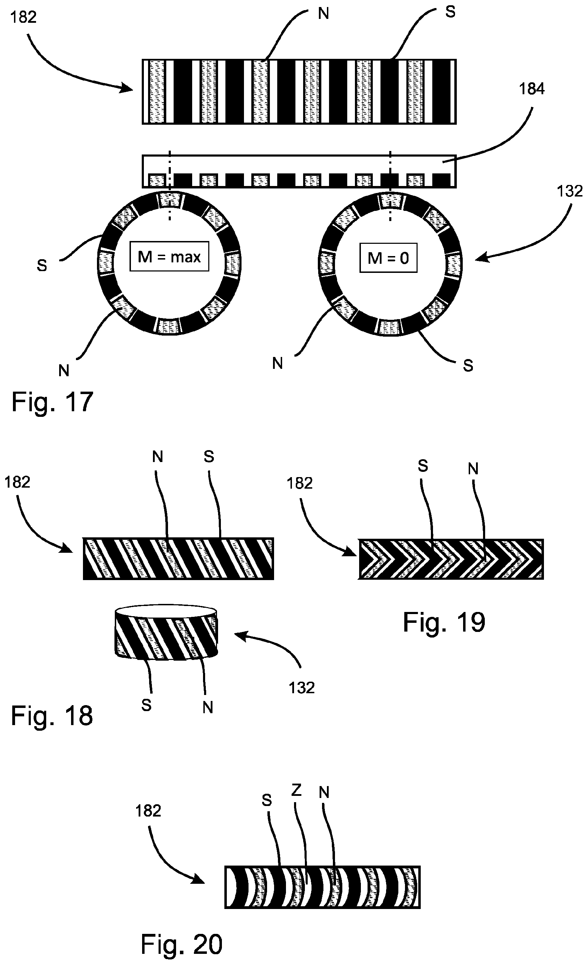

Fig. 17 veranschaulicht die Erzeugung der Drehbewegung des Rotors 132 und damit des daran gehaltenen Kunststoffvorformlings. Dabei ist neben dem Rotor 132 auch ein Stator 180 vorgesehen. Dieser Stator kann dabei an einer Innenwandung des Gehäuses der Vorrichtung angeordnet sein. Der Stator weist hier eine Vielzahl von Magneten, auf, die durch ihre magnetischen Nordpole NP und ihre magnetischen Südpole SP aufgebaut sind.

Das Bezugszeichen 184 zeigt einen Statorträger, an dem die Magnete angeordnet sind. In Fig. 17 wird weiterhin eine Problematik bei der Übertragung der Drehmomente M gezeigt. Bei der im linken Teil der Figur gezeigten Situation ist die Übertragung des Drehmoments auf den Rotor maximal und bei der im rechten Teil der Figur gezeigten Situation 0. Aus diesem Grund kommt es zu einer ungleichmäßigen Übertragung von Drehmomenten und damit, insbesondere bei hohen Transportgeschwindigkeiten dazu, dass der Kunststoffvorformling keine kontinuierliche Drehung mehr ausführt.

Die Fig. 18 - 20 zeigen eine verbesserte Ausgestaltung des Stators 180 sowie des Rotors 132. Man erkennt hier, dass sich die Magnete NP und SP nicht wie in Fig. 17 parallel zur Drehachse erstrecken sondern abschnittsweise schräg. Bei der in Fig. 18 dargestellten Ausgestaltung erstrecken sich die Magnete geradlinig, jedoch schräg gegenüber der Drehachse bzw. der Längsrichtung L des Kunststoffvorformlings, bei der in Fig. 19 gezeigten Darstellung nehmen sie einen insgesamt pfeilartigen oder gezackten Verlauf an und bei der in Fig. 20 gezeigten Darstellung einer bogenförmig gekrümmten Verlauf.

Auf diese Weise kommt es zu insgesamt schräg verzahnten Magnetanordnung, welche einen glättenden Verlauf auf die Übertragung des Drehmoments bewirken (wenngleich ggfs das maximal übertragene Drehmoment geringer ist).

Das Bezugszeichen Z kennzeichnet jeweils Zwischenräume zwischen den einzelnen Magneten SP und NP.

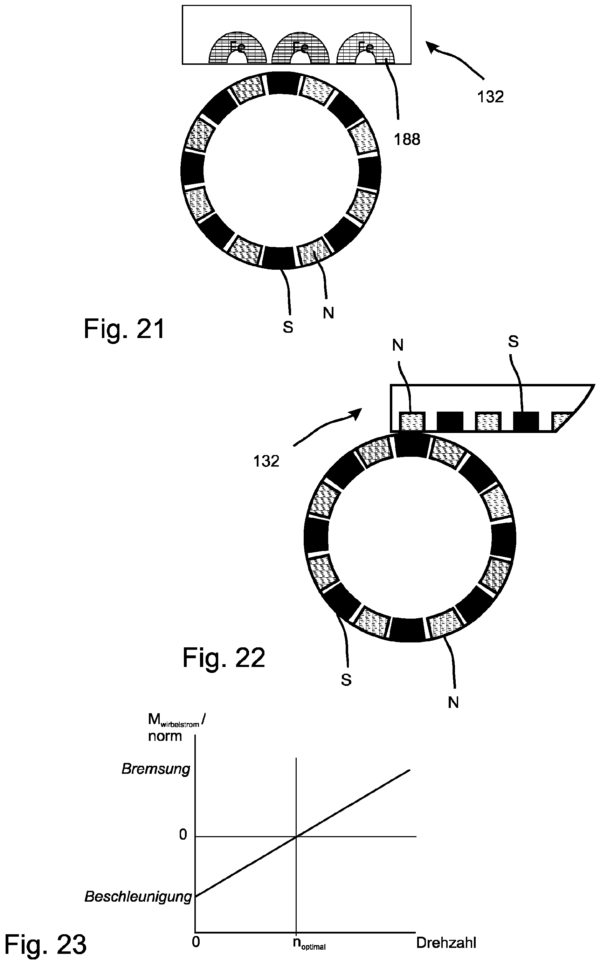

Bei der in Fig. 21 gezeigten Ausführungsform sind entweder an dem Stator 180 oder an dem Rotor 132 (hier an dem Stator 180) keine Magnete vorgesehen sondern ein Material mit einer hohen magnetischen Permeabilität, wie etwa Eisen (Fe). Durch eine geeignet Führung der magnetischen Flüsse können hier die Drehmomentschwankungen in günstiger Weise beeinflusst werden. Dieses Prinzip wird auch bei Reluktanzmotoren genutzt.

Bevorzugt wird das Material des Magnetträgers, wie oben ausgeführt so gewählt, dass es einen möglichst geringen spezifischen elektrischen Widerstand aufweist.

Fig. 22 zeigt eine weitere Veranschaulichung dieses Sachverhalts. Bei dieser Ausgestaltung sind der Träger 184 des Stators und der Träger 131 des Rotors jeweils aus diesem Material mit geringem spezifischen Widerstand ausgestattet.

Fig. 23 veranschaulicht den Effekt dieser Ausgestaltung. Wenn immer die Tangentialgeschwindigkeit ungleich der idealen Abrollgeschwindigkeit an der Leiste ist, induzieren die gegenüberliegenden Permanentmagnete eine Spannung in des leitfähige Trägermaterial und verursachen Wirbelströme. Diese bremsen oder Beschleunigen die Rolle und drängen sie in Richtung der Idealgeschwindigkeit.

Grundsätzlich entspricht die Idee dem Prinzip der Wirbelstrombremse, d. h. hier wird der Umfang der Rolle "gebremst" und somit die Rolle selbst in Drehung versetzt.
Aus Gründen des Korrosionsschutzes muss die Oberfläche ggf. beschichtet werden.

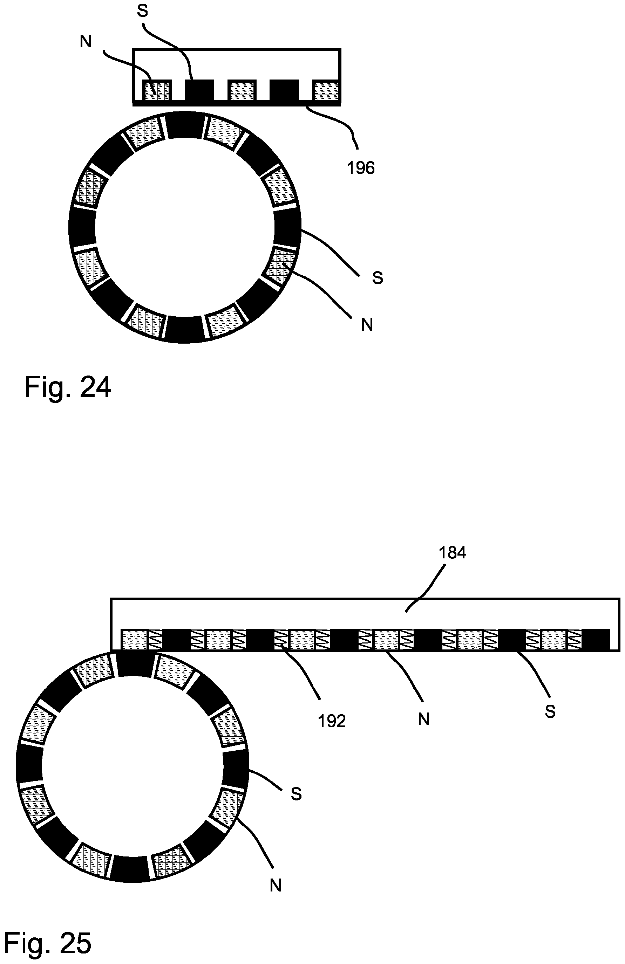

Fig. 24 veranschaulicht eine Ausgestaltung, bei der die Magnete mit einer Abdeckung 196, beispielsweise einem plattenartigen Körper aus Aluminium abgedeckt sind.

Das Bezugszeichen 190 kennzeichnet grob schematisch eine Überwachungseinrichtung, welche die Übertragung der Drehbewegung auf den Rotor 132 überwacht. Dies kann beispielsweise eine Kamera sein, welche auf den Rotor ausgerichtet ist. Daneben könnten auch andere berührungslose Sensoren zur Überwachung der Drehbewegung eingesetzt werden. Ein Problem hierbei ist, dass sich die Rotoren selbst entlang des Transportpfads der Kunststoffvorformlinge bewegen.

Fig. 25 zeigt eine weitere vorteilhafte Ausgestaltung, welche eine Überwachung der übertragenen Drehmomente bzw. der Drehbewegung des Rotors ermöglicht. Bei dieser Ausgestaltung sind, wie oben erwähnt, zwischen den Magneten NP und SP Spulen 192 angeordnet, welche hier axial in Richtung der Rolle zeigen. Sofern sich die Rolle bzw. der Rotor 132 mit der idealen Geschwindigkeit dreht und wie gewünscht an der Leiste bzw. dem Stator "abrollt", wird nur wenig Spannung in die Spulen induziert. Rutscht die Rolle jedoch durch oder schwingt in ihrer Drehgeschwindigkeit, dann wird durch die großen Flussdichteänderung relativ viel Spannung in den Spulen induziert.

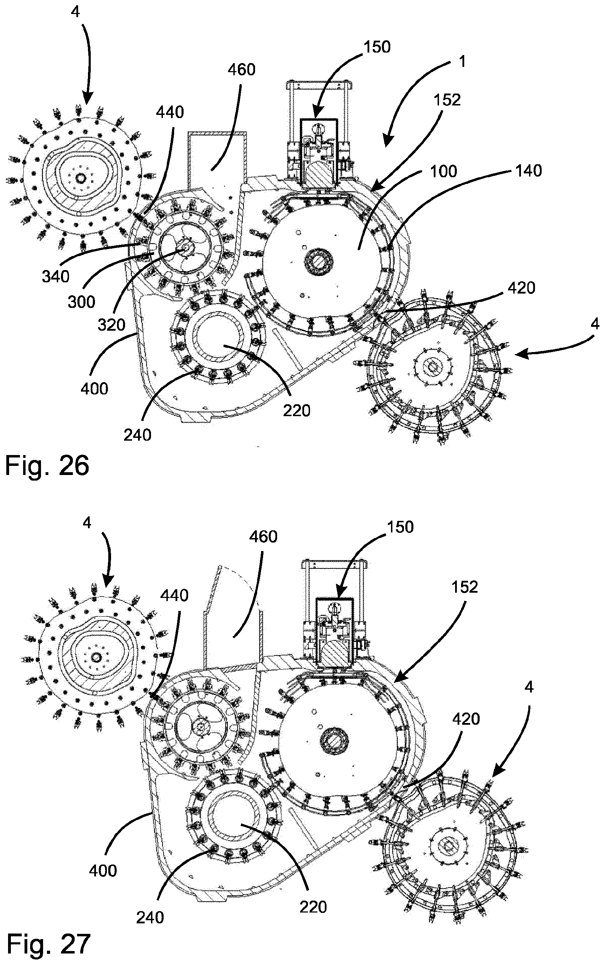

Fig. 26 zeigt eine vorteilhafte Vorrichtung 1 zum Behandeln von Behältnissen, insbesondere von Kunststoffvorformlingen 10. Dabei werden die Kunststoffvorformlinge 10 über eine Transporteinrichtung 4, welche sich außerhalb des Gehäuses 400 der Vorrichtung 1 befindet, durch einen Eingang bzw. das Fenster 420 bzw. auf eine Transporteinrichtung 100, insbesondere ein Transportstern, übergegeben.

Diese Transporteinrichtung 100 befindet sich auf einem rotierbaren Träger (bzw. weist einen n solchen auf) und befördert die Kunststoffvorformlinge zum Beaufschlagen der Außenoberflächen durch eine bzw. zu einer Behältnisbeaufschlagungseinrichtung 150. Anschließend erfolgt eine Übergabe an eine weitere Transporteinrichtung 200, insbesondere ein Transportstern, welche sich auf einem rotierbaren Träger 220 befindet oder einen solchen aufweist.

An dieser Transporteinrichtung ist eine Behältnisbeaufschlagungseinrichtung, insbesondere eine Vielzahl von Strahlfingere zum Beaufschlagen des Innenbereiches der Kunststoffvorformlinge 10 angeordnet.

Fertig beaufschlagte Kunststoffvorformlinge 10 werden über eine Transporteinrichtung 300, insbesondere ein Transportstern durch einen Ausgang 440 an eine weitere Transporteinrichtung 4, die außerhalb des Gehäuses angeordnet ist, übergeben.

Das Bezugszeichen 152 kennzeichnet einen Behältnisbeaufschlagungsbereich. Die Kunststoffvorformlinge 10 werden auf einer Transporteinrichtung 100 in diesem Bereich befördert und dort von einer Behältnisbeaufschlagungseinrichtung 150 beaufschlagt. Es werden dabei die Außenoberflächen der Kunststoffvorformlinge 10 beaufschlagt.

Die Bezugszeichen 140, 240 und 340 kennzeichnen Halteelemente, insbesondere Haltedorne zum Halten der Kunststoffvorformlinge 10. Die sind dafür eingerichtet einen Übergang zwischen den Transporteinrichtungen zu ermöglichen.

Das Bezugszeichen 460 kennzeichnet eine Schleuseeinrichtung, welche fehlerhafte Kunststoffvorformlinge 10 oder Kunststoffvorformlinge 10, die bestimmten Erfordernisse nicht erfüllen können, aus der Vorrichtung 1 über die Transporteinrichtung 300 entfernt.

Fig. 27 zeigt eine erfindungsgemäße Vorrichtung 1 mit einer Schleuseeinrichtung 460, welche sich in einem geöffneten Zustand befindet.

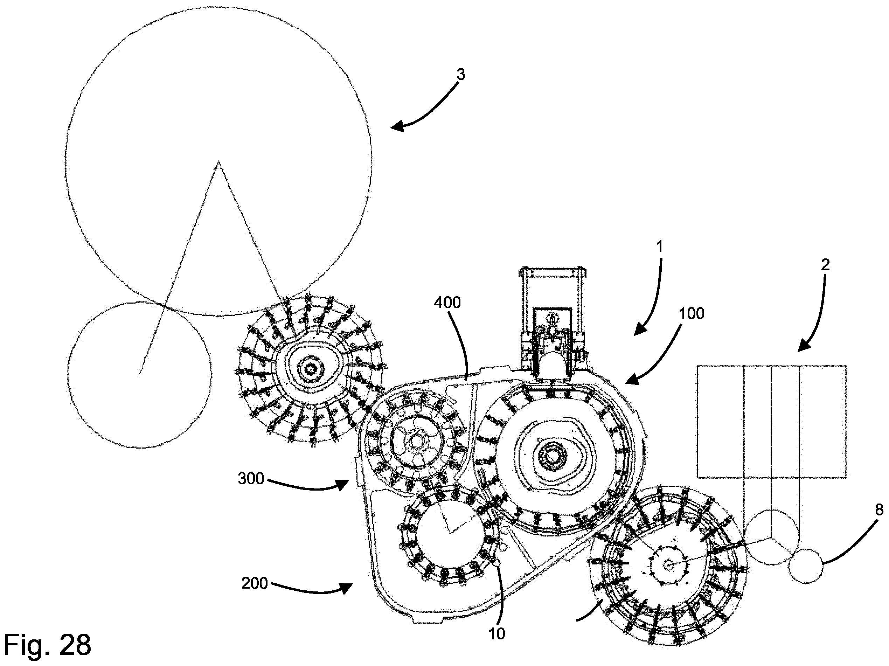

Auch Figur 28 zeigt eine schematische Aufsicht auf eine Behältnisbehandlungsvorrichtung 1 in einer beispielhaften Ausführungsform. Erkennbar sind dabei insbesondere die Transporteinrichtungen 100, 200, 300 zum Transport eines Behältnisses 10 entlang eines in dieser Darstellung nicht hervorgehobenen Transportpfads innerhalb eines Gehäuses 400. Auch bei Figur 13 handelt es sich bei der Transporteinrichtungen 100 um eine Behältnisaußenflächenbehandlungseinrichtung 100 und bei der Transporteinrichtung 200 um eine Behältnisinnenflächenbehandlungseinrichtung 200.

Ebenfalls erkennbar sind in Figur 28 die Heizvorrichtung 2 und die weitere Transporteinrichtung 4, die zwischen der Heizvorrichtung 2 und der Behältnisaußenflächenbehandlungseinrichtung 100 und außerhalb des Gehäuses 400, angeordnet ist. Unmittelbar vor der Heizvorrichtung 2 befindet sich eine Beaufschlagungseinrichtung 8, die ein Behältnis mit einem fließfähigen Medium beaufschlagen kann.

Das Bezugszeichen 3 kennzeichnet eine Umformungseinrichtung, insbesondere eine Blasmaschine zum Umformen von Kunststoffvorformlingen zu Kunststoffbehältnissen. Erkennbar ist, dass die Heizvorrichtung 2 in einer anderen Winkelposition zur Umformungseinrichtung 3 angeordnet ist, als dies beispielsweise bei Figur 1 der Fall ist. Während in Figur 1 die Heizvor-richtung 2 und die Umformungseinrichtung 3 im Wesentlichen in einem 90°-Winkel zueinander angeordnet sind, sind sie in Figur 28 in einem 0°-Winkel zueinander angeordnet. Dies ist besonders vorteilhaft, wenn in die Heizvorrichtung 2 eine Beaufschlagungseinrichtung 8 integriert ist.

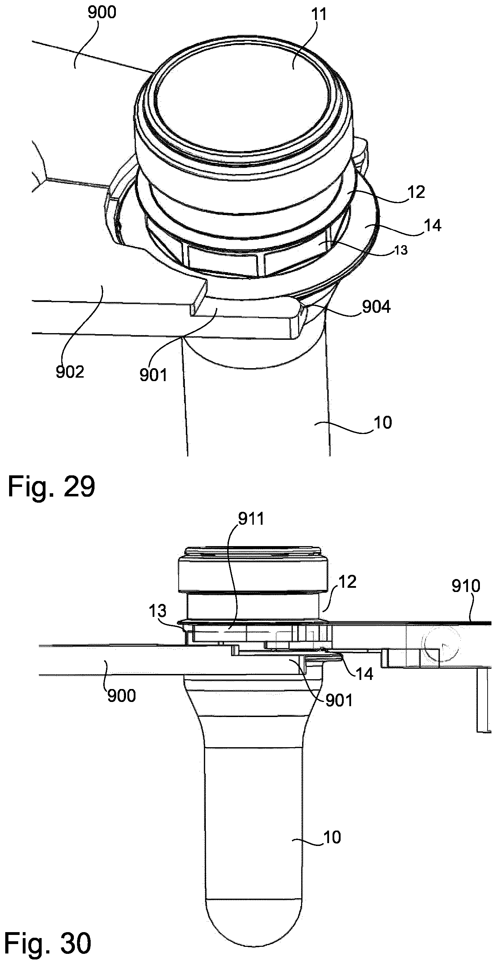

Figur 29 zeigt ein Behältnis 10, insbesondere ein Kunststoffvorformling, dessen Kopfbereich eine Mündung 11, einen Verschlussring 12, eine Mündungsnut 13 und einen Tragring 14 umfasst.

Das Behältnis 10 wird von einer ersten Halteeinrichtung 900 gehalten, welche als zweiteilige Klammer ausgestaltet ist. Die erste Halteeinrichtung 900 weist eine Haltenut 903 auf, welche die Außenfläche des Tragrings 14 kontaktiert. Die erste Halteeinrichtung 900 ist derart gestaltet, dass die Unterseite des Tragrings 14 im gesamten Bereich der ersten Halteeinrichtung 900 kontaktiert wird. Zusätzlich weist die erste Halteeinrichtung 900 einen Bereich 902 auf, welcher die Oberseite des Tragrings 14 kontaktiert.

Insgesamt wird also der Tragring 14 des Behältnisses 10 durch die erste Halteeinrichtung 900 sowohl seitlich als auch von oben und unten eingeklemmt und ist dadurch fixiert und gegen ein ungewolltes seitliches Verkippen gesichert.

Deutlich zu erkennen ist auch, dass das dem Behältnis 10 zugewandte Ende der ersten Halteeinrichtung 900 bzw. die beiden Enden der einzelnen Klammern ein verjüngten Bereich 901 aufweisen. Eine genauere Beschreibung des verjüngten Bereichs erfolgt im Zusammenhang mit der Beschreibung der Figur 2.

Figur 30 zeigt eine Halteanordnung mit einem Behältnis 10, an welchem eine erste Halteeinrichtung 900 und eine zweite Halteinrichtung 910 angeordnet ist, beispielsweise zum Zeitpunkt der Übergabe zwischen einer ersten und einer zweiten Transporteinrichtung.

Man erkennt, analog zu Figur 29, dass die erste Halteinrichtung 900 den Tragring 14 des Behältnisses 10 erfasst und der verjüngte Bereich 901 der ersten Halteeinrichtung 900 in die Richtung der zweiten Halteeinrichtung 910 zeigt. Die zweite Halteeinrichtung 910 greift in die Mundstücksnut 13 des Behältnisses 10 ein, wobei die zweite Halteeinrichtung 910 an der Unterseite des Verschlussrings 12 anliegt.

Die zweite Halteinrichtung 910 zeigt ebenfalls einen verjüngten Bereich 911, welcher in Richtung der ersten Halteeinrichtung 900 zeigt. Weiterhin erkannt man, dass die verjüngten Bereiche 901 und 911 komplementär zueinander ausgebildet sind. Somit wird gewährleistet, dass die beiden Halteeinrichtungen 900 und 910 einander nicht berühren.

Die Anmelderin behält sich vor, sämtliche in den Anmeldungsunterlagen offenbarten Merkmale als erfindungswesentlich zu beanspruchen, sofern sie einzeln oder in Kombination gegenüber dem Stand der Technik neu sind. Es wird weiterhin darauf hingewiesen, dass in den einzelnen Figuren auch Merkmale beschrieben wurden, welche für sich genommen vorteilhaft sein können. Der Fachmann erkennt unmittelbar, dass ein bestimmtes in einer Figur beschriebenes Merkmal auch ohne die Übernahme weiterer Merkmale aus dieser Figur vorteilhaft sein kann. Ferner erkennt der Fachmann, dass sich auch Vorteile durch eine Kombination mehrerer in einzelnen oder in unterschiedlichen Figuren gezeigter Merkmale ergeben können.

### Bezugszeichenliste

- 1: Behältnisbehandlungsvorrichtung
- 2: Heizvorrichtung
- 3: Behältnisumformungsvorrichtung
- 4: Transporteinrichtung, Transportstern (außerhalb des Gehäuses)
- 5: Heizeinrichtung
- 6: Antriebseinrichtung der Transporteinrichtung bzw. des Transportsterns 4
- 10: Behältnis, Vorformling
- 11: Mündung des Kunststoffvorformlings
- 12: Verschlussring
- 13: Mündungsnut
- 14: Tragring
- 20: rotierbarer Träger
- 40: Halteelement, Dorn
- 100: Behältnisaußenflächenbehandlungseinrichtung, Transporteinrichtung
- 105: Hub- und Dreheinrichtung
- 110: Verschiebeeinrichtung, Verschiebemechanismus
- 120: rotierbarer Träger
- 131: Träger des Rotors
- 132: Rotor der Hub- und Dreheinrichtung
- 133: Führungseinrichtung
- 134: Ausleger
- 135: Führungseinrichtung
- 136: Kurvenrolle
- 137: Träger
- 140: Halteelement, Halteeinrichtung, Dorn
- 150: Behältnisaußenflächenbeaufschlagungseinrichtung, Strahlungsquelle
- 152: Behältnisaußenflächenbeaufschlagungsbereich
- 158: Wandung
- 160: Antriebseinrichtung (des rotierbaren Trägers der Behältnisaußenflächenbehandlungseinrichtung)
- 172: Hochspannungskabel mit Stecker
- 174: Vakuumkammer
- 176: Strahlerfläche des Flächenstrahlers
- 178: Vakuumpumpe
- 180: Stator
- 182: untere Abschirmung
- 183: obere Abschirmung
- 184: Statorträger
- 190: Überwachungseinrichtung
- 192: Spulen
- 196: Abdeckung
- 200: Behältnisinnenflächenbehandlungseinrichtung, Transporteinrichtung
- 220: rotierbarer Träger
- 240: Halteelement, Halteeinrichtung, Klammer
- 250: Behältnisinnenflächenbeaufschlagungseinrichtung, Strahlfinger
- 252: Strahlungserzeugungseinrichtung
- 260: Antriebseinrichtung (des rotierbaren Trägers der Behältnisinnenflächenbehandlungseinrichtung)
- 270: Behältnisaufnahmebereich, Behältnisübernahmebereich, -sektor
- 272: Behältnisbehandlungsbereich, -sektor
- 274: Behältnisabgabebereich, Behältnisübergabebereich, -sektor
- 276: Totzeit
- 278: Bereich/Sektor der Verschiebung der unbesetzten Halteeinrichtung
- 280: Profil, Höhenprofil
- 290: (zur Behältnisinnenbehandlung zur Verfügung stehender) Kreisbogen
- 300: Transporteinrichtung, Transportstern (innerhalb des Gehäuses)
- 320: rotierbarer Träger
- 340: Halteelement, Halteeinrichtung, Klammer, Dorn
- 400: Gehäuse
- 402: erste Hubkurve
- 403: zweite Hubkurve
- 405: Abschirmungseinrichtung
- 406: Führungsrolle
- 410: Raum innerhalb des Gehäuses, Gehäuseinneres, Reinraum
- 412: Raum außerhalb des Gehäuses, Umgebung
- 420: Fenster (zum Einschleusen in das Gehäuseinnere)
- 440: Fenster (zum Ausschleusen aus dem Gehäuseinneren)
- 460: Schleuse
- 900: Halteeinrichtung
- 901: verjüngter Bereich
- 902: Bereich
- 904: Haltenut
- 910: Halteinrichtung
- 911: verjüngter Bereich
- v: vertikale Richtung
- T: Transportpfad
- L: Längsrichtung des Kunststoffvorformlings
- K: Kreisbahn
- t: Zeit
- S: Sektor
- G: geradlinige Bewegungsrichtung
- H: Höhenrichtung
- Θ: Winkel
- Θ₁ - Θ₈: Punkt auf Kreis (-bogen)
- R: Radius
- M: Drehmoment
- NP: (magnetischer) Nordpol
- SP: (magnetischer) Südpol
- Z: Zwischenräume zwischen Magneten

## Patentansprüche

1. Behältnisbehandlungsvorrichtung (1), insbesondere Behältnissterilisationsvorrichtung, mit mehreren Transporteinrichtungen (100, 200, 300), zum Transport eines Behältnisses (10) entlang eines vorgegebenen Transportpfads (T), wobei mindestens eine der Transporteinrichtungen (100, 200, 300) mindestens eine Halteeinrichtung (140, 240) zum Halten mindestens eines Behältnisses (10) während einer Behältnisbehandlung und/oder des Transports entlang des Transportpfads (T) aufweist,
**dadurch gekennzeichnet, dass**
die Transporteinrichtung (100, 200, 300) einen rotierbaren Träger (120, 220) und eine Vielzahl von an diesem Träger angeordneten Halteelementen (140, 240) aufweist, wobei ein Haltelement (140, 240) in einem ersten Sektor (270), in dem eine Aufnahme eines Behältnisses (10) erfolgt, eine Höhe (H) gegenüber einer senkrechten Projektion des Transportpfads (T) aufweist, welche von derjenigen in einem zweiten Sektor (274) verschieden ist, in welchem eine Abgabe eines Behältnisses (10) erfolgt und wobei ein Sektor (276), in welchem ein zumindest teilweiser Ausgleich des Höhenunterschieds der Halteelemente erfolgt, entlang einer Rotationsrichtung des Trägers nach dem zweiten Sektor (274) und vor dem ersten Sektor (270) liegt.

2. Behältnisbehandlungsvorrichtung (1) nach Anspruch 1,
**gekennzeichnet durch**
ein Gehäuse (400) innerhalb dessen sich der Transportpfad (T) zumindest abschnittsweise erstreckt und wobei vorzugsweise mindestens eine der Transporteinrichtungen (100, 200, 300) eine Behältnisaußenflächenbehandlungseinrichtung (100) ist und mindestens eine der Transporteinrichtungen (100, 200, 300) eine Behältnisinnenflächenbehandlungseinrichtung (200) ist.

3. Behältnisbehandlungsvorrichtung (1) nach Anspruch 2,
**dadurch gekennzeichnet, dass**
die Behältnisaußenflächenbehandlungseinrichtung (100) und die Behältnisinnenflächenbehandlungseinrichtung (200) entlang des Transportpfads (T) unmittelbar aufeinander folgend angeordnet sind, wobei die Behältnisaußenflächenbehandlungseinrichtung (100) und die Behältnisinnenflächenbehandlungseinrichtung (200) insbesondere für eine Übergabe eines Behältnisses (10) von Behältnisaußenflächenbehandlungseinrichtung (100) an die Behältnisinnenflächenbehandlungseinrichtung (200) vorgesehen und eingerichtet sind.

4. Behältnisbehandlungsvorrichtung (1) nach Anspruch 1 oder 2,
**dadurch gekennzeichnet, dass**
ein Haltelement (240) der Transporteinrichtung (100, 200, 300), insbesondere der Behältnisinnenflächenbehandlungseinrichtung (200) in dem ersten Sektor (270) bezüglich einer senkrechten Projektion des Transportpfads (T) eine geringere Höhe (H) aufweist als in dem zweiten Sektor (274).

5. Behältnisbehandlungsvorrichtung (1) nach einem der vorangegangenen Ansprüche,
**dadurch gekennzeichnet, dass**
ein Haltelement (240) der Transporteinrichtung (100, 200, 300), insbesondere der Behältnisinnenflächenbehandlungseinrichtung (200) in einem dritten Sektor (272), welcher entlang des Transportpfads (T) zwischen dem ersten (270) und zweiten Sektor (274) liegt, zumindest abschnittsweise bezüglich einer senkrechten Projektion des Transportpfads (T) auf einer größeren Höhe (H) angeordnet als in dem ersten Sektor (270) und als in dem zweiten Sektor (274).

6. Behältnisbehandlungsvorrichtung (1) nach einem der vorangegangenen Ansprüche,
**dadurch gekennzeichnet, dass**
in einem dritten Sektor (272) zumindest abschnittsweise eine Behältnisbehandlungseinrichtung (250) zumindest abschnittsweise im Inneren eines Behältnisses (10) angeordnet ist, wobei bevorzugt die Behältnisbehandlungseinrichtung (250) bezüglich der senkrechten Projektion des Transportpfads (T) eine konstante Höhe aufweist.

7. Verfahren zur Behandlung, insbesondere zur Sterilisation von Behältnissen (10) wobei die Behältnisse (10) entlang eines vorgegebenen Transportpfads (T) transportiert werden, wobei sie während des Transports und/oder der Behältnisbehandlung jeweils von mindestens einer Halteeinrichtung (140, 240) gehalten werden,
**dadurch gekennzeichnet, dass**
die Behältnisse (10) zumindest abschnittsweise durch eine Halteeinrichtung (140, 240) der Transporteinrichtung (100, 200, 300)auf einem Abschnitt des Transportpfads (T) geführt werden dessen senkrecht Projektion ein Abschnitt einer Kreisbahn (K) ist, wobei ein Behältnis (10) in einem ersten Sektor (270) von einem Haltelement (240) auf einer ersten Höhe bezüglich der senkrecht Projektion des Transportpfads (T) aufgenommen wird und in einem zweiten Sektor (274) auf einer von der ersten Höhe verschiedenen zweiten Höhe abgegeben wird, wobei ein zumindest teilweiser Ausgleich des Höhenunterschieds der Halteelemente in einem Sektor (276) erfolgt, der entlang einer Rotationsrichtung des Trägers nach dem zweiten Sektor (274) und vor dem ersten Sektor (270) liegt.

8. Verfahren nach Anspruch 7,
**dadurch gekennzeichnet, dass**
sich der Transportpfad (T) zumindest abschnittsweise durch ein Gehäuse (400) erstreckt und wobei die Behältnisse (10) vorzugsweise zumindestens abschnittsweise durch eine Behältnisaußenflächenbehandlungseinrichtung (100) und eine Behältnisinnenflächenbehandlungseinrichtung (200) transportiert werden, wobei insbesondere bevorzugt die Halteeinrichtung (140, 240) der Behältnisaußenflächenbehandlungseinrichtung (100) und/oder die Behältnisinnenflächenbehandlungseinrichtung (200) im ersten Sektor das Behältnis von einer Behältnisaußenflächenbehandlungseinrichtung (100) oder Behältnisinnenflächenbehandlungseinrichtung (200) aufnimmt.

9. Verfahren nach einem der Ansprüche 7 - 8,
**dadurch gekennzeichnet, dass**
das Behältnis (10) im zweiten Sektor (274) in einer zweiten Höhe abgegeben wird, die größer ist als die erste Höhe in der das Behältnis im ersten Sektor (270) aufgenommen wird.

10. Verfahren nach einem der Ansprüche 7 - 9,
**dadurch gekennzeichnet, dass**
das Behältnis (10) in einem dritten Sektor (272), der auf dem Transportpfad (T) zwischen dem ersten (270) und dem zweiten Sektor (274) angeordnet ist, durch eine Behältnisbehandlungseinrichtung (100, 200) mit einem Behandlungsmittel beaufschlagt wird.
